# EUROPEAN PATENT APPLICATION

(11) **EP 3 981 784 A1**
(43) Date of publication of application: **13.04.2022**
(21) Application number: 20200499.0
(22) Date of filing: 07.10.2020
(51) Int. Cl.: C07K 14/435

(54) **REGULATORS OF CELL DIVISION**

(71) Applicant: Technische Universität Dresden, 01069 Dresden (DE)
(72) Inventor: Pisabarro, Maria Teresa, 01307 Dresden (DE); Ruiz-Gómez, Gloria, 01307 Dresden (DE); Uvizl, Alena, 01307 Dresden (DE); Mansfeld, Jörg, 01307 Dresden (DE)
(74) Representative: Dentons Patent Solutions Rechtsanwaltsgesellschaft mbH

(57) **Abstract**

The present invention relates to peptides and peptidomimetics as well as their medical use in the treatment of hyperproliferative diseases.

SEQUENCE LISTING

<110> Technische Universität Dresden

<120> Inhibitors of mitosis

<130> TUDRES.TBC01

<160> 100

<170> BiSSAP 1.3.6

<210> 1
<211> 12
<212> PRT
<213> Artificial Sequence

<220>
<223> Artificial Sequence

<400> 1
Arg Cys His Trp Gly Pro Glu Thr Phe Trp Cys Arg
1               5                   10

<210> 2
<211> 12
<212> PRT
<213> Artificial Sequence

<220>
<223> Artificial Sequence

<400> 2
Arg Cys Ser Trp Gly Pro Glu Thr Phe Trp Cys Arg
1               5                   10

<210> 3
<211> 12
<212> PRT
<213> Artificial Sequence

<220>
<223> Artificial Sequence

<400> 3
Arg Cys His Trp Gly Pro Arg Thr Phe Trp Cys Arg
1               5                   10

<210> 4
<211> 12
<212> PRT
<213> Artificial Sequence

<220>
<223> Artificial Sequence

<220>

<221> MOD_RES
<222> 2
<223> L-allylglycine (Agl)

<220>
<221> MOD_RES
<222> 11
<223> L-allylglycine (Agl)

<400> 4
Arg Xaa Ser Trp Gly Pro Glu Thr Phe Trp Xaa Arg
1               5                   10

<210> 5
<211> 12
<212> PRT
<213> Artificial Sequence

<220>
<223> Artificial Sequence

<220>
<221> MOD_RES
<222> 2
<223> L-allylglycine (Agl)

<220>
<221> MOD_RES
<222> 11
<223> L-allylglycine (Agl)

<400> 5
Arg Xaa His Trp Gly Pro Arg Thr Phe Trp Xaa Arg
1               5                   10

<210> 6
<211> 16
<212> PRT
<213> Artificial Sequence

<220>
<223> Artificial Sequence

<400> 6
Thr His Cys Asp Asp Pro Glu Thr Trp Arg Trp Asn Cys Gln Arg Val
1               5                   10                  15

<210> 7
<211> 16
<212> PRT
<213> Artificial Sequence

<220>
<223> Artificial Sequence

<400> 7

Thr Ser Cys Asp Asp Pro Glu Thr Trp Arg Trp Asn Cys Gln Arg Val
1               5                   10                  15

<210> 8
<211> 16
<212> PRT
<213> Artificial Sequence

<220>
<223> Artificial Sequence

<220>
<221> MOD_RES
<222> 3
<223> L-allylglycine (Agl)

<220>
<221> MOD_RES
<222> 13
<223> L-allylglycine (Agl)

<400> 8
Thr His Xaa Asp Asp Pro Glu Thr Trp Arg Trp Asn Xaa Gln Arg Val
1               5                   10                  15

<210> 9
<211> 16
<212> PRT
<213> Artificial Sequence

<220>
<223> Artificial Sequence

<220>
<221> MOD_RES
<222> 3
<223> L-allylglycine (Agl)

<220>
<221> MOD_RES
<222> 13
<223> L-allylglycine (Agl)

<400> 9
Thr Ser Xaa Asp Asp Pro Glu Thr Trp Arg Trp Asn Xaa Gln Arg Val
1               5                   10                  15

<210> 10
<211> 17
<212> PRT
<213> Artificial Sequence

<220>
<223> Artificial Sequence

<400> 10
Gly Asp Thr Cys Asp Asp Pro Glu Thr Trp Arg Trp Asn Cys Gln Arg
1               5                   10                  15
His



<210> 11
<211> 17
<212> PRT
<213> Artificial Sequence



<220>
<223> Artificial Sequence

<220>
<221> MOD_RES
<222> 4
<223> L-allylglycine (Agl)

<220>
<221> MOD_RES
<222> 14
<223> L-allylglycine (Agl)

<400> 11
Gly Asp Thr Xaa Asp Asp Pro Glu Thr Trp Arg Trp Asn Xaa Gln Arg
1               5                   10                  15
His



<210> 12
<211> 14
<212> PRT
<213> Artificial Sequence



<220>
<223> Artificial Sequence

<400> 12
Cys Glu Ala Gly Pro Ser Gln Trp His Cys Thr Trp Arg Tyr
1               5                   10

<210> 13
<211> 14
<212> PRT
<213> Artificial Sequence



<220>
<223> Artificial Sequence

<400> 13
Cys Glu Ala Gly Pro Ser Gln Trp His Cys Glu Trp Arg Tyr
1               5                   10

<210> 14

<211> 14
<212> PRT
<213> Artificial Sequence

<220>
<223> Artificial Sequence

<220>
<221> MOD_RES
<222> 2
<223> 2-naphthyl-L-alanine (2-NaI)

<220>
<221> MOD_RES
<222> 6
<223> D-Thr (t)

<220>
<221> MOD_RES
<222> 13
<223> D-Pro (p)

<400> 14
Leu Xaa Leu Trp Lys Xaa Glu Trp Glu Lys Ser Lys Xaa Pro
1               5                   10

<210> 15
<211> 14
<212> PRT
<213> Artificial Sequence

<220>
<223> Artificial Sequence

<220>
<221> MOD_RES
<222> 2
<223> 2-naphthyl-L-alanine (2-NaI)

<220>
<221> MOD_RES
<222> 13
<223> D-Pro (p)

<400> 15
Leu Xaa Leu Trp Lys Trp Glu Trp Glu Lys Ser Lys Xaa Pro
1               5                   10

<210> 16
<211> 14
<212> PRT
<213> Artificial Sequence

<220>
<223> Artificial Sequence

<220>
<221> MOD_RES

<222> 2
<223> 2-naphthyl-L-alanine (2-NaI)

<220>
<221> MOD_RES
<222> 6
<223> D-Leu (l)

<220>
<221> MOD_RES
<222> 11
<223> L-citrulline (Cit)

<220>
<221> MOD_RES
<222> 13
<223> D-Pro (p)

<400> 16
Leu Xaa Leu Trp Lys Xaa Glu Trp Glu Lys Xaa Lys Xaa Pro
1               5                   10

<210> 17
<211> 14
<212> PRT
<213> Artificial Sequence


<220>
<223> Artificial Sequence

<220>
<221> MOD_RES
<222> 2
<223> 2-naphthyl-L-alanine (2-NaI)

<220>
<221> MOD_RES
<222> 6
<223> D-Leu (l)

<220>
<221> MOD_RES
<222> 13
<223> D-Pro (p)

<400> 17
Leu Xaa Leu Trp Lys Xaa Glu Trp Glu Lys Arg Lys Xaa Pro
1               5                   10

<210> 18
<211> 14
<212> PRT
<213> Artificial Sequence


<220>
<223> Artificial Sequence

<220>
<221> MOD_RES

<222> 2
<223> 2-naphthyl-L-alanine (2-NaI)

<220>
<221> MOD_RES
<222> 6
<223> D-Thr (t)

<220>
<221> MOD_RES
<222> 11
<223> L-citrulline (Cit)

<220>
<221> MOD_RES
<222> 13
<223> D-Pro (p)

<400> 18
Leu Xaa Leu Trp Lys Xaa Glu Trp Glu Lys Xaa Lys Xaa Pro
1               5                   10

<210> 19
<211> 14
<212> PRT
<213> Artificial Sequence


<220>
<223> Artificial Sequence

<220>
<221> MOD_RES
<222> 2
<223> 2-naphthyl-L-alanine (2-NaI)

<220>
<221> MOD_RES
<222> 13
<223> D-Pro (p)

<400> 19
Leu Xaa Leu Trp Lys Trp Glu Trp Glu Lys Arg Lys Xaa Pro
1               5                   10

<210> 20
<211> 14
<212> PRT
<213> Artificial Sequence


<220>
<223> Artificial Sequence

<220>
<221> MOD_RES
<222> 2
<223> 2-naphthyl-L-alanine (2-NaI)

<220>
<221> MOD_RES

<222> 12
<223> L-propargylglycine (Pra)

<220>
<221> MOD_RES
<222> 13
<223> D-Pro (p)

<400> 20
Leu Xaa Leu Trp Lys Trp Glu Trp Glu Lys Arg Xaa Xaa Pro
1               5                   10

<210> 21
<211> 14
<212> PRT
<213> Artificial Sequence


<220>
<223> Artificial Sequence

<220>
<221> MOD_RES
<222> 2
<223> 2-naphthyl-L-alanine (2-NaI)

<220>
<221> MOD_RES
<222> 6
<223> D-Glu (e)

<220>
<221> MOD_RES
<222> 13
<223> D-Pro (p)

<400> 21
Leu Xaa Leu Trp Lys Xaa Glu Trp Glu Lys Arg Lys Xaa Pro
1               5                   10

<210> 22
<211> 14
<212> PRT
<213> Artificial Sequence


<220>
<223> Artificial Sequence

<220>
<221> MOD_RES
<222> 13
<223> D-Pro (p)

<400> 22
Thr Phe Leu Trp Lys Trp Glu Trp Thr Lys Arg Lys Xaa Pro
1               5                   10

<210> 23
<211> 14
<212> PRT

<213> Artificial Sequence

<220>
<223> Artificial Sequence

<220>
<221> MOD_RES
<222> 13
<223> D-Pro (p)

<400> 23
Thr Phe Leu Trp Lys Trp Glu Trp Glu Lys Arg Lys Xaa Pro
1               5                   10

<210> 24
<211> 14
<212> PRT
<213> Artificial Sequence

<220>
<223> Artificial Sequence

<220>
<221> MOD_RES
<222> 13
<223> D-Pro (p)

<400> 24
Thr Phe Lys Trp Lys Trp Glu Trp Glu Lys Arg Leu Xaa Pro
1               5                   10

<210> 25
<211> 14
<212> PRT
<213> Artificial Sequence

<220>
<223> Artificial Sequence

<220>
<221> MOD_RES
<222> 13
<223> D-Pro (p)

<400> 25
Thr Phe Leu Trp Lys Ala Glu Trp Thr Lys Ser Lys Xaa Pro
1               5                   10

<210> 26
<211> 14
<212> PRT
<213> Artificial Sequence

<220>
<223> Artificial Sequence

<220>

<221> MOD_RES
<222> 2
<223> 2-naphthyl-L-alanine (2-NaI)

<220>
<221> MOD_RES
<222> 6
<223> D-Thr (t)

<220>
<221> MOD_RES
<222> 11
<223> L-homoserine (hSer)

<220>
<221> MOD_RES
<222> 13
<223> D-Pro (p)

<400> 26
Leu Xaa Leu Trp Lys Xaa Glu Trp Glu Lys Xaa Lys Xaa Pro
1               5                   10

<210> 27
<211> 14
<212> PRT
<213> Artificial Sequence

<220>
<223> Artificial Sequence

<220>
<221> MOD_RES
<222> 5
<223> (S)-2-(4'-pentenyl)Ala

<220>
<221> MOD_RES
<222> 9
<223> (S)-2-(4'-pentenyl)Ala

<400> 27
Arg Trp Pro Gln Xaa Lys Trp Asp Xaa Gln Val Arg Arg Trp
1               5                   10

<210> 28
<211> 14
<212> PRT
<213> Artificial Sequence

<220>
<223> Artificial Sequence

<220>
<221> MOD_RES
<222> 4
<223> L-propargylglycine (Pra)

<220>

<221> MOD_RES
<222> 5
<223> (S)-2-(4'-pentenyl)Ala

<220>
<221> MOD_RES
<222> 9
<223> (S)-2-(4'-pentenyl)Ala

<400> 28
Arg Trp Pro Xaa Xaa Lys Trp Asp Xaa Gln Val Arg Arg Trp
1               5                   10

<210> 29
<211> 14
<212> PRT
<213> Artificial Sequence

<220>
<223> Artificial Sequence

<220>
<221> MOD_RES
<222> 5
<223> (S)-2-(4'-pentenyl)Ala

<220>
<221> MOD_RES
<222> 9
<223> (S)-2-(4'-pentenyl)Ala

<400> 29
Arg Glu Pro Gln Xaa Ile Lys Asp Xaa Gln Val Arg Arg Trp
1               5                   10

<210> 30
<211> 14
<212> PRT
<213> Artificial Sequence

<220>
<223> Artificial Sequence

<220>
<221> MOD_RES
<222> 5
<223> (S)-2-(4'-pentenyl)Ala

<220>
<221> MOD_RES
<222> 9
<223> (S)-2-(4'-pentenyl)Ala

<400> 30
Arg Trp Pro Gln Xaa Ile Lys Asp Xaa Gln Val Arg Trp Trp
1               5                   10

<210> 31
<211> 14

<212> PRT
<213> Artificial Sequence

<220>
<223> Artificial Sequence

<220>
<221> MOD_RES
<222> 4
<223> (R)-2-(7'-octenyl)Ala

<220>
<221> MOD_RES
<222> 11
<223> (S)-2-(4'-pentenyl)Ala

<400> 31
Glu Trp Trp Xaa Lys Leu Glu Ile Gln Arg Xaa Arg Arg Ala
1               5                   10

<210> 32
<211> 14
<212> PRT
<213> Artificial Sequence

<220>
<223> Artificial Sequence

<220>
<221> MOD_RES
<222> 4
<223> (R)-2-(7'-octenyl)Ala

<220>
<221> MOD_RES
<222> 11
<223> (S)-2-(4'-pentenyl)Ala

<400> 32
Glu Trp Trp Xaa Lys Leu Glu Leu Gln Arg Xaa Arg Arg Ala
1               5                   10

<210> 33
<211> 14
<212> PRT
<213> Artificial Sequence

<220>
<223> Artificial Sequence

<220>
<221> MOD_RES
<222> 4
<223> (R)-2-(7'-octenyl)Ala

<220>
<221> MOD_RES
<222> 11

<223> (S)-2-(4′-pentenyl)Ala

<400> 33
Glu Trp Trp Xaa Ile Lys Glu Ile Gln Arg Xaa Arg Arg Ala
1               5                   10

<210> 34
<211> 14
<212> PRT
<213> Artificial Sequence

<220>
<223> Artificial Sequence

<220>
<221> MOD_RES
<222> 4
<223> (R)-2-(7′-octenyl)Ala

<220>
<221> MOD_RES
<222> 11
<223> (S)-2-(4′-pentenyl)Ala

<400> 34
Glu Trp Trp Xaa Ile Lys Glu Ile Gln Arg Xaa Arg Arg Ala
1               5                   10

<210> 35
<211> 14
<212> PRT
<213> Artificial Sequence

<220>
<223> Artificial Sequence

<220>
<221> MOD_RES
<222> 4
<223> (R)-2-(7′-octenyl)Ala

<220>
<221> MOD_RES
<222> 11
<223> (S)-2-(4′-pentenyl)Ala

<400> 35
Glu Trp Trp Xaa Ile Lys Glu Ile Gln Arg Xaa Arg Arg Ala
1               5                   10

<210> 36
<211> 14
<212> PRT
<213> Artificial Sequence

<220>
<223> Artificial Sequence

<220>
<221> MOD_RES
<222> 4
<223> (R)-2-(7'-octenyl)Ala

<220>
<221> MOD_RES
<222> 11
<223> (S)-2-(4'-pentenyl)Ala

<400> 36
Glu Trp Trp Xaa Ile Lys Glu Ile Gln Arg Xaa Arg Arg Ala
1               5                   10

<210> 37
<211> 14
<212> PRT
<213> Artificial Sequence

<220>
<223> Artificial Sequence

<220>
<221> MOD_RES
<222> 3
<223> L-propargylglycine (Pra)

<220>
<221> MOD_RES
<222> 4
<223> (R)-2-(7'-octenyl)Ala

<220>
<221> MOD_RES
<222> 11
<223> (S)-2-(4'-pentenyl)Ala

<400> 37
Glu Trp Xaa Xaa Ile Lys Glu Ile Gln Arg Xaa Arg Arg Ala
1               5                   10

<210> 38
<211> 14
<212> PRT
<213> Artificial Sequence

<220>
<223> Artificial Sequence

<220>
<221> MOD_RES
<222> 4
<223> (R)-2-(7'-octenyl)Ala

<220>
<221> MOD_RES
<222> 12
<223> (S)-2-(4'-pentenyl)Ala

<400> 38
Glu Trp Trp Xaa Ile Lys Glu Ile Gln Arg Xaa Arg Arg Ala
1               5                   10

<210> 39
<211> 14
<212> PRT
<213> Artificial Sequence

<220>
<223> Artificial Sequence

<220>
<221> MOD_RES
<222> 4
<223> (R)-2-(7'-octenyl)Ala

<220>
<221> MOD_RES
<222> 11
<223> (S)-2-(4'-pentenyl)Ala

<400> 39
Glu Trp Trp Xaa Ile Lys Glu Ile Gln Arg Xaa Arg Arg Ala
1               5                   10

<210> 40
<211> 14
<212> PRT
<213> Artificial Sequence

<220>
<223> Artificial Sequence

<220>
<221> MOD_RES
<222> 4
<223> (R)-2-(7'-octenyl)Ala

<220>
<221> MOD_RES
<222> 11
<223> (S)-2-(4'-pentenyl)Ala

<400> 40
Glu Trp Trp Xaa Ile Lys Glu Ile Gln Arg Xaa Arg Arg Ala
1               5                   10

<210> 41
<211> 12
<212> PRT
<213> Artificial Sequence

<220>
<223> Artificial Sequence

<220>
<221> MOD_RES

<222> 2
<223> L-allylglycine (Agl)

<220>
<221> MOD_RES
<222> 11
<223> L-allylglycine (Agl)

<400> 41
Arg Xaa Ser Trp Gly Pro Glu Thr Phe Trp Xaa Arg
1               5                   10

<210> 42
<211> 12
<212> PRT
<213> Artificial Sequence

<220>
<223> Artificial Sequence

<220>
<221> MOD_RES
<222> 2
<223> L-allylglycine (Agl)

<220>
<221> MOD_RES
<222> 11
<223> L-allylglycine (Agl)

<400> 42
Arg Xaa His Trp Gly Pro Arg Thr Phe Trp Xaa Arg
1               5                   10

<210> 43
<211> 12
<212> PRT
<213> Artificial Sequence

<220>
<223> Artificial Sequence

<220>
<221> MOD_RES
<222> 2
<223> L-propargylglycine (Pra)

<220>
<221> MOD_RES
<222> 10
<223> 5-azido-L-norvaline (Nva(δ-N3))

<400> 43
Arg Xaa His Trp Gly Pro Arg Thr Phe Trp Xaa Arg
1               5                   10

<210> 44
<211> 12
<212> PRT

<213> Artificial Sequence

<220>
<223> Artificial Sequence

<220>
<221> MOD_RES
<222> 2
<223> L-allylglycine (Agl)

<220>
<221> MOD_RES
<222> 4
<223> L-crotylglycine (Crt)

<220>
<221> MOD_RES
<222> 9
<223> L-crotylglycine (Crt)

<220>
<221> MOD_RES
<222> 11
<223> L-allylglycine (Agl)

<400> 44
Arg Xaa Ser Xaa Gly Pro Arg Thr Xaa Trp Xaa Arg
1               5                   10

<210> 45
<211> 12
<212> PRT
<213> Artificial Sequence

<220>
<223> Artificial Sequence

<220>
<221> MOD_RES
<222> 2
<223> L-allylglycine (Agl)

<220>
<221> MOD_RES
<222> 4
<223> L-crotylglycine (Crt)

<220>
<221> MOD_RES
<222> 9
<223> L-crotylglycine (Crt)

<220>
<221> MOD_RES
<222> 11
<223> L-allylglycine (Agl)

<400> 45
Arg Xaa His Xaa Gly Pro Arg Thr Xaa Trp Xaa Arg

1                   5                       10

<210> 46
<211> 12
<212> PRT
<213> Artificial Sequence

<220>
<223> Artificial Sequence

<220>
<221> MOD_RES
<222> 2
<223> L-allylglycine (Agl)

<220>
<221> MOD_RES
<222> 4
<223> L-crotylglycine (Crt)

<220>
<221> MOD_RES
<222> 9
<223> L-crotylglycine (Crt)

<220>
<221> MOD_RES
<222> 11
<223> L-allylglycine (Agl)

<400> 46
Arg Xaa His Xaa Gly Pro Arg Thr Xaa Trp Xaa Arg
1               5                   10

<210> 47
<211> 12
<212> PRT
<213> Artificial Sequence

<220>
<223> Artificial Sequence

<220>
<221> MOD_RES
<222> 2
<223> L-propargylglycine (Pra)

<220>
<221> MOD_RES
<222> 4
<223> L-propargylglycine (Pra)

<220>
<221> MOD_RES
<222> 9
<223> (S)-2-amino-4-azido-butyric acid (L-azidohomoalanine),
      (2Abu(γ-N3))

<220>

<221> MOD_RES
<222> 11
<223> 5-azido-L-norvaline (Nva(δ-N3))

<400> 47
Arg Xaa His Xaa Gly Pro Arg Thr Xaa Trp Xaa Arg
1               5                   10

<210> 48
<211> 14
<212> PRT
<213> Artificial Sequence

<220>
<223> Artificial Sequence

<400> 48
Gly Arg Cys His Trp Gly Pro Glu Thr Phe Trp Cys Arg Thr
1               5                   10

<210> 49
<211> 12
<212> PRT
<213> Artificial Sequence

<220>
<223> Artificial Sequence

<400> 49
Arg Cys His Trp Gly Pro Arg Thr Phe Trp Cys Arg
1               5                   10

<210> 50
<211> 12
<212> PRT
<213> Artificial Sequence

<220>
<223> Artificial Sequence

<400> 50
Arg Cys His Trp Gly Pro Arg Thr Phe Trp Cys Arg
1               5                   10

<210> 51
<211> 12
<212> PRT
<213> Artificial Sequence

<220>
<223> Artificial Sequence

<400> 51
Arg Cys His Trp Gly Pro Arg Thr Phe Trp Cys Arg
1               5                   10

<210> 52

<211> 12
<212> PRT
<213> Artificial Sequence

<220>
<223> Artificial Sequence

<220>
<221> MOD_RES
<222> 2
<223> L-allylglycine (Agl)

<220>
<221> MOD_RES
<222> 11
<223> L-allylglycine (Agl)

<400> 52
Arg Xaa His Trp Gly Pro Arg Thr Phe Trp Xaa Arg
1               5                   10

<210> 53
<211> 12
<212> PRT
<213> Artificial Sequence

<220>
<223> Artificial Sequence

<220>
<221> MOD_RES
<222> 2
<223> L-allylglycine (Agl)

<220>
<221> MOD_RES
<222> 11
<223> L-allylglycine (Agl)

<400> 53
Arg Xaa His Trp Gly Pro Arg Thr Phe Trp Xaa Arg
1               5                   10

<210> 54
<211> 12
<212> PRT
<213> Artificial Sequence

<220>
<223> Artificial Sequence

<220>
<221> MOD_RES
<222> 2
<223> L-allylglycine (Agl)

<220>
<221> MOD_RES

<222> 11
<223> L-allylglycine (Agl)

<400> 54
Arg Xaa His Trp Gly Pro Arg Thr Phe Trp Xaa Arg
1               5                   10

<210> 55
<211> 16
<212> PRT
<213> Artificial Sequence

<220>
<223> Artificial Sequence

<220>
<221> MOD_RES
<222> 3
<223> L-allylglycine (Agl)

<220>
<221> MOD_RES
<222> 13
<223> L-allylglycine (Agl)

<400> 55
Thr His Xaa Asp Asp Pro Glu Thr Trp Arg Trp Asn Xaa Gln Arg Val
1               5                   10                  15

<210> 56
<211> 16
<212> PRT
<213> Artificial Sequence

<220>
<223> Artificial Sequence

<400> 56
Thr His Cys Asp Asp Pro Glu Thr Trp Arg Trp Asn Cys Gln Arg Val
1               5                   10                  15

<210> 57
<211> 16
<212> PRT
<213> Artificial Sequence

<220>
<223> Artificial Sequence

<220>
<221> MOD_RES
<222> 3
<223> L-allylglycine (Agl)

<220>
<221> MOD_RES
<222> 13
<223> L-allylglycine (Agl)

<400> 57
Thr His Xaa Asp Asp Pro Glu Thr Trp Arg Trp Asn Xaa Gln Arg Val
1               5                   10                  15

<210> 58
<211> 16
<212> PRT
<213> Artificial Sequence

<220>
<223> Artificial Sequence

<400> 58
Thr His Cys Asp Asp Pro Glu Thr Trp Arg Trp Asn Cys Gln Arg Val
1               5                   10                  15

<210> 59
<211> 16
<212> PRT
<213> Artificial Sequence

<220>
<223> Artificial Sequence

<220>
<221> MOD_RES
<222> 3
<223> L-allylglycine (Agl)

<220>
<221> MOD_RES
<222> 13
<223> L-allylglycine (Agl)

<400> 59
Thr His Xaa Asp Asp Pro Glu Thr Trp Arg Trp Asn Xaa Gln Arg Val
1               5                   10                  15

<210> 60
<211> 17
<212> PRT
<213> Artificial Sequence

<220>
<223> Artificial Sequence

<220>

<221> MOD_RES
<222> 4
<223> L-allylglycine (Agl)

<220>
<221> MOD_RES
<222> 14
<223> L-allylglycine (Agl)

<400> 60
Gly Asp Thr Xaa Asp Asp Pro Glu Thr Trp Arg Trp Asn Xaa Gln Arg
1               5                   10                  15
His

<210> 61
<211> 17
<212> PRT
<213> Artificial Sequence

<220>
<223> Artificial Sequence

<400> 61
Gly Asp Thr Cys Asp Asp Pro Glu Thr Trp Arg Trp Asn Cys Gln Arg
1               5                   10                  15
His

<210> 62
<211> 17
<212> PRT
<213> Artificial Sequence

<220>
<223> Artificial Sequence

<220>
<221> MOD_RES
<222> 4
<223> L-allylglycine (Agl)

<220>
<221> MOD_RES
<222> 14
<223> L-allylglycine (Agl)

<400> 62
Gly Asp Thr Xaa Asp Asp Pro Glu Thr Trp Arg Trp Asn Xaa Gln Arg
1               5                   10                  15
His

<210> 63
<211> 17
<212> PRT
<213> Artificial Sequence

<220>
<223> Artificial Sequence

<400> 63
Gly Asp Thr Cys Asp Asp Pro Glu Thr Trp Arg Trp Asn Cys Gln Arg
1               5                   10                  15
His

<210> 64
<211> 17
<212> PRT
<213> Artificial Sequence

<220>
<223> Artificial Sequence

<400> 64
Gly Asp Thr Cys Asp Asp Pro Glu Thr Trp Arg Trp Asn Cys Gln Arg
1               5                   10                  15
His

<210> 65
<211> 17
<212> PRT
<213> Artificial Sequence

<220>
<223> Artificial Sequence

<220>
<221> MOD_RES
<222> 4
<223> L-allylglycine (Agl)

<220>
<221> MOD_RES
<222> 14
<223> L-allylglycine (Agl)

<400> 65
Gly Asp Thr Xaa Asp Asp Pro Glu Thr Trp Arg Trp Asn Xaa Gln Arg
1               5                   10                  15
His

<210> 66
<211> 17
<212> PRT
<213> Artificial Sequence

<220>
<223> Artificial Sequence

<220>
<221> MOD_RES
<222> 4

<223> L-allylglycine (Agl)

<220>
<221> MOD_RES
<222> 14
<223> L-allylglycine (Agl)

<400> 66
Gly Asp Thr Xaa Asp Asp Pro Glu Thr Trp Arg Trp Asn Xaa Gln Arg
1               5                   10                  15
His



<210> 67
<211> 14
<212> PRT
<213> Artificial Sequence



<220>
<221> MOD_RES
<222> 1
<223> L-allylglycine (Agl)

<220>
<223> Artificial Sequence

<220>
<221> MOD_RES
<222> 10
<223> L-allylglycine (Agl)

<400> 67
Xaa Glu Ala Gly Pro Ser Gln Trp His Xaa Thr Trp Arg Tyr
1               5                   10

<210> 68
<211> 14
<212> PRT
<213> Artificial Sequence



<220>
<221> MOD_RES
<222> 1
<223> L-allylglycine (Agl)

<220>
<223> Artificial Sequence

<220>
<221> MOD_RES
<222> 10
<223> L-allylglycine (Agl)

<400> 68
Xaa Glu Ala Gly Pro Ser Gln Trp His Xaa Thr Trp Arg Tyr
1               5                   10

<210> 69
<211> 14

<212> PRT
<213> Artificial Sequence

<220>
<221> MOD_RES
<222> 1
<223> L-allylglycine (Agl)

<220>
<223> Artificial Sequence

<220>
<221> MOD_RES
<222> 10
<223> L-allylglycine (Agl)

<400> 69
Xaa Glu Ala Gly Pro Ser Gln Trp His Xaa Thr Trp Arg Tyr
1               5                   10

<210> 70
<211> 14
<212> PRT
<213> Artificial Sequence

<220>
<223> Artificial Sequence

<220>
<221> MOD_RES
<222> 2
<223> 2-naphthyl-L-alanine (2-NaI)

<220>
<221> MOD_RES
<222> 13
<223> NH2COCH2NH-Allylamine

<220>
<221> MOD_RES
<222> 14
<223> 4-pentenoic acid

<400> 70
Leu Xaa Leu Trp Lys Trp Glu Trp Glu Lys Arg Lys Xaa Xaa
1               5                   10

<210> 71
<211> 13
<212> PRT
<213> Artificial Sequence

<220>
<223> Artificial Sequence

<220>
<221> MOD_RES
<222> 2

<223> 2-naphthyl-L-alanine (2-NaI)

<220>
<221> MOD_RES
<222> 13
<223> L-ornithine (Orn)

<400> 71
Leu Xaa Leu Trp Lys Trp Glu Trp Glu Lys Arg Lys Xaa
1               5                   10

<210> 72
<211> 14
<212> PRT
<213> Artificial Sequence

<220>
<223> Artificial Sequence

<220>
<221> MOD_RES
<222> 5
<223> (S)-2-(4'-pentenyl)Ala

<220>
<221> MOD_RES
<222> 9
<223> (S)-2-(4'-pentenyl)Ala

<400> 72
Arg Trp Pro Gln Xaa Ile Lys Asp Xaa Gln Val Arg Trp Trp
1               5                   10

<210> 73
<211> 14
<212> PRT
<213> Artificial Sequence

<220>
<223> Artificial Sequence

<220>
<221> MOD_RES
<222> 5
<223> (S)-2-(4'-pentenyl)Ala

<220>
<221> MOD_RES
<222> 9
<223> (S)-2-(4'-pentenyl)Ala

<400> 73
Arg Trp Pro Gln Xaa Ile Lys Asp Xaa Gln Val Arg Trp Trp
1               5                   10

<210> 74
<211> 14
<212> PRT
<213> Artificial Sequence

<220>
<223> Artificial Sequence

<220>
<221> MOD_RES
<222> 5
<223> (S)-2-(4'-pentenyl)Ala

<220>
<221> MOD_RES
<222> 9
<223> (S)-2-(4'-pentenyl)Ala

<400> 74
Arg Trp Pro Gln Xaa Ile Lys Asp Xaa Gln Val Arg Trp Trp
1               5                   10

<210> 75
<211> 14
<212> PRT
<213> Artificial Sequence

<220>
<223> Artificial Sequence

<220>
<221> MOD_RES
<222> 5
<223> L-propargylglycine (Pra)

<220>
<221> MOD_RES
<222> 9
<223> L-azidolysine (K(N3))

<400> 75
Arg Trp Pro Gln Xaa Ile Lys Asp Xaa Gln Val Arg Trp Trp
1               5                   10

<210> 76
<211> 14
<212> PRT
<213> Artificial Sequence

<220>
<223> Artificial Sequence

<400> 76
Glu Trp Lys Trp Trp Ile Asp Lys Ile Gln Val Asp Arg Ala
1               5                   10

<210> 77
<211> 14
<212> PRT
<213> Artificial Sequence

<220>
<223> Artificial Sequence

<400> 77
Glu Trp Lys Trp Trp Ile Asp Lys Ile Gln Val Asp Trp Ala
1               5                   10

<210> 78
<211> 14
<212> PRT
<213> Artificial Sequence


<220>
<223> Artificial Sequence

<400> 78
Glu Trp Lys Trp Arg Ile Asp Lys Ile Gln Val Asp Arg Ala
1               5                   10

<210> 79
<211> 14
<212> PRT
<213> Artificial Sequence


<220>
<223> Artificial Sequence

<400> 79
Glu Trp Lys Trp Trp Ile Asp Lys Ile Gln Val Asp Arg Ala
1               5                   10

<210> 80
<211> 14
<212> PRT
<213> Artificial Sequence


<220>
<223> Artificial Sequence

<400> 80
Glu Trp Lys Trp Trp Ile Asp Lys Ile Gln Val Asp Arg Ala
1               5                   10

<210> 81
<211> 15
<212> PRT
<213> Artificial Sequence


<220>
<223> Artificial Sequence

<400> 81
Glu Trp Trp Lys Arg Ile Lys Asp Ile Gln Lys Trp Arg Arg Asp
1               5                   10                  15

<210> 82
<211> 15

<212> PRT
<213> Artificial Sequence

<220>
<223> Artificial Sequence

<400> 82
Glu Trp Glu Lys Arg Ile Lys Asp Ile Gln Lys Trp Arg Arg Asp
1               5                   10                  15

<210> 83
<211> 15
<212> PRT
<213> Artificial Sequence

<220>
<223> Artificial Sequence

<400> 83
Glu Trp Trp Lys Arg Ile Lys Asp Ile Gln Lys Trp Trp Arg Asp
1               5                   10                  15

<210> 84
<211> 15
<212> PRT
<213> Artificial Sequence

<220>
<223> Artificial Sequence

<400> 84
Glu Trp Trp Lys Arg Ile Lys Asp Ile Gln Lys Trp Arg Arg Asp
1               5                   10                  15

<210> 85
<211> 15
<212> PRT
<213> Artificial Sequence

<220>
<223> Artificial Sequence

<400> 85
Glu Trp Trp Lys Arg Ile Lys Asp Ile Gln Lys Trp Arg Arg Asp
1               5                   10                  15

<210> 86
<211> 15
<212> PRT
<213> Artificial Sequence

<220>
<223> Artificial Sequence

<400> 86
Glu Trp Trp Lys Arg Ile Lys Asp Ile Gln Lys Trp Arg Arg Asp

1               5                   10                  15

<210> 87
<211> 12
<212> PRT
<213> Artificial Sequence

<220>
<223> Artificial Sequence

<220>
<221> MOD_RES
<222> 2
<223> L-propargylglycine (Pra)

<220>
<221> MOD_RES
<222> 4
<223> L-allylglycine (Agl)

<220>
<221> MOD_RES
<222> 9
<223> L-allylglycine (Agl)

<220>
<221> MOD_RES
<222> 11
<223> 5-azido-L-norvaline (Nva(δ-N3))

<400> 87
Arg Xaa His Xaa Gly Pro Arg Thr Xaa Trp Xaa Arg
1               5                   10

<210> 88
<211> 12
<212> PRT
<213> Artificial Sequence

<220>
<223> Artificial Sequence

<220>
<221> MOD_RES
<222> 2
<223> L-propargylglycine (Pra)

<220>
<221> MOD_RES
<222> 4
<223> L-allylglycine (Agl)

<220>
<221> MOD_RES
<222> 9
<223> L-allylglycine (Agl)

<220>
<221> MOD_RES

<222> 11
<223> 5-azido-L-norvaline (Nva(δ-N3))

<400> 88
Arg Xaa His Xaa Gly Pro Arg Thr Xaa Trp Xaa Arg
1               5                   10

<210> 89
<211> 12
<212> PRT
<213> Artificial Sequence

<220>
<223> Artificial Sequence

<220>
<221> MOD_RES
<222> 4
<223> L-homopropargylglycine (Hpg)

<220>
<221> MOD_RES
<222> 9
<223> 3-azido-L-alanine (Dap(N3))

<400> 89
Arg Trp His Xaa Gly Pro Arg Thr Xaa Trp Phe Arg
1               5                   10

<210> 90
<211> 14
<212> PRT
<213> Artificial Sequence

<220>
<223> Artificial Sequence

<220>
<221> MOD_RES
<222> 4
<223> (R)-2-(7'-octenyl)Ala

<220>
<221> MOD_RES
<222> 11
<223> (S)-2-(4'-pentenyl)Ala

<400> 90
Glu Trp Trp Xaa Ile Lys Glu Ile Gln Arg Xaa Arg Arg Ala
1               5                   10

<210> 91
<211> 14
<212> PRT
<213> Artificial Sequence

<220>
<223> Artificial Sequence

<220>
<221> MOD_RES
<222> 4
<223> (R)-2-(7'-octenyl)Ala

<220>
<221> MOD_RES
<222> 11
<223> (S)-2-(4'-pentenyl)Ala

<400> 91
Glu Trp Trp Xaa Ile Lys Glu Ile Gln Arg Xaa Arg Arg Ala
1               5                   10

<210> 92
<211> 14
<212> PRT
<213> Artificial Sequence

<220>
<223> Artificial Sequence

<220>
<221> MOD_RES
<222> 4
<223> (R)-2-(7'-octenyl)Ala

<220>
<221> MOD_RES
<222> 11
<223> (S)-2-(4'-pentenyl)Ala

<400> 92
Glu Trp Trp Xaa Ile Lys Glu Ile Gln Arg Xaa Arg Arg Ala
1               5                   10

<210> 93
<211> 14
<212> PRT
<213> Artificial Sequence

<220>
<223> Artificial Sequence

<220>
<221> MOD_RES
<222> 4
<223> (R)-2-(7'-octenyl)Ala

<220>
<221> MOD_RES
<222> 11
<223> (S)-2-(4'-pentenyl)Ala

<400> 93
Glu Trp Trp Xaa Ile Lys Glu Ile Gln Arg Xaa Arg Arg Ala
1               5                   10

<210> 94
<211> 12
<212> PRT
<213> Artificial Sequence

<220>
<223> Artificial Sequence

<220>
<221> MOD_RES
<222> 2
<223> L-allylglycine (Agl)

<220>
<221> MOD_RES
<222> 11
<223> L-allylglycine (Agl)

<400> 94
Arg Xaa Ser Trp Gly Pro Glu Thr Phe Trp Xaa Arg
1               5                   10

<210> 95
<211> 16
<212> PRT
<213> Artificial Sequence

<220>
<223> Artificial Sequence

<220>
<221> MOD_RES
<222> 3
<223> L-allylglycine (Agl)

<220>
<221> MOD_RES
<222> 13
<223> L-allylglycine (Agl)

<400> 95
Thr His Xaa Asp Asp Pro Glu Thr Trp Arg Trp Asn Xaa Gln Arg Val
1               5                   10                  15

<210> 96
<211> 14
<212> PRT
<213> Artificial Sequence

<220>
<223> Artificial Sequence

<220>
<221> MOD_RES
<222> 5
<223> (S)-2-(4'-pentenyl)Ala

<220>
<221> MOD_RES
<222> 9
<223> (S)-2-(4'-pentenyl)Ala

<400> 96
Arg Trp Pro Gln Xaa Ile Lys Asp Xaa Gln Val Arg Trp Trp
1               5                   10

<210> 97
<211> 14
<212> PRT
<213> Artificial Sequence

<220>
<223> Artificial Sequence

<400> 97
Glu Trp Lys Trp Trp Ile Asp Lys Ile Gln Val Asp Arg Ala
1               5                   10

<210> 98
<211> 15
<212> PRT
<213> Artificial Sequence

<220>
<223> Artificial Sequence

<400> 98
Glu Trp Trp Lys Arg Ile Lys Asp Ile Gln Lys Trp Arg Arg Asp
1               5                   10                  15

<210> 99
<211> 14
<212> PRT
<213> Artificial Sequence

<220>
<223> Artificial Sequence

<220>
<221> MOD_RES
<222> 5
<223> Alpha-aminoisobutyric acid (Aib)

<220>
<221> MOD_RES
<222> 9
<223> Alpha-aminoisobutyric acid (Aib)

<400> 99
Arg Trp Pro Gln Xaa Lys Trp Asp Xaa Gln Val Arg Arg Trp
1               5                   10

<210> 100
<211> 5

<212> PRT
<213> Artificial Sequence

<220>
<223> Artificial Sequence

<400> 100
Gly Phe Trp Phe Gly
1               5

## Description

The present invention is defined by the claims. It relates to peptides and peptidomimetics as well as their medical use in the treatment of hyperproliferative diseases.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

There are two key players in regulating cell cycle progression and genome integrity: the activity of the anaphase-promoting complex (APC/C) E3 ubiquitin ligase and the cullin-RING E3 ubiquitin ligase Skpl/Cull/F-box (SCF) complex, which are very often mis-regulated in cancer. The APC/C consists of at least 14 subunits in human cells and utilizes two co-activators, Cdc20 and Cdh1, to recognize its substrates. There are three main causes of APC/C mis-regulation - alterations in the APC/C itself, Cdc20 and Cdh1 and in the APC/C E2 ubiquitin conjugating enzymes UBE2C and UBE2S. In a similar manner, SCF, consisting of an F-box protein and three core units, can be mis-regulated through altering its interaction with the E2 conjugating enzyme Cdc34. Both, APC/C and SCF are targeted by inhibitory proteins, Emi1 and GLMN, respectively, allowing to futher fine-tune their activities.

The alterations in the APC/C itself are not very frequent though present in several cancer types. Firstly, in colon cancer cells, mutations of several APC/C subunits were found - APC4, APC6 and APC8. Interestingly, all these mutations were heterozygous indicating the crucial role of the APC/C. It is thought that a quantitative or qualitative disruption of APC/C subunits is sufficient to cause a mis-regulation of APC/C activity and thereby contributes to tumorigenesis. Further, the overexpression of APC/C subunits is associated with poor prognosis and could serve as new markers for cancer evaluation. It was shown that APC3 overexpression is associated with colorectal cancer progression and APC11 overexpression with progression of lung adenocarcinoma and colorectal cancer, respectively. Conversely, downregulation of the APC7 in invasive ductal carcinoma was associated with poor prognosis indicating high variability in APC/C subunits alterations between distinct cancer types. More frequently, APC/C activity is mis-regulated via alterations in APC/C co-activators Cdc20 and Cdh1.

Cdc20 has been frequently overexpressed in a wide range of cancer such as B-cell non-Hodgkin lymphomas, bladder cancer, colorectal cancer, glioblastoma, non-small cell lung cancer and pancreatic ductal adenocarcinoma. Further, Cdc20 overexpression is associated with a poor prognosis. Overall, Cdc20 was proposed to be a new cancer prognostic marker as well as a target of cancer therapy. Interestingly, Cdc20 overexpression is likely to be associated with decrease of the tumour suppressor p53, since Cdc20 was identified as the p53-suppressive gene. Conversely, Cdh1 has been frequently downregulated in distinct cancers such as breast cancer, colorectal cancer, ovary cancer and prostate cancer. Alternatively, it was shown that also invalid Cdh1 hyperphosphorylation drives APC/C inactivation occurring in glioblastoma. As a consequence of downregulation of APC/C^{Cdh1} activity, the levels of APC/C^{Cdh1} substrates are elevated, some of which are oncogenes for instance Skp2 or Plk1.

Finally, the APC/C activity is often mis-regulated by overexpression of its E2 ubiquitin conjugating enzymes UBE2C and UBE2S. Overexpression of UBE2C as well as UBE2S was described in a wide range of cancer and is associated with poor prognosis. Further, UBE2C and UBE2S were suggested to be cancer prognostic markers. UBE2C overexpression was described for instance in breast cancer, bladder cancer, glioblastoma, lung cancer, ovary cancer and melanoma. UBE2C overexpression could be partially explained by its gene amplification, supported by the example of gastric adenocarcinoma in which the amplification of the chromosomal region 20q13.1, where the *UBE2C* gene is localized, has been described. Further, UBE2S overexpression was described for instance in breast cancer, cervical cancer, hepatocellular carcinoma, lung adenocarcinoma and pancreatic ductal adenocarcinoma.

### Indirect inhibition of the APC/C

The primary target of drugs interfering indirectly with the APC/C activity are mitotic cells and their eradication, since cancer cells proliferate faster compared to most of the healthy tissue. There are two main drug classes indirectly inhibiting the APC/C activity - (i) drugs perturbing spindle assembly and activating the spindle assembly checkpoint (SAC) such as microtubule targeting agents and (ii) drugs inhibiting the proteasome.

### Microtubule targeting agents

Microtubule targeting agents bind to tubulin, thus interfering with spindle assembly and activating the SAC. There are two main classes of microtubule targeting agents used in clinics - taxanes and vinca alkaloids. Taxanes, such as paclitaxel and docetaxel, are microtubule-stabilising drugs and vinca alkaloids, such as vinblastine, vincristine, vinolrebine, are microtubule-depolymerizing drugs. Drug-induced SAC activation leads to prolonged mitotic arrest followed in an ideal case by cell death in mitosis. However, an alternative pathway, called mitotic slippage also exists, in which cells progress slowly through mitosis despite activated SAC and form tetraploid cells entering the G1 phase. Tetraploid cells in the G1 phase have different possible fates - post-slippage cell death, entering senescence or continuing in cell cycle. Of note, the response to microtubule targeting agents is very heterogenous, and different cell fates can occur even in the related cells. Importantly, cell polyploidy is connected to drug resistance, thus with cancer relapse.

Mitotic slippage is a naturally occurring process caused by slow background degradation of cyclin B, since always a small part of APC/C^{Cdc20} is active. The cell fate decision between mitotic slippage and mitotic cell death is tightly regulated by the balance between two opposing activities, APC/C mediated cyclin B degradation and the activation of the apoptotic pathways. To eliminate mitotic slippage and prefer mitotic cell death, strategies accelerating apoptosis or delay mitotic slippage could be employed. For instance, it was shown that downregulation of p31^{comet} prolongs a mitotic arrest and promotes apoptosis. Mitotic slippage is one of the biggest obstacles of microtubule targeting agents, causing drug resistance and cancer relapse. Further, microtubule targeting agents have strong side effects including neuropathy, limiting their application. To overcome problems with drug resistance as well as with side effects, a combinatorial therapy with lower drug doses should be considered, for example combination with drugs accelerating apoptosis or inhibiting the APC/C activity.

### Proteasome inhibitors

Cellular homeostasis is maintained by keeping balance between protein synthesis and protein degradation. Ubiquitin-proteasome pathway is essential for protein degradation including proteins involved in cell cycle, cell survival, apoptosis or DNA repair. Also, many proteasome substrates are known to be mis-regulated in cancer. Disruption of proteasome activity causes cell arrest followed by cell death due to rapid protein accumulation including APC/C substrates such as cyclins A, B, securin and geminin, key regulators of cell cycle progression.

Interestingly, contrary to predictions, cancer cells are more sensitive to proteasome inhibition than non-cancerous cells, making proteasome a rational cancer therapy target. For instance, it was shown that acute myelogenous leukaemia stem cells are more sensitive to proteasome inhibition than normal hematopoietic stem cells. Higher sensitivity towards proteasome inhibitors in cancer cells is still not fully understood. In general, since for cancer cells protein overexpression and expression of misfolded proteins are typical, they are more dependent on proteasome activity to keep cellular homeostasis.

To this date, three proteasome inhibitors, Bortezomib, Carfilzomib and Ixazomib, have received approval to be used in clinics for treatment of multiple myeloma and mantle cell lymphoma. Bortezomib is the first-generation proteasome inhibitor, it has relatively severe side effects including neuropathy, thrombocytopenia and weakness compared to the second-generation inhibitors Carfilzomib and Ixazomib. Except monotherapy, proteasome inhibitors are also tested in the combined therapy, since it was shown that proteasome inhibition sensitises cells to other chemotherapy drugs and radiation therapy. The advantage of the combination therapy is also lower doses of drugs, thereby fewer side effects.

Even though proteasome inhibitors seem to be very promising in cancer therapy, their biggest disadvantage and current challenge is a frequent development of resistance, thereby cancer relapse. Further, they were successfully used only in treatment of hematopoietic malignancies, however, they are not efficient in solid tumours. Mentioned challenges could be partially solved by the combined therapy.

### Direct inhibition of the APC/C

### Downregulation of the APC/C activity and its effect on cells

The APC/C is essential for cell cycle progression. In mouse models, it has been shown that loss of the catalytic core subunit APC2 as well as loss of the APC10 subunit is lethal during early embryogenesis. Further, loss of the APC2 subunit in a mouse model leads to the cell-cycle reentry of hepatocytes followed by cell cycle arrest in mitosis. More studies about different APC/C subunits have been performed in various cell lines. For instance, the APC3 downregulation inhibits cell growth of colon cancer cells HCT116. Specifically, APC3 downregulated cells accumulate in the G1 phase with the increased p21 protein level. Conversely, APC3 overexpression induces cell proliferation. Further, APC4 downregulation induces prolonged mitosis and metaphase arrest with increased levels of cyclin A and cyclin B in cervical cancer HeLa cells. Interestingly, not all APC/C subunits are essential for successful progression through mitosis. APC7 and APC16 are not required for mitosis in colon cancer cells HCT116 under unperturbed conditions.

Since the APC/C is a large complex with many subunits, it is easier to interfere with its co-activators Cdc20 and Cdh1 to study APC/C functions. For both co-activators, failed embryogenesis was observed in mouse knock-out models. Specifically, Cdc20 knockout mouse embryos die at the two-cell stage following metaphase arrest with high levels of cyclin B. Cdh1 knockout mouse embryos die later at E9.5 - E10.5 and not due to basic cell cycle defects, however, due to defects in the endoreduplication of trophoblast cells forming placenta. Suggesting that even though Cdc20 and Cdh1 recognize partially same substrates, they are not functionally redundant and have own specific functions. Consistently with the mouse model, Cdc20 downregulation in various cell lines causes mitotic arrest followed by impaired cell proliferation and eventually cell death. Cdh1 is not essential for completion of mitosis, however, it plays a crucial role in controlling G1 phase and followed S phase. Downregulation of Cdh1 causes shortening G1 phase, due to faster accumulation of cyclin A, and conversely, prolonging S phase, due to caused replication stress and shortage of the dNTPs. Further, Cdh1 deficient cells accumulate DNA breaks and chromosomal aberrations indicating the role of Cdh1 in maintenance of genome stability. Cdh1 deficient cells also fail to maintain the DNA damage induced G2 phase arrest, thus allowing cells to enter mitosis with DNA damage, thereby causing cell death in mitosis.

As mentioned above, there are two E2 ubiquitin conjugating enzymes catalysing ubiquitination reactions together with the APC/C, UBE2C and UBE2S. In various non-cancerous and cancer cell lines, it was shown that UBE2C downregulation supresses cell growth and induces cell cycle arrest in mitosis, specifically in metaphase. Further, in glioma cells, an enhanced apoptosis was observed. Conversely, UBE2S depletion in various non-cancerous and cancer cell lines causes just a minor delay in mitosis and does not have a strong phenotype under unperturbed conditions. However, UBE2S depletion prolongs drug-induced mitotic arrest and suppresses mitotic slippage suggesting the role of UBE2S in SAC inactivation. Co-depletion of UBE2C and UBE2S prolong mitosis more and result in the accumulation of APC/C substrates in agreement with their joint role in assembling ubiquitin chains on APC/C substrates. In the absence of UBE2C and UBE2S, the E2 enzyme UBE2D, which interacts with the APC/C using the same binding site as UBE2C, takes over to support ubiquitination of APC/C subtrates.

The potential of APC/C inhibition for clinical applications is even bigger due to a phenomenon called synthetic lethality. Synthetic lethality is characterised as the setting in which inactivation of either two genes or pathways individually has little effect on cell viability but loss of function of both genes simultaneously leads to cell death. In cancer therapy, the ideal scenario is that cell death occurs only in cancer cells with no or minimum side effect on healthy cells. For instance, cells carrying K-Ras mutations are more sensitive to inhibition of the APC/C resulting in strong accumulation in prometaphase followed by cell death. *K-Ras* mutations are frequently found in various cancers, such as pancreatic, colon and lung cancers and are correlated with poor prognosis. Interestingly, decreased APC/C activity in patients with lung cancer carrying K-Ras mutation is associated with increased survival. Further, cells with defects in sister chromatid cohesion are more sensitive to APC/C inhibition resulting in mitotic arrest followed by cell death. Importantly, cohesion defects are reported in many cancers. Finally, lack of Cdh1 in mouse embryonic fibroblasts results in a dramatically increased sensitivity to etoposide, a topoisomerase II inhibitor. Around 70 % of Cdh1 knockout cells died after treatment with etoposide compared to less than 10 % of control cells. Increase sensitivity to topoisomerase II inhibitors is most probably due to the increased level of Top2*α*, which is an APC/C^{Cdh1} substrate. Similarly, in a B-cell acute leukaemia mouse model and corresponding human cell lines, the lack of Cdh1 results in increased sensitivity to DNA damage due to doxorubicin, topoisomerase II inhibition, or irradiation treatment. Increased radio-sensitivity following Cdh1 downregulation has also been observed in nasopharyngeal carcinoma cells. Similarly, UBE2S downregulation sensitizes HeLa cells to etoposide and doxorubicin resulting in a suppression of cell proliferation.

To conclude, the APC/C is being viewed as a promising therapeutic target for cancer therapy, but, with the few exceptions discussed above, difficult to target by small- to medium-sized drugs - a property which is also referred to as "non druggable". This property applies in particular to the enzyme-enzyme interactions at the APC/C involved in the chain of events leading to susbtrate ubiquitination and thus subsequent proteasomal degradation. These enzyme-enzyme interactions include E1-E2 and, in particular, E2-E3 interactions.
Hence, current therapeutical approaches in cancer therapy target the APC/C indirectly by activating the SAC (e.g. paclitaxel) or by inhibiting proteasomal degradation of ubiquitinated proteins (e.g. Bortezomib). What has been found so far in terms of agents, is generally associated with one or more undesirable properties or side effects. As a consequence, the technical problem underlying the present invention can be seen in the provision of improved means and methods of interfering with the function of the APC/C, in particular in an inhibitory manner. Given the central function of this complex, its involvement in cell division and, accordingly, in proliferation and hyperproliferation, the technical problem underlying the present invention can also be seen in the provision of improved means and methods for the treatment of hyperproliferative conditions and diseases such as cancer.

As is evident from the examples which are part of this disclosure, these technical problems have been solved by the subject-matter of the attached claims.

Accordingly, in a first aspect, the present invention relates to a peptide or peptidomimetic comprising or consisting of the following sequences
(a) X1-X2-X3-X4-X5-X6-X7-X8-X9-X10-X11-X12-X13-X14
   wherein
   a. X1 is an alpha-amino acid, preferably a proteinogenic amino acid, more preferably Glu or Nmglu;
   b. X2 is an alpha-amino acid, preferably a proteinogenic amino acid, or preferably selected from Trp, 2-NaI and 1-NaI;
   c. X3 is an alpha-amino acid, preferably a proteinogenic amino acid, or preferably selected from Trp, 2-NaI, 1-NaI and Pra;
   d. X4 is an alpha-amino acid, preferably a proteinogenic amino acid, or preferably selected from Lys, Cys, D-Cys, Asp, Glu, Dap, Aib and (R)-2-(7'-octenyl)Ala;
   e. X5 is an alpha-amino acid, preferably a proteinogenic amino acid, more preferably Ile or Lys;
   f. X6 is an alpha-amino acid, preferably a proteinogenic amino acid, more preferably Leu or Lys;
   g. X7 is an alpha-amino acid, preferably a proteinogenic amino acid, more preferably Asp or Glu;
   h. X8 is an alpha-amino acid, preferably a proteinogenic amino acid, more preferably Ile or Leu;
   i. X9 is an alpha-amino acid, preferably a proteinogenic amino acid, more preferably Gln;
   j. X10 is an alpha-amino acid, preferably a proteinogenic amino acid, more preferably Arg;
   k. X11 is an alpha-amino acid, preferably a proteinogenic amino acid, or preferably Asp, Glu, Cys, D-Cys, Aib or (S)-2-(4'-pentenyl)Ala;
   l. X12 is an alpha-amino acid, preferably a proteinogenic amino acid, more preferably Arg;
   m. X13 is an alpha-amino acid, preferably a proteinogenic amino acid, more preferably Arg;
   n. X14 is an alpha-amino acid, preferably a proteinogenic amino acid, more preferably Ala;
(b) X1-X2-X3-X4-X5-X6-X7-X8-X9-X10-X11-X12
   wherein
   a. X1 is an amino acid, preferably an alpha-amino acid, more preferably a proteinogenic amino acid, or preferably selected from Arg, Phe(2-guanidino) and Phe(3-guanidino);
   b. X2 is an alpha-amino acid, preferably a proteinogenic amino acid, or preferably selected from Trp, Phe, Tyr, Ile, Leu, Cys, L-propargylglycine (Pra), L-homopropargylglycine (Hpg), allylglycine (Agl), prenylglycine (Pre), crotylglycine (Crt) and (S)-2-amino-4-bromobutyric acid;
   c. X3 is an alpha-amino acid, preferably a proteinogenic amino acid, or preferably selected from Ser, His and Dab;
   d. X4 is an alpha-amino acid, preferably a proteinogenic amino acid, or preferably selected from Trp, Cys, Pra, Hpg, Agl, Pre, Crt and (S)-2-amino-4-bromobutyric acid;
   e. X5 is an alpha-amino acid, preferably a proteinogenic amino acid, or preferably Gly or D-Ala;
   f. X6 is an alpha-amino acid, preferably a proteinogenic amino acid, or preferably Pro or D-Pro;
   g. X7 is an alpha-amino acid, preferably a proteinogenic amino acid, or preferably selected from Glu, Arg, Trp, Cit, Orn and pSer;
   h. X8 is an alpha-amino acid, preferably a proteinogenic amino acid, more preferably Thr;
   i. X9 is an alpha-amino acid, preferably a proteinogenic amino acid, or preferably selected from Phe, Cys, Agl, Pre, Crt, 3-azido-L-alanine (Dap(N3)), (S)-2-amino-4-azido-butyric acid (2Abu(y-N3), 5-azido-noravline (Nva(δ-N3) and (S)-2-amino-4-bromobutyric acid;
   j. X10 is an alpha-amino acid, more preferably a proteinogenic amino acid, or preferably selected from Trp, 2-NaI and 1-NaI;
   k. X11 is an alpha-amino acid, preferably a proteinogenic amino acid, or preferably selected from Trp, Phe, Tyr, Ile, Leu, Cys, (S)-2-amino-4-bromobutyric acid, Dap(N3), 2Abu(γ-N3), Nva(δ-N3), Agl and Crt;
   l. X12 is an alpha-amino acid, preferably a proteinogenic amino acid, or preferably selected from Arg, Phe(3-guanidino), Phe(4-guanidino), Cit and Orn;
(c) X1-X2-X3-X4-X5-X6-X7-X8-X9-X10-X11-X12-X13-X14-X15-X16
   wherein
   a. X1 is an alpha-amino acid, preferably a proteinogenic amino acid, more preferably Thr;
   b. X2 is an alpha-amino acid, preferably a proteinogenic amino acid, or preferably selected from Ser, His and hSer;
   c. X3 is an alpha-amino acid, preferably a proteinogenic amino acid, or preferably selected from Cys, Pra, Hpg, Agl, Crt and (S)-2-amino-4-bromobutyric acid;
   d. X4 is an alpha-amino acid, preferably a proteinogenic amino acid, more preferably Asp;
   e. X5 is an alpha-amino acid, preferably a proteinogenic amino acid, more preferably Asp;
   f. X6 is an alpha-amino acid, preferably a proteinogenic amino acid, more preferably Pro;
   g. X7 is an alpha-amino acid, preferably a proteinogenic amino acid, or preferably Glu, Trp or Aad;
   h. X8 is an alpha-amino acid, preferably a proteinogenic amino acid, more preferably Thr;
   i. X9 is an alpha-amino acid, preferably a proteinogenic amino acid, or preferably selected from Trp, 2-NaI and 1-NaI;
   j. X10 is a group consisting of any natural amino acid, preferably a proteinogenic amino acid, more preferably Arg;
   k. X11 is alpha-amino acid, preferably a proteinogenic amino acid, or preferably selected from Trp, 2-NaI and 1-NaI;
   l. X12 is an alpha-amino acid, preferably a proteinogenic amino acid, more preferably Asn;
   m. X13 is an alpha-amino acid, preferably a proteinogenic amino acid, or preferably selected from Cys, (S)-2-amino-4-bromobutyric acid, Dap(N3), 2Abu(γ-N3), Nva(δ-N3), Agl and Crt;
   n. X14 is a group consisting of any natural amino acid, preferably a proteinogenic amino acid, more preferably Gln;
   o. X15 is alpha-amino acid, preferably a proteinogenic amino acid, or preferably selected from Arg, Phe(3-guanidino), Phe(4-guanidino) and Cit;
   p. X16 is an alpha-amino acid, preferably a proteinogenic amino acid, more preferably Val;
   wherein a side chain of X3 is covalently connected to a side chain of X13;
(d) X1-X2-X3-X4-X5-X6-X7-X8-X9-X10-X11-X12-X13-X14-X25-X16-X17
   wherein
   a. X1 is an alpha-amino acid, preferably a proteinogenic amino acid, more preferably Gly;
   b. X2 is an alpha-amino acid, preferably a proteinogenic amino acid, more preferably Asp;
   c. X3 is an alpha-amino acid, preferably a proteinogenic amino acid, or preferably Thr or hSer;
   d. X4 is an alpha-amino acid, preferably a proteinogenic amino acid, or preferably selected from Cys, Pra, Hpg, Agl, Crt and (S)-2-amino-4-bromobutyric acid;
   e. X5 is an alpha-amino acid, preferably a proteinogenic amino acid, more preferably Asp;
   f. X6 is an alpha-amino amino acid, preferably a proteinogenic amino acid, more preferably Asp;
   g. X7 is an alpha-amino amino acid, preferably a proteinogenic amino acid, more preferably Pro;
   h. X8 is an alpha-amino acid, preferably a proteinogenic amino acid, or preferably Glu, Trp or Aad;
   i. X9 is an alpha-amino acid, preferably a proteinogenic amino acid, more preferably Thr;
   j. X10 is an alpha-amino acid, preferably a proteinogenic amino acid, or preferably selected from Trp, 2-NaI and 1-NaI;
   k. X11 is an alpha-amino acid, preferably a proteinogenic amino acid, more preferably Arg;
   l. X12 is an alpha-amino acid, preferably a proteinogenic amino acid, or preferably selected from Trp, 2-NaI and 1-NaI;
   m. X13 is an alpha-amino acid, preferably a proteinogenic amino acid, more preferably Asn;
   n. X14 is an alpha-amino acid, preferably a proteinogenic amino acid, or preferably selected from Cys, (S)-2-amino-4-bromobutyric acid, Dap(N3), 2Abu(γ-N3), Nva(δ-N3), Pra, Agl and Crt;
   o. X15 is an alpha-amino acid, preferably a proteinogenic amino acid, more preferably Gln;
   p. X16 is an alpha-amino acid, preferably a proteinogenic amino acid, or preferably Arg or Cit;
   q. X17 is an alpha-amino acid, preferably a proteinogenic amino acid, more preferably His;
   wherein a side chain of X4 is covalently connected to a side chain of X14;
(e) X1-X2-X3-X4-X5-X6-X7-X8-X9-X10-X11-X12-X13-X14
   wherein
   a. X1 is an alpha-amino acid, preferably a proteinogenic amino acid, or preferably selected from Cys, Pra, Hpg, Agl, Crt and (S)-2-amino-4-bromobutyric acid;
   b. X2 is an alpha-amino acid, preferably a proteinogenic amino acid, more preferably Glu;
   c. X3 is an alpha-amino acid, preferably a proteinogenic amino acid, or preferably Ala or Aib;
   d. X4 is an alpha-amino acid, preferably a proteinogenic amino acid, more preferably Gly;
   e. X5 is an alpha-amino acid, preferably a proteinogenic amino acid, more preferably Pro;
   f. X6 is an alpha-amino acid, preferably a proteinogenic amino acid, more preferably Ser;
   g. X7 is an alpha-amino acid, preferably a proteinogenic amino acid, more preferably Gln;
   h. X8 is an alpha-amino acid, preferably a proteinogenic amino acid, or preferably selected from Trp, 2-NaI and 1-NaI;
   i. X9 is an alpha-amino acid, preferably a proteinogenic amino acid, more preferably selected from His, Asn and Gln;
   j. X10 is an alpha-amino acid, preferably a proteinogenic amino acid, or preferably selected from Cys, (S)-2-amino-4-bromobutyric acid, Dap(N3), 2Abu(γ-N3), Nva(δ-N3), Agl and Crt;
   k. X11 is an alpha-amino acid, preferably a proteinogenic amino acid, more preferably Thr or Glu;
   l. X12 is an alpha-amino acid, preferably a proteinogenic amino acid, or preferably selected from Trp, 2-NaI and 1-NaI;
   m. X13 is an alpha-amino acid, preferably a proteinogenic amino acid, or preferably Arg or Cit;
   n. X14 is an alpha-amino acid, preferably a proteinogenic amino acid, more preferably Tyr;
   wherein a side chain of X1 is covalently connected to a side chain of X10;
(f) X1-X2-X3-X4-X5-X6-X7-X8-X9-X10-X11-X12-X13-X14
   wherein
   a. X1 is an alpha-amino acid, preferably a proteinogenic amino acid, more preferably Leu or Thr;
   b. X2 is an alpha-amino acid, preferably a proteinogenic amino acid, or preferably selected from Trp, Phe and 2-NaI;
   c. X3 is an alpha-amino acid, preferably a proteinogenic amino acid, more preferably Leu;
   d. X4 is an alpha-amino acid, preferably a proteinogenic amino acid, more preferably Trp;
   e. X5 is an alpha-amino acid, preferably a proteinogenic amino acid, more preferably Lys;
   f. X6 is an alpha-amino acid, preferably a proteinogenic amino acid, or preferably selected from Ala, Trp, D-Leu, D-Thr and D-Glu;
   g. X7 is an alpha-amino acid, preferably a proteinogenic amino acid, more preferably Glu;
   h. X8 is an alpha-amino acid, preferably a proteinogenic amino acid, more preferably Trp;
   i. X9 is an alpha-amino acid, preferably a proteinogenic amino acid, more preferably Glu or Thr;
   j. X10 is an alpha-amino acid, preferably a proteinogenic amino acid, more preferably Lys;
   k. X11 is an alpha-amino acid, preferably a proteinogenic amino acid, or preferably selected from Arg, Ser, Cit and hSer;
   l. X12 is an alpha-amino acid, preferably a proteinogenic amino acid, more preferably Lys or Pra;
   m. X13 is an alpha-amino acid, preferably a non-proteinogenic amino acid, or preferably selected from D-Pro, allylamine, cysteamine and Orn;
   n. X14 is an alpha-amino acid, preferably a proteinogenic amino acid, more preferably selected from Pro, Aib or an acid comprising 4-pentenoyl or 3-bromopropionyl moiety;
      wherein
      (1) the alpha-amino group of X1 is bound to a carboxy group of X14; or
      (2) if X13 is Orn, the alpha-amino group of X1 is bound to the alpha-carboxy group of X13; and a side chain of X13 is bound to the alpha-carboxy group of X12;
(g) X1-X2-X3-X4-X5-X6-X7-X8-X9-X10-X11-X12-X13-X14
   wherein
   a. X1 is an alpha-amino acid, preferably a proteinogenic amino acid, more preferably Arg or Nmarg;
   b. X2 is an alpha-amino acid, preferably a proteinogenic amino acid, or preferably selected from Trp, Glu, 2-NaI and 1-NaI;
   c. X3 is an alpha-amino acid, preferably a proteinogenic amino acid, more preferably selected from Pro, Trp and Glu;
   d. X4 is an alpha-amino acid, preferably a proteinogenic amino acid, or preferably selected from Gln, Leu, Arg and Pra;
   e. X5 is an alpha-amino acid, preferably a proteinogenic amino acid, or preferably selected from Lys, Glu, Ser, Lys(N3), (S)-2-(4'-pentenyl)Ala and Aib;
   f. X6 is an alpha-amino acid, preferably a proteinogenic amino acid, more preferably Ile or Lys;
   g. X7 is an alpha-amino acid, preferably a proteinogenic amino acid, or preferably selected from Lys, Trp, 2-NaI and 1-NaI;
   h. X8 is an alpha-amino acid, preferably a proteinogenic amino acid, more preferably Asp or Glu;
   i. X9 is an alpha-amino acid, preferably a proteinogenic amino acid, or preferably selected from Asp, Lys, Ser, (S)-2-(4'-pentenyl)Ala, Pra, D-Pra and Aib;
   j. X10 is an alpha-amino acid, preferably a proteinogenic amino acid, more preferably Gln;
   k. X11 is an alpha-amino acid, preferably a proteinogenic amino acid, more preferably Arg or Val;
   l. X12 is an alpha-amino acid, preferably a proteinogenic amino acid, more preferably Arg or Thr;
   m. X13 is an alpha-amino acid, preferably a proteinogenic amino acid, more preferably Arg or Trp;
   n. X14 is an alpha-amino acid, preferably a proteinogenic amino acid, more preferably Trp or Arg;
(h) X1-X2-X3-X4-X5-X6-X7-X8-X9-X10-X11-X12-X13-X14
   wherein
   a. X1 is an alpha-amino acid, preferably a proteinogenic amino acid, more preferably Glu or Nmglu;
   b. X2 is an alpha-amino acid, preferably a proteinogenic amino acid, or preferably selected from Trp, 2-NaI and 1-NaI;
   c. X3 is an alpha-amino acid, preferably a proteinogenic amino acid, more preferably Lys, Glu, Ser, Lys(N3) or (S)-2-(4'-pentenyl)Ala;
   d. X4 is an alpha-amino acid, preferably a proteinogenic amino acid, more preferably selected from Trp, 2-NaI and 1-NaI;
   e. X5 is an alpha-amino acid, preferably a proteinogenic amino acid, more preferably Ile, Trp or Arg;
   f. X6 is an alpha-amino acid, preferably a proteinogenic amino acid, more preferably selected from Ile, Leu and Lys;
   g. X7 is an alpha-amino acid, preferably a proteinogenic amino acid, more preferably Asp, Lys, Ser, Pra, D-Pra or (S)-2-(4'-pentenyl)Ala;
   h. X8 is an alpha-amino acid, preferably a proteinogenic amino acid, more preferably Lys, Glu, Ser, Lys(N3) or (S)-2-(4'-pentenyl)Ala;
   i. X9 is an alpha-amino acid, preferably a proteinogenic amino acid, more preferably Ile;
   j. X10 is an alpha-amino acid, preferably a proteinogenic amino acid, more preferably Gln;
   k. X11 is an alpha-amino acid, preferably a proteinogenic amino acid, more preferably Ala or Val;
   l. X12 is an alpha-amino acid, preferably a proteinogenic amino acid, more preferably Asp, Lys, Ser, Pra, D-Pra or (S)-2-(4'-pentenyl)Ala;
   m. X13 is an alpha-amino acid, preferably a proteinogenic amino acid, more preferably selected from Arg, Lys and Trp;
   n. X14 is an alpha-amino acid, preferably a proteinogenic amino acid, more preferably Ala or Trp;
   wherein a side chain of X3 is covalently connected to a side chain of X7; and
   wherein a side chain of X8 is covalently connected to a side chain of X12;
   or
(i) X1-X2-X3-X4-X5-X6-X7-X8-X9-X10-X11-X12-X13-X14-X15
   wherein
   a. X1 is an alpha-amino acid, preferably a proteinogenic amino acid, more preferably Glu, Arg, Nmglu or Nmarg;
   b. X2 is an alpha-amino acid, preferably a proteinogenic amino acid, or preferably selected from Trp, 2-NaI and 1-NaI;
   c. X3 is an alpha-amino acid, preferably a proteinogenic amino acid, or preferably selected from Trp, 2-NaI, 1-NaI and Glu;
   d. X4 is an alpha-amino acid, preferably a proteinogenic amino acid, more preferably Lys, Glu, Ser, Lys(N3) or (S)-2-(4'-pentenyl)Ala;
   e. X5 is an alpha-amino acid, preferably a proteinogenic amino acid, more preferably Ile or Arg;
   f. X6 is an alpha-amino acid, preferably a proteinogenic amino acid, more preferably selected from Ile, Leu and Lys;
   g. X7 is an alpha-amino acid, preferably a proteinogenic amino acid, more preferably Lys;
   h. X8 is an alpha-amino acid, preferably a proteinogenic amino acid, more preferably Asp, Lys, Ser, Pra, D-Pra or (S)-2-(4'-pentenyl)Ala;
   i. X9 is an alpha-amino acid, preferably a proteinogenic amino acid, more preferably Ile;
   j. X10 is an alpha-amino acid, preferably a proteinogenic amino acid, more preferably Gln;
   k. X11 is an alpha-amino acid, preferably a proteinogenic amino acid, more preferably Lys, Glu, Ser, Lys(N3) or (S)-2-(4'-pentenyl)Ala;
   l. X12 is an alpha-amino acid, preferably a proteinogenic amino acid, more preferably Trp or Ile;
   m. X13 is an alpha-amino acid, preferably a proteinogenic amino acid, more preferably selected from Arg, Lys and Trp;
   n. X14 is an alpha-amino acid, preferably a proteinogenic amino acid, more preferably Arg or Trp;
   o. X15 is an alpha-amino acid, preferably a proteinogenic amino acid, more preferably Asp, Lys, Ser, Pra, D-Pra or (S)-2-(4'-pentenyl)Ala;
   wherein a side chain of X4 is covalently connected to a side chain of X8; and
   wherein a side chain of X11 is covalently connected to a side chain of X15.

The present inventors surprisingly found short peptides and peptidomimetics (in the following also denoted "compounds of the invention" or "compounds") to be capable of interfering with APC/C function, which is evidenced by the Examples. In particular, said compounds of the invention bind to the APC11 subunit of APC/C which is an E3 ligase. Such binding is in a manner which competes with the binding of APC11 to E2 enzymes. By interfering with the recruitment of E2 enzymes by the E3 ligase comprised in APC/C the said compounds provide for inhibition of mitosis, which in turn opens a new avenue for treating and preventing hyperproliferative diseases.

A preferred APC11 is human APC11. This applies to all proteins identified in this disclosure, i.e., a preferred species of origin is human.

The pathway leading to the ubiquitination and subsequent degradation of target proteins is well known in the art and involves enzymes generally designated as E1, E2 and E3 (said nomenclature being used above as well as in the introductory part of this disclosure).

Moreover, the inventors provided a plurality of classes of compounds which, while exhibiting similarities in terms of primary structure, preferably adopt different secondary structures. These different secondary structures are preferably defined in terms of the canonical secondary structure elements (helix, sheet, or combination of those with turns) as they are known to occur in polypeptides and proteins. The preferred secondary structures of the peptides and peptidomimetics are subject of a preferred embodiment disclosed further below. Yet, and despite differences in secondary structure, all compounds of the invention share physicochemical features in the three-dimensional space which are required for APC/C binding and inhibition. These physicochemical properties are features of the primary sequence of the compounds of the invention.

Indeed, the overarching property of the compounds of the invention is their capability to interfere with APC/C function. Given the central role of APC/C in cell division and proliferation, this opens the door to a host of therapeutic applications which are described in more detail below. Of note, the Examples enclosed herewith provide ample proof of this capability for a host of peptides and peptidomimetics implementing the subject-matter of this invention.

Moreover, the inventors did not only provide proof of the compounds' capability to slow down or stop cell division or induce apoptosis, but furthermore provide optimized compounds which are successfully delivered to cells. Also, this is documented in the Examples. Of note, optimization of delivery, while being preferred, is not a compulsory feature. In structural terms, delivery is optimized by N- and or C-terminal modifications. On the other hand, APC11 binding is governed by the sequence of amino acids.

Indeed, the compounds of the invention are composed of residues or building blocks, which generally are amino acids. These residues are designated by X and a number indicating their position within the sequence, e.g., X1, X2 etc. or generally Xi.

In terms of structure, the building blocks of the peptides are preferably proteinogenic amino acids; see Table 1 below.

**Table 1. Proteogenic amino acids**

| Amino Acid | One letter code | Three letter code | Amino Acid | One letter code | Three letter code |
|---|---|---|---|---|---|
| L-alanine | A | Ala | L-leucine | L | Leu |
| L-arginine | R | Arg | L-lysine | K | Lys |
| L-asparagine | N | Asn | L-methionine | M | Met |
| L-aspartic acid | D | Asp | L-phenylalanine | F | Phe |
| L-cysteine | C | Cys | L-proline | P | Pro |
| L-glutamine | Q | Gin | L-serine | S | Ser |
| L-glutamic acid | E | Glu | L-threonine | T | Thr |
| glycine | G | Gly | L-tryptophan | W | Trp |
| L-histidine | H | His | L-tyrosine | Y | Tyr |
| L-isoleucine | I | Ile | L-valine | V | Val |

Yet, for the purpose of optimizing and fine-tuning APC/C binding and inhibition and/or delivery, the inventors also developed peptidomimetics. In such peptidomimetics, non-proteinogenic amino acids and/or non-naturally occurring amino acids take the place of proteinogenic amino acids at certain positions in the amino acid sequence of the compounds of the invention. Such amino acids are also termed "non-natural" or "unusual" herein and are listed in Table 2 below.

**Table 2. Non-natural or unusual amino acids**

| Unusual Amino Acids | Code | Unusual Amino Acids | Code |
|---|---|---|---|
| D-alanine | D-Ala | D-lysine | D-Lys |
| D-arginine | D-Arg | D-methionine | D-Met |
| D-asparagine | D-Asn | D-phenylalanine | D-Phe |
| D-aspartic acid | D-Asp | D-proline | D-Pro |
| D-cysteine | D-Cys | D-serine | D-Ser |
| D-glutamine | D-Gln | D-threonine | D-Thr |
| D-glutamic acid | D-Glu | D-tryptophan | D-Trp |
| D-histidine | D-His | D-tyrosine | D-Tyr |
| D-isoleucine | D-Ile | D-valine | D-Val |
| D-leucine | D-Leu | | |
| α-aminobutyric acid | Abu | α-aminoisobutyric acid | Aib |
| γ-aminobutyric acid | GABA | Dehydroalanine | Dha |
| (S)-2-amino-4-azido-butyric acid (L-azidohomoalanine) | 2Abu(γ-N3) | (R)-2-amino-4-azido-butyric acid (D-azidohomoalanine) | D-2Abu(γ-N3) |
| (S)-2-amino-4-bromobutyric acid | 2Abu(γ-Br) | (R)-2-amino-4-bromobutyric acid | D-2Abu(γ-Br) |
| 1-aminocyclopentanecarboxyli c acid | Ac₅C | 4-amino-piperidine-4-carboxylic acid | 4-Pip |
| L-homopropargylglycine | Hpg | D-homopropargylglycine | D-Hpg |
| α-methyl-L-alanine | mAla | α-methyl-D-alanine | D-mAla |
| *N*-methyl-L-alanine | Nmala | *N*-methyl-D-alanine | D-Nmala |
| (*S*)-2-(4'-pentenyl)Ala | pentAla | (*R*)-2-('-pentenyl)Ala | D-pentAla |
| (*S*)-2-(7'-octenyl)Ala | octAla | (*R*)-2-(7'-octenyl)Ala | D-octAla |
| 3-azido-L-alanine | Dap(N3) | 3-azido-D-alanine | D-Dap(N3) |
| 1-naphthyl-L-alanine | 1-NaI | 1-naphthyl-D-alanine | D-1-NaI |
| α-methyl-1-naphthyl-L-alanine | ml-NaI | α-methyl-1-naphthyl-D-alanine | D-m1-NaI |
| 2-naphthyl-L-alanine | 2-NaI | 2-naphthyl-D-alanine | D-2-NaI |
| α-methyl-2-naphthyl-L-alanine | m2-NaI | α-methyl-2-naphthyl-D-alanine | D-m2-NaI |
| β-2-pyridyl-L-alanine | 2Pal | β-2-pyridyl-D-alanine | D-2PaI |
| β-2-thienyl-L-alanine | 2Tal | β-2-thienyl-D-alanine | D-2Tal |
| β-3-pyridyl-L-alanine | 3Pal | β-3-pyridyl-D-alanine | D-3Pal |
| *N*-(2,4-dimethoxybenzyl)-L-alanine | N(Dmob)Ala | *N*-(2,4-dimethoxybenzyl)-D-alanine | D-N(Dmob)Ala |
| β-*tert*-butyl-L-alanine | Tba | β-tert-Butyl-D-alanine | D-Tba |
| β-chloro-L-alanine | 3Clal | β-Chloro-D-alanine | D-3Clal |
| β-cyano-1-alanine | 3CNal | β-Cyano-D-alanine | D-3CNal |
| β-cyclohexyl-L-alanine | Cha | β-Cyclohexyl-D-alanine | D-Cha |
| α-methyl-β-cyclohexyl-L-alanine | mCha | α-methyl-β-cyclohexyl-D-alanine | D-mCha |
| *N*-methyl-β-cyclohexyl-L-alanine | NmCha | *N*-methyl-β-cyclohexyl-D-alanine | D-NmCha |
| β-cyclopentyl-L-alanine | Cpa | β-cyclopentyl-D-alanine | D-Cpa |
| α-methyl-β-cyclopentyl-L-alanine | mCpa | α-methyl-β-cyclopentyl-D-alanine | D-mCpa |
| β-cyclopropyl-L-alanine | Cppa | β-Cyclopropyl-D-alanine | D-Cppa |
| β-iodo-L-alanine | 3Ial | β-iodo-D-alanine | D-3Ial |
| β,β-diphenyl-L-alanine | Ppal | β,β-diphenyl-D-alanine | D-Ppal |
| β-(7-methoxy-coumarin-4-yl)-L-alanine | Mcal | β-(7-methoxy-coumarin-4-yl)-D-alanine | D-Mcal |
| β-(1-piperazinyl)-L-alanine | 1Pza | β-(1-piperazinyl)-D-alanine | D-1Pza |
| β-(2-quinolyl)-L-alanine | 2Qal | β-(2-quinolyl)-D-alanine | D-2Qal |
| β-(2thiazolyl)-L-alanine | 2Tza | β-(2-thiazolyl)-D-alanine | D-2Tza |
| β-(3-benzothienyl)-L-alanine | 3Btza | β-(3-benzothienyl)-D-alanine | D-3Btza |
| α-methyl-L-arginine | mArg | α-methyl-D-arginine | D-mArg |
| *N*-methyl-L-arginine | Nmarg | *N*-methyl-D-arginine | D-Nmarg |
| L-citrulline | Cit | D-citrulline | D-Cit |
| L-thiocitruline | Tcit | D-thiocitruline | D-Tcit |
| L-homoarginine | hArg | D-homoarginine | D-hArg |
| L-homocitruline | hCit | D-homocitruline | D-hCit |
| L-homothiocitruline | hTcit | D-homothiocitruline | D-hTcit |
| L-albizziine | Abz | D-albizziine | D-Abz |
| α-methyl-L-asparagine | mAsn | α-methyl-D-asparigine | D-mAsn |
| *N*-methyl-L-asparigine | NmAsn | *N*-methyl-D-asparigine | D-Nmasn |
| α-methyl-L-aspartic acid | mAsp | α-methyl-D-aspartic acid | D-mAsp |

| *N*-methyl-L-aspartic acid | Nmasp | *N*-methyl-D-aspartic acid | D-Nmasp |
|---|---|---|---|
| L-azetidine-2-carboxylic acid | Aze | D-azetidine-2-carboxylic acid | D-Aze |
| α-methyl-L-cysteine | mCys | α-methyl-D-cysteine | D-mCys |
| *N*-methyl-L-cysteine | NmCys | *N*-methyl-D-cysteine | D-Nmcys |
| L-homocysteine | hCys | D-homocysteine | D-hCys |
| S-ethyl-L-cysteine | Cys(Et) | S-ethyl-L-cysteine | D-Cys(Et) |
| L-thiosine | Cys(EtNH₂) | D-thiosine | D-Cys(EtNH₂) |
| S-2-hydroxtethyl-L-cysteine | Cys(EtOH) | S-2-hydroxtethyl-D-cysteine | D-Cys(EtOH) |
| S-propyl-L-cysteine | Cys(Pr) | S-propyl-L-cysteine | D-Cys(Pr) |
| α-methyl-L-glutamine | mGln | α-methyl-D-glutamine | D-mGln |
| *N*-methyl-L-glutamine | Nmgln | *N*-methyl-D-glutamine | D-Nmgln |
| α-methyl-L-glutamic acid | mGlu | α-methyl-D-glutamic acid | D-mGlu |
| *N*-methyl-L-glutamic acid | Nmglu | *N*-methyl-D-glutamic acid | D-Nmglu |
| L-α-aminosuberic acid | Asu | D-α-aminosuberic acid | D-Asu |
| γ-carboxy-L-glutamic acid | Gla | γ-carboxy-D-glutamic acid | D-Gla |
| L-α-aminoadipic acid (L-homoglutamic acid) | Aad | D-α-aminoadipic acid (D-homoglutamic acid) | D-Aad |
| L-pyroglutamic acid | Pyr | D-pyroglutamic acid | D-Pyr |
| L-propargylglycine | Pra | D-propargylglycine | D-Pra |
| L-allylglycine | Agl | D-allylglycine | D-Agl |
| L-prenylglycine | Pre | D-prenylglycine | D-Pre |
| L-crotylglycine | Crt | D-crotylglycine | D-Crt |
| Octyl-L-glycine | Ogl | Octyl-D-glycine | D-Ogl |
| L-cyclohexylglycine | Chg | D-cyclohexylglycine | D-Chg |
| L-decyline | Dec | D-decyline | D-Dec |
| L-cyclopentylglycine | Cpg | D-cyclopentylglycine | D-Cpg |
| α-*tert*-butylglycine | Tle | α-*tert*-butyl-D-glycine | D-Tle |
| L-phenylglycine | Phg | D-phenylglycine | D-Phg |
| *N*-methyl-L-phenylglycine | Nmphg | *N*-methyl-D-phenylglycine | D-Nmphg |
| *N*-*tert*-butylglycine | Ntbg | *N*-( 1-methylethyl)glycine | Nmeg |
| *N*-benzylglycine | Nphe | *N*-(4-aminobutyl)glycine | N4abgly |
| *N*-(2-aminoethyl)glycine | N2aegly | *N*-(3-aminopropyl)glycine | N3apgly |
| *N*-(aminocarbonyl)glycine (hydantoic acid) | Hyd | | |
| sarcosine (N-methylglycine) | Sar | phenylsulfonylsarcosine | PhSO₂Sar |
| *N*-(2-amino-2-oxoethyl)glycine | Nasngly | *N*-(carboxymethyl)glycine | Naspgly |
| *N*-(3-amino-3-oxopropyl)glycine | Nglngly | *N*-(carboxyethyl) glycine | Nglugly |
| *N*-(carboxy)glycine | Ncbgly | *N-*(3-methylcyclobutyl)glycine | Nmcbgly |
| α-methyl-L-histidine | mHis | α-methyl-D-histidine | D-mHis |
| *N*-methyl-L-histidine | Nmhis | *N*-methyl-D-histidine | D-Nmhis |
| 1-methyl-L-histidine | His(τ-Me) | 1-methyl-D-histidine | D-His(τ-Me) |
| 3-methyl-L-histidine | His(π-Me) | 3-methyl-D-histidine | D-His(π-Me) |
| 2,5-diiodo-L-histidine | His(2,5-I) | 2,5-diiodo-D-histidine | D-His(2,5-I) |
| 2-mercapto-L-histidine | His(C=S) | 2-mercapto-D-histidine | D-His(C=S) |
| α-methyl-L-isoleucine | mIle | α-methyl-D-isoleucine | D-mIle |
| *N*-methyl-L-isoleucine | Nmile | *N*-methyl-D-isoleucine | D-Nmile |
| L-allo-isoleucine | Ail | D-allo-isoleucine | D-Ail |
| *N*-methyl-L-allo-isoleucine | Nmaile | *N*-methyl-D-allo-isoleucine | D-Nmaile |
| α-methyl-L-leucine | mLeu | α-methyl-D-leucine | D-mLeu |
| *N*-methyl-L-leucine | Nmleu | *N*-methyl-D-leucine | D-Nmleu |
| L-homoleucine | hLeu | D-homoleucine | D-hLeu |
| L-norleucine | Nle | D-norleucine | D-Nle |
| α-methyl-L-norleucine | mNle | α-methyl-D-norleucine | D-mNle |
| *N*-methyl-L-norleucine | Nmnle | *N*-methyl-D-norleucine | D-Nmnle |
| 4,5-dehydro-L-leucine | dhLeu | 4,5-dehydro-D-leucine | D-dhLeu |
| α-methyl-L-lysine | mLys | α-methyl-D-lysine | D-mLys |
| *N*-methyl-L-lysine | Nmlys | *N*-methyl-D-lysine | D-Nmlys |
| L-homolysine | hLys | D-homolysine | D-hLys |
| L-azidolysine | Lys(N3) | D-azidolysine | D-Lys(N3) |
| L-ornithine | Orn | D-ornithine | D-Orn |
| α-methyl-L-ornithine | mOrn | α-methyl-D-ornithine | D-mOrn |
| *N*-methyl-L-ornithine | Nmorn | *N*-methyl-D-ornithine | D-Nmorn |
| L-diaminobutyric acid | Dab | D-diaminobutyric acid | D-Dab |
| L-γ-azidohomoalanine | γ-Aha | D-γ-azidohomoalanine | D-γ-Aha |
| 3-amino-L-alanine (L-(2,3)-diaminopropionic acid) | Dap | 3-amino-D-alanine (D-(2,3)-diaminopropionic acid) | D-Dap |
| α-methyl-L-methionine | mMet | α-methyl-D-methionine | D-mMet |
| *N*-methyl-L-methionine | Nmmet | *N*-methyl-D-methionine | D-Nmmet |
| L-homomethionine | hMet | D-homomethionine | D-hMet |
| L-methionine sulfoxide | Met(O) | D-methionine sulfoxide | D-Met(O) |
| L-methionine sulfone | Met(O₂) | D-methionine sulfone | D-Met(O₂) |
| L-penicillamine | Pen | D-penicillamine | D-Pen |
| α-methyl-L-pencinillamine | mPen | α-methyl-D-pencinillamine | D-mPen |
| α-methyl-L-phenylalanine | mPhe | α-methyl-D-phenylalanine | D-mPhe |
| *N*-methyl-L-phenylalanine | Nmphe | *N*-methyl-D-phenylalanine | D-Nmphe |
| 4-amino-L-phenylalanine | Phe(*p*NH₂) | 4-amino-D-phenylalanine | D-Phe(*p*NH₂) |
| 4-azido-L-phenylalanine | Phe(*p*N₃) | 4-azido-D-phenylalanine | D-Phe(*p*N₃) |
| 4-nitro-L-phenylalanine | Phe(*p*NO₂) | 4-nitro-D-phenylalanine | D-Phe(*p*NO₂) |
| 4-methyl-L-phenylalanine | Phe(*p*Me) | 4-methyl-D-phenylalanine | D-Phe(pMe) |
| 4-benzoyl-L-phenylalanine | Bpa | 4-benzoyl-D-phenylalanine | D-Bpa |
| 4-phenyl-L-phenylalanine | Bip | 4-phenyl-D-phenylalanine | D-Bip |
| 4-(dimethylamino)-L-phenylalanine | Phe(4-NMe₂) | 4-(dimethylamino)-D-phenylalanine | D-Phe(4-NMe₂) |
| 4-(diethylamino)-L-phenylalanine | Phe(4-NEt₂) | 4-(diethylamino)-D-phenylalanine | D-Phe(4-NEt₂) |
| 4-methoxy-L-phenylalanine | Tyr(Me) | 4-methoxy-D-phenylalanine | D-Tyr(Me) |
| 4-ethoxy-L-phenylalanine | Tyr(Et) | 4-ethoxy-D-phenylalanine | D-Tyr(Et) |
| 4-tert-butyl-L-phenylalanine | Tbp | 4-tert-butyl-D-phenylalanine | D-Tbp |
| 4-carboxy-L-phenylalanine | Phe(*p*CO₂H) | 4-carboxy-D-phenylalanine | D-Phe(*p*CO₂H) |
| 4-cyano-L-phenylalanine | Phe(*p*CN) | 4-cyano-D-phenylalanine | D-Phe(*p*CN) |
| 4-bromo-L-phenylalanine | Phe(*p*Br) | 4-bromo-D-phenylalanine | D-Phe(*p*Br) |
| 4-chloro-L-phenylalanine | Phe(*p*Cl) | 4-chloro-D-phenylalanine | D-Phe(*p*Cl) |
| 4-iodo-L-phenylalanine | Phe(*p*I) | 4-iodo-D-phenylalanine | D-Phe(*p*I) |
| 4-fluoro-L-phenylalanine | Phe(*p*F) | 4-fluoro-D-phenylalanine | D-Phe(*p*F) |
| pentafluoro-L-phenylalanine | Phe(*p*F) | pentafluoro- D-phenylalanine | D-Phe(*p*F) |
| 2,6-difluoro-L-phenylalanine | Phe(2,6-F) | 2,6-difluoro-D-phenylalanine | D-Phe(2,6-F) |
| 3-fluoro-L-phenylalanine | Phe(*m*F) | 3-fluoro-D-phenylalanine | D-Phe(*m*F) |
| 3,4-dichloro-L-phenylalanine | Phe(3,4-F) | 3,4-dichloro-D-phenylalanine | D-Phe(3,4-F) |
| 4-phosphono-L-phenylalanine | Ppa | 4-phosphono-D-phenylalanine | D-Ppa |
| 4-sulfomethyl-L-phenylalanine | Phe(*p*CH₂SO₃H) | 4-sulfomethyl-D-phenylalanine | D-Phe(*p*CH₂SO₃H) |
| 2-guanidino-L-phenylalanine | Phe(*o-*N=CH(NH₂)₂) | 2-guanidino-D-phenylalanine | D-Phe(*o-*N=CH(NH₂)₂) |
| 3-guanidino-L-phenylalanine | Phe(*m-*N=CH(NH₂)₂) | 3-guanidino-D-phenylalanine | D-Phe(*m-*N=CH(NH₂)₂) |
| 4-guanidino-L-phenylalanine | Phe(*p-*N=CH(NH₂)₂) | 4-guanidino-D-phenylalanine | D-Phe(p-N=CH(NH₂)₂) |
| L-homophenylalanine | hPhe | D-homophenylalanine | D-hPhe |
| α-methyl-L-homophenylalanine | mhPhe | α-methyl-D-homophenylalanine | D-mhPhe |
| *N*-methyl-L-homophenylalanine | Nmhphe | *N*-methyl-D-homophenylalanine | D-Nmhphe |
| α-methyl-L-proline | mPro | α-methyl-D-proline | D-mPro |
| *N*-methyl-L-proline | Nmpro | *N*-methyl-D-proline | D-Nmpro |
| L-homoproline | hPro | D-homoproline | D-hPro |
| *trans*-hydroxy-L-proline | Hyp | *trans*-hydroxy-D-proline | D-Hyp |
| *cis*-hydroxy-L-proline | Hypc | *cis*-hydroxy-D-proline | D-Hypc |
| *trans*-4-fluoro-L-proline | 4Fp | *trans*-4-fluoro-D-proline | D-4Fp |
| *cis*-4-fluoro-L-proline | 4Fpc | *cis*-4-fluoro-D-proline | D-4Fpc |
| L-thyronine | Phe(*p*O-4OH-Ph) | D-thyronine | D-Phe(*p*O-4OH-Ph) |
| α-methyl-L-serine | mSer | α-methyl-D-serine | D-mSer |
| *N*-methyl-L-serine | Nmser | *N*-methyl-D-serine | D-Nmser |
| L-phosphoserine | pSer | D-phosphoserine | D-pSer |
| L-sulfoserine | sSer | D-sulfoserine | D-sSer |
| L-homoserine | hSer | D-homoserine | D-hSer |
| α-methyl-L-threonine | mThr | α-methyl-D-threonine | D-mThr |
| *N*-methyl-L-threonine | Nmthr | *N*-methyl-D-threonine | D-Nmthr |
| α-methyl-L-allo-threonine | mAth | α-methyl-D-allo-threonine | D-mAth |
| L-phosphothreonine | pThr | D-phosphothreonine | D-pThr |
| *O*-sulfo-L-threonine | sThr | *O*-sulfo- D-threonine | D-sThr |
| α-methyl-L-tryptophan | mTrp | α-methyl-D-tryptophan | D-mTrp |
| *N*-methyl-L-tryptophan | Nmtrp | *N*-methyl-D-tryptophan | D-Nmtrp |
| 1-methyl-L-tryptophan | Trp(1-Me) | 1-methyl-D-tryptophan | D-Trp(1-Me) |
| 2-methyl-L-tryptophan | Trp(2-Me) | 2-methyl-D-tryptophan | D-Trp(2-Me) |
| 5-methyl-L-tryptophan | Trp(5-Me) | 5-methyl-LDtryptophan | D-Trp(5-Me) |
| 5-hydroxy-L-tryptophan | Trp(5-OH) | 5-hydroxy-D-tryptophan | D-Trp(5-OH) |
| *N*-methyl-L-tyrosine | Nmtyr | *N*-methyl-D-tyrosine | D-Nmtyr |
| α-methyl-L-tyrosine | mTyr | α-methyl-D-tyrosine | D-mTyr |
| L-homotyrosine | hTyr | D-homotyrosine | D-hTyr |
| 3-hydroxy-L-tyrosine | Dopa | 3-hydroxy-D-tyrosine | D-Dopa |
| 3-amino-L-tyrosine | Tyr(*m*NH₂) | 3-amino-D-tyrosine | D-Tyr(*m*NH₂) |
| 3-iodo-L-tyrosine | Tyr(*m*I) | 3-iodo-D-tyrosine | D-Tyr(*m*I) |
| 4-phospho-L-tyrosine | pTyr | 4-phospho-D-tyrosine | D-pTyr |
| 4-sulfo-L-tyrosine | sTyr | 4-sulfo-D-tyrosine | D-sTyr |
| 3,5-dibromo-L-tyrosine | Tyr(3,5-Br) | 3,5-dibromo-D-tyrosine | D-Tyr(3,5-Br) |
| 3,5-diiodo-L-tyrosine | Tyr(3,5-I) | 3,5-diiodo-D-tyrosine | D-Tyr(3,5-I) |
| 3,5-dinitro-L-tyrosine | Tyr(3,5-NO₂) | 3,5-dinitro-L-tyrosine | D-Tyr(3,5-NO₂) |
| *O*-acetyl-L-tyrosine | Tyr(Ac) | *O*-acetyl-D-tyrosine | D-Tyr(Ac) |
| *O*-benzyl-L-tyrosine | Tyr(Bzl) | *O*-benzyl-D-tyrosine | D-Tyr(Bzl) |
| *O*-dimethylphosphono-L-tyrosine | Tyr(PO₃Me₂) | *O*-dimethylphosphono-D-tyrosine | D-Tyr(PO₃Me₂) |
| *O*-benzyl-L-phosphotyrosine | Tyr(PO(OBzl)O H) | *O*-benzyl-D-phosphotyrosine | D-Tyr(PO(OBzl)OH) |
| α-methyl-L-valine | mVal | α-methyl-D-valine | D-mVal |
| *N*-methyl-L-valine | Nmval | *N*-methyl-D-valine | D-Nmval |
| L-norvaline | Nva | D-norvaline | D-Nva |
| 5 -azido-L-norvaline | Nva(δ-N3) | 5 -azido- D-norvaline | D-Nva(δ-N3) |
| α-methyl-L-norvaline | mNva | α-methyl-D-norvaline | D-mNva |
| *N*-methyl-L-norvaline | Nmnva | *N*-methyl-D-norvaline | D-Nmnva |
| L-1,2,3,4-terahydroisoquinoline-3-carboxylic acid | Tic | D-1,2,3,4-terahydroisoquinoline-3-carboxylic acid | D-Tic |
| L-7-hydroxy-1,2,3,4-terahydroisoquinoline-3-carboxylic acid | Tic(7-OH) | D-7-hydroxy-1,2,3,4-terahydroisoquinoline- 3-carboxylic acid | D-Tic(7-OH) |
| L-1,2,3,4-tetrahydronorharman-3-carboxylic acid | Tpi | D-1,2,3,4-tetrahydronorharman-3-carboxylic acid | D-Tpi |
| L-octahydroindol-2-carboxylic acid | Oic | D-octahydroindol-2-carboxylic acid | D-Oic |

In terms of connectivity between the amino acids, in most instances this is a canonical main chain peptide bond connecting the alpha carboxylate of an amino acid with the alpha amino group of the adjacent (in direction from N- to C-terminus) amino acid. In particular, if not specified otherwise, the connection between adjacent residues Xi and Xi+1 (e.g. X1 and X2, or X2 and X3 etc.) is a main chain peptide bond (amide). In other words, if not explicitly specified otherwise, the term "peptidomimetic" as used herein does not extend to other types of connectivities between adjacent amino acids or building blocks of compounds of the invention.

In a number of instances, cross-links are either preferred or compulsory. Accordingly, these cross-links are specified either in the first aspect of the invention or in preferred embodiments thereof. These cross-links add circular elements to the compounds of the invention. This is a preferred means of stabilizing secondary- and/or three-dimensional structure, thereby facilitating binding to APC/C.

There are three preferred ways of implementing such cross-links: (i) main chain peptide bonds connecting the first with the last residue, (ii) peptide bonds (amides) which involve a main chain functional group (carboxylate or amino group) and a side chain functional group (amino group or carboxylate) or only corresponding side chain functional groups, and (iii) cross-links which are not amides.

Having said that, the invention is not limited to these particular and preferred cross-links. As known in the art, cross-links, especially in the context of peptides and peptidomimetics, generally serve to constrain the structure of said peptides and peptidomimetics. The chemical moieties involved in target binding are preferably not located in the cross-links of the compounds of the invention. Target binding is conferred by the sequence of amino acids of said compounds. In that sense, cross-links are means of enhancing, and depending on the type of cross-link used, they can also participate in binding to APC11.

Class (iii) of cross-links may be implemented using functional groups of proteinogenic amino acids. An example is the S-S bond (disulfide) connecting two Cys residues within a given compound. Other implementations make use of the side chains of non-natural amino acids. This is specified in the preferred embodiments disclosed further below.

The presence of cross-links is also designated by the term "covalently connected" herein, given that cross-links involve a covalent bond. Generally speaking, and in line with the above, such cross-links are not particularly limited. Preference is given to cross-links which, in terms of structure, connect the main chain of the compounds of the invention by a chain of atoms which has a length of 20 or less atoms, such as 1, 2, 3, 4, 5, 6, 7, 8, 9, 10, 11, 12, 13, 14, 15, 16, 17, 18 or 19 atoms. In one preferred embodiment, said chain of atoms consitsts of 3, 4 or 5 atoms. In another preferred embodiment, said cross-link comprises one or more group of atoms; for instance, 2 aromatic rings such as benzene, pyridine or triazole. In case of 2 aromatic rings, this may be implemented as a biphenyl or bipyridine moiety. In terms of counting the number of atoms forming said chain of atoms, only one edge of a given ring is counted, e.g. 4 atoms in case of benzene and pyridine. In case of a ring with an uneven number of atoms, the shorter edge is counted, e.g. 2 atoms in case of triazole. Preferred lengths of the chain of atoms in case of the presence of two aromatic rings are 10, 11, 12, 13, 14 and 15.

The term "main chain" has its usual meaning. It refers to the backbone of the peptides and peptidomimetics of the invention. As such it is defined by the following formula: (-N-C-CO-)n, wherein N is the nitrogen of the alpha-amino group, C in the middle is the alpha-carbon of the respective amino acid, CO is the alpha-carbonyl group, and n is the number of residues.

Said chain of atoms may consist of any atoms, preferably C-, N-, O- and S-atoms, at least one half of said atoms being preferably C. The chain of atoms may also consist only of carbon atoms. It is understood that, depending on the chosen chemistry of the cross-link, said chain of atoms may be substituted, but does not have to be. In case of absence of substituents at one or more or all given sites (i.e., the atoms forming said chain of atoms), the free valences are filled with hydrogen. Said chain of atoms may comprise one or more such as 2 or 3 double bonds, preferably C=C double bonds. The chain of atoms may comprise functional groups such as -S-S-, -COO- and -CONH-, wherein the carbonyl oxygens in -COO- and -CONH- and the hydrogen in -CONH- do not add to the count of atoms in said chain of atoms. Examples of said chain of atoms include -C-C-C-C- and -C-C=C-C-, i.e., chains of 4 atoms, wherein in these examples preference is given to all free valences being filled with hydrogen (not including the free valences connecting to the main chain, preferably to the alpha-carbon of the main chain at the respective positions). Further examples include -C-S-S-C- and -C-S-C-C-. Also in these cases, the preferred attachment site on the main chain is an alpha-carbon, and otherwise the free valences are filled with hydrogen.

Preferred implementations of -S-S- for items (b)-(f), -COO- for items (g)-(i) and -CONH- for items (a) and (g)-(i) connecting side chains by covalent bonds are as follows:

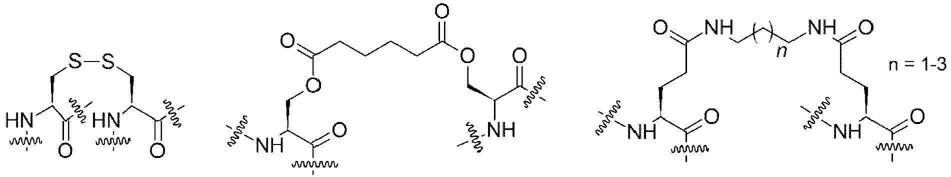

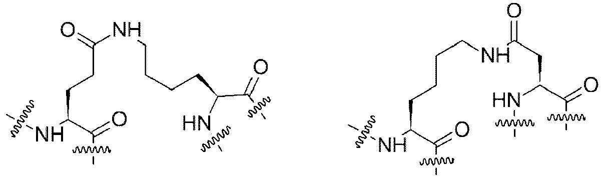

Preferred implementations of -C-C=C-C- for items (a)-(i), -C-C-C-C- for items (a)-(i), -C-S-S-C-for items (b)-(f) and -C-S-C-C- for items (b)-(f) connecting main and side chains by covalent bonds are as follows:

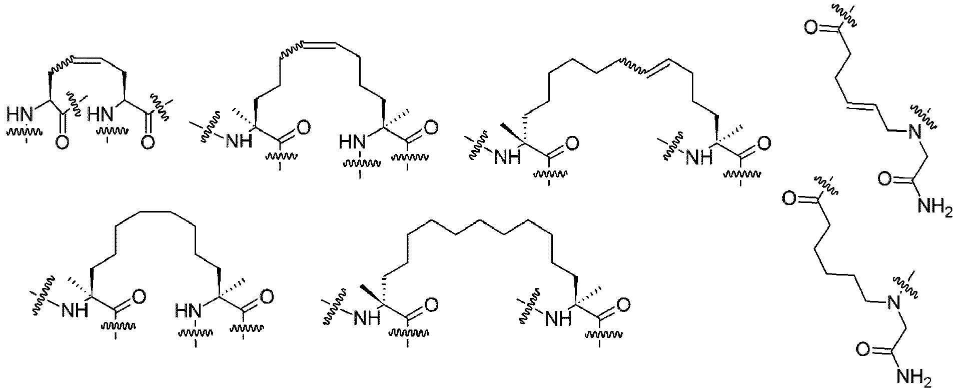

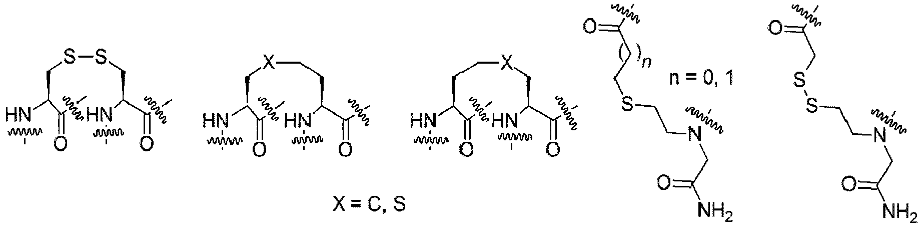

Arg mimetics with the guanidino group being derivatized may be used for positions X12 of item (a), X1 and X12 of item (b), X15 of item (c), X16 of item (d), X13 of item (e), X11 of item (f), X13 of items (g)-(i). Preferred implementations thereof are as follows:

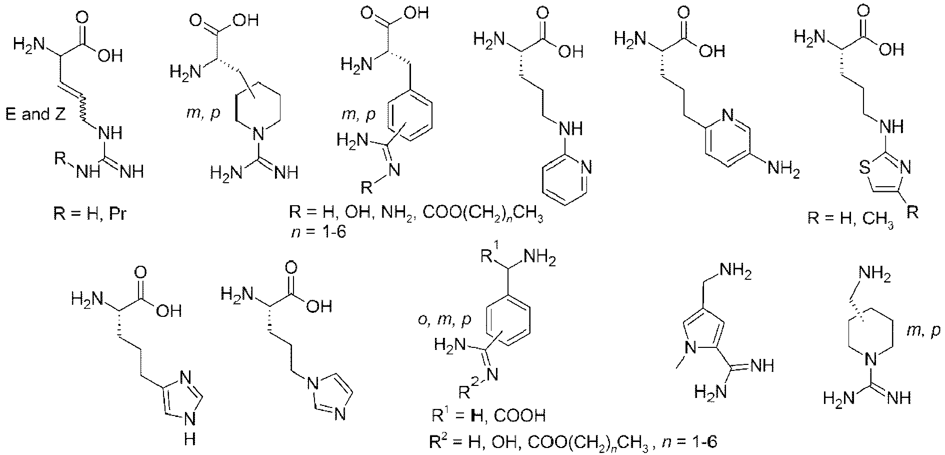

The invention also encompasses compounds which comprise the peptides or peptidomimetics defined in the first aspect and all its preferred embodiments. This does not apply in those instances where an alpha-amino group of the first residue (X1) and/or an alpha-carboxylate of the respective last defined residue (e.g. X14 in case of part (a) of the first aspect) are involved in a cross-link. It also does not apply to those preferred embodiments detailed further below where the N- and/or the C-terminus are further derivatized (e.g., in the simplest case, by acylation, preferably acetylation of the N-terminus and/or amidation of the C-terminus).

Otherwise, there is room for flanking sequences at one or both termini, given that the APC/C-interfering functionality is provided by the sequence which is specifically spelled out herein. Such flanking sequences are preferably peptidic in nature, more preferably they comprise or consist of proteinogenic amino acids (Table 1) as building blocks. Flanking sequences may be of any length, preference being given to a length of 1, 2, 3, 4, 5, 6, 7, 8, 9 or 10 amino acids. In case flanking sequences are present at either terminus, their length may be chosen independently.

Of note, compounds of the invention may be labelled or equipped with tags.

Labels include those which do not entail structural modification of the compounds such as isotope labels and radioactive labels. Other labels entail the attachment of a detectable moiety to the compounds of the invention, e.g. fluorescent or luminescent labels. A further type of label is a label which is a substrate of an enzyme, wherein preferably said enzyme converts said substrate into a product which is amenable to detection.

Labels confer advantages for certain methods and uses of compounds of the invention which methods and uses are disclosed further below.

Generally speaking, the compounds of the invention exhibit tolerance as regards exchange and modification of residues. Yet a distinction can be made between residues which are more tolerant and less tolerant in that respect. This is the subject of the following preferred embodiments which disclose implementations of parts (a) to (i) of the first aspect.

In a preferred embodiment of part (a) of the first aspect
X2 is selected from Trp, 2-NaI and 1-NaI, preferably Trp;
X4 is selected from Lys, Cys, D-Cys, Asp, Glu, Dap, Aib and (R)-2-(7'-octenyl)Ala, preferably (R)-2-(7'-octenyl)Ala;
X5 is Ile or Lys, preferably Ile;
X8 is Ile or Leu, preferably Ile;
X9 is Gln;
X11 is Asp, Glu, Cys, D-Cys, Aib or (S)-2-(4'-pentenyl)Ala, preferably (S)-2-(4'-pentenyl)Ala;
and
X12 is Arg.

In a preferred embodiment of part (b) of the first aspect
X1 is selected from Arg, Phe(2-guanidino) and Phe(3-guanidino), preferably Arg;
X2 is selected from Trp, Phe, Tyr, Ile, Leu, Cys, Pra, Hpg, Agl, Pre Crt and (S)-2-amino-4-bromobutyric acid, preferably Cys or Agl;
X3 is selected from Ser, His and Dab, preferably His;
X6 is Pro or D-Pro, preferably Pro;
X7 is selected from Glu, Arg, Trp, Cit, Orn and pSer, preferably Arg;
X8 is Thr;
X10 is selected from Trp, 2-NaI and 1-NaI, preferably Trp;
X11 is selected from Trp, Phe, Tyr, Ile, Leu, Cys, (S)-2-amino-4-bromobutyric acid, Dap(N3), (S)-2-amino-4-azido-butyric acid (2Abu(γ-N3)), 5-azido-norvaline (Nva(δ-N3)), Agl and Crt, preferably Cys or Agl;
and
X12 is selected from Arg, Phe(3-guanidino), Phe(4-guanidino), Cit and Orn, preferably Arg.

In a preferred embodiment of part (c) of the first aspect
X2 is selected from Ser, His and hSer, preferably His;
X3 is selected from Cys, Pra, Hpg, Agl, Crt and (S)-2-amino-4-bromobutyric acid, preferably Cys or Agl;
X6 is Pro;
X7 is Glu, Trp or Aad, preferably Glu;
X8 is Thr;
X9 is selected from Trp, 2-NaI and 1-NaI, preferably Trp;
X11 is selected from Trp, 2-NaI and 1-NaI, preferably Trp;
X12 is Asn;
X13 is selected from Cys, (S)-2-amino-4-bromobutyric acid, Dap(N3), 2Abu(γ-N3), Nva(δ-N3), Agl and Crt, preferably Cys or Agl;
and
X15 is selected from Arg, Phe(3-guanidino), Phe(4-guanidino) and Cit, preferably Arg.

In a preferred embodiment of part (d) of the first aspect
X3 is Thr or hSer, preferably Thr;
X4 is selected from Cys, Pra, Hpg, Agl, Crt and (S)-2-amino-4-bromobutyric acid, preferably Cys or Agl;
X8 is Glu, Trp or Aad, preferably Glu;
X9 is Thr;
X10 is selected from Trp, 2-NaI and 1-NaI, preferably Trp;
X12 is selected from Trp, 2-NaI and 1-NaI, preferably Trp;
X13 is Asn;
X14 is selected from Cys, (S)-2-amino-4-bromobutyric acid, Dap(N3), 2Abu(γ-N3), Nva(δ-N3), Agl and Crt, preferably Cys or Agl;
and
X16 is Arg or Cit, preferably Arg.

In a preferred embodiment of part (e) of the first aspect
X1 is selected from Cys, Pra, Hpg, Agl, Crt and (S)-2-amino-4-bromobutyric acid, preferably Cys or Agl;
X2 is Glu;
X5 is Pro;
X8 is selected from Trp, 2-NaI and 1-NaI, preferably Trp;
X9 is selected from His, Asn and Gln, preferably His;
X10 is selected from Cys, (S)-2-amino-4-bromobutyric acid, Dap(N3), 2Abu(γ-N3), Nva(δ-N3), Agl and Crt, preferably Cys or Agl;
X12 is selected from Trp, 2-NaI and 1-NaI, preferably Trp;
and
X13 is Arg or Cit, preferably Arg.

In a preferred embodiment of part (f) of the first aspect
X2 is selected from Trp, Phe and 2-NaI, preferably Phe or 2-NaI;
X4 is Trp;
X6 is selected from Ala, Trp, D-Leu, D-Thr and D-Glu, preferably Trp;
X7 is Glu;
X9 is Glu or Thr;
X11 is selected from Arg, Ser, Cit and hSer, preferably Arg;
X13 is selected from D-Pro, allylamine, cysteamine and Orn, preferably D-Pro; and
X14 is selected from Pro, Aib, 4-pentenoic acid and 3-bromopropionic acid, preferably Pro.

In a preferred embodiment of part (g) of the first aspect
X2 is selected from Trp, Glu, 2-NaI and 1-NaI, preferably Trp;
X5 is selected from Lys, Glu, Ser, Lys(N3), (S)-2-(4'-pentenyl)Ala and Aib, preferably (S)-2-(4'-pentenyl)Ala;
X6 is Ile or Lys, preferably Ile;
X9 is selected from Asp, Lys, Ser, (S)-2-(4'-pentenyl)Ala, Pra, D-Pra and Aib, preferably (S)-2-(4'-pentenyl)Ala;
X10 is Gln;
X13 is Arg or Trp, preferably Trp;
and
X14 is Trp or Arg, preferably Trp.

In a preferred embodiment of part (h) of the first aspect
X2 is selected from Trp, 2-NaI and 1-NaI, preferably Trp;
X3 is Lys, Glu, Ser, Lys(N3) or (S)-2-(4'-pentenyl)Ala, preferably Lys;
X5 is Ile, Trp or Arg, preferably Trp;
X6 is selected from Ile, Leu and Lys, preferably Ile;
X7 is Asp, Lys, Ser, Pra, D-Pra or (S)-2-(4'-pentenyl)Ala, preferably Asp;
X8 is Lys, Glu, Ser, Lys(N3) or (S)-2-(4'-pentenyl)Ala, preferably Lys;
X9 is Ile;
X10 is Gln;
X12 is Asp, Lys, Ser, Pra, D-Pra or (S)-2-(4'-pentenyl)Ala, preferably Asp;
and
X13 is selected from Arg, Lys and Trp, preferably Arg.

In a preferred embodiment of part (i) of the first aspect
X2 is selected from Trp, 2-NaI and 1-NaI, preferably Trp;
X4 is Lys, Glu, Ser, Lys(N3) or (S)-2-(4'-pentenyl)Ala, preferably Lys;
X6 is selected from Ile, Leu and Lys, preferably Ile;
X8 is Asp, Lys, Ser, Pra, D-Pra or (S)-2-(4'-pentenyl)Ala, preferably Asp;
X9 is Ile;
X10 is Gln;
X11 is Lys, Glu, Ser, Lys(N3) or (S)-2-(4'-pentenyl)Ala, preferably Lys;
X13 is selected from Arg, Lys and Trp, preferably Arg;
and
X15 is Asp, Lys, Ser, Pra, D-Pra or (S)-2-(4'-pentenyl)Ala, preferably Asp.

In a preferred embodiment of the peptide or peptidomimetic of the invention
(a) X2 to X12 of said peptide or peptidomimetic of claim 1 (a) assume an alpha-helix structure under physiological conditions;
(b) said peptide or peptidomimetic of claim 1 (b) assumes a beta-hairpin structure under physiological conditions, wherein preferably X1 to X4 and X9 to X12 are in beta-sheet conformation and/or a turn is formed by residues X5 to X8;
(c) said peptide or peptidomimetic of claim 1 (c) assumes a helix-turn-helix structure under physiological conditions, wherein preferably X1 to X4 and X10 to X15 are in alpha-helical conformation and/or a turn is formed by X5 to X9;
(d) said peptide or peptidomimetic of claim 1 (d) assumes a helix-turn-helix structure under physiological conditions, wherein preferably X2 to X5 and X11 to X16 are in alpha-helical conformation and/or a turn is formed by X6 to X10;
(e) said peptide or peptidomimetic of claim 1 (e) assumes a helix-turn structure under physiological conditions, wherein preferably X7 to X14 are in alpha-helical conformation and/or a turn is formed by X1 to X6;
(f) said peptide or peptidomimetic of claim 1 (f) assumes a beta-hairpin structure under physiological conditions, wherein X2 to X4 and X9 to X11 are in beta-sheet conformation and/or turns are formed by X5 to X8, and X12 to X14 and X1;
(g) X2 to X13 of said peptide or peptidomimetic of claim 1 (g) assume an alpha-helix structure under physiological conditions;
(h) X1 to X12 of said peptide or peptidomimetic of claim 1 (h) assume an alpha-helix structure under physiological conditions;
(i) X1 to X15 of said peptide or peptidomimetic of claim 1 (i) assume an alpha-helix structure under physiological conditions.

The term "physiological conditions" is known in the art. Preferably, for example if intracellular conditions are to be mimicked, it may be implemented by conditions comprising: 14 mM Na⁺, 140 mM K⁺, 10⁻⁷ mM Ca²⁺, 20 mM Mg²⁺, 4 mM Cl, 10 mM HCO₃⁻, 11 mM HPO₄²⁻ and H₂PO₄⁻, 1 mM SO₄²⁻, 45 mM phosphocreatine, 14 mM carnosine, 8 mM amino acids, 9 mM creatine, 1.5 mM lactate, 5 mM ATP, 3.7 mM hexose monophosphate, 4 mM protein and 4 mM urea.

The terminology used in this embodiment to define secondary structure elements is common in the art. For example, a beta-turn is generally composed of four amino acids; see also the above residue ranges for compounds comprising beta-turns. These four positions are numbered from N- to C-terminus as positions 1 to 4. Positions 2 and 3 are particularly relevant for inducing and maintaining the turn conformation.

It is understood that the terms "alpha-helix" (or equivalently "alpha-helical"), "beta-strand/sheet" and "turn" define the main chain dihedral angles, in particular those adjacent to the alpha-carbon of the amino acid under consideration. These angles are commonly designated Φ and Ψ. They fully define the main chain conformation, given that the peptide bond is conformationally constrained. In the case of the canonical alpha-helix, typical Φ (-64°) and Ψ (-42°) dihedral angles may allowed to vary by ± 35°. For beta-sheet, consisting as said of two beta strands joined by a turn, the most typical values of Φ (-120°) and Ψ (+135°) for the strand can vary by ± 35°, respectively. For the joining turn, Φ and Ψ dihedral angles may vary depending on the turn-type (Table 3). Values at positions *i*+1 and *i*+2 can vary by ± 30°, being possible to have one of these values with a deviation up to ± 45°. Preferred are type II' turns.

**Table 3. Classification of typical beta-turns (taken from Wilmot, C.M. et al. Protein Eng. 1990, 3, 479-493).**

| Beta-turn Type | **φ**_{*i*+*1*} | **ψ**_{*i*+*1*} | **φ**_{*i*+2} | **ψ**_{*i*+2} |
|---|---|---|---|---|
| I | -60° | -30° | -90° | 0° |
| II | -60° | 120° | 80° | 0° |
| I' | 60° | 30° | 90° | 0° |
| II' | 60° | -120° | -80° | 0° |
| III | -60° | -30° | -60° | -30° |
| VIa | -60 | 120 | -90 | 0 |
| VIb | -120 | 120 | -60 | 0 |
| VIII | -60 | -30 | -120 | 120 |

Means and methods for determining secondary structure are known in the art and include circular dichroism and high-resolution structure determination, the latter including NMR and X-ray crystallography. Peptide structure determination by NMR is reviewed, for example, in Williamson, Methods Mol. Biol. 1993, 17,69-85. X-ray crystallography of peptides is described, for example, in Spencer and Nowick, Isr J Chem. 2015, 56, 678-710 (doi:10.1002/ijch.201400179).

Furthermore, in silico methods are available which permit to predict secondary structure. The use of such methods is deliberately envisaged for the purpose of verifying the features required by the above embodiment. These methods are reviewed by Pande, V. in Protein Folding Studied with Molecular Dynamics Simulation, in Roberts G.C.K. (eds.) Encyclopedia of Biophysics. Springer, Berlin, Heidelberg (2013) (doi.org/10.1007/978-3-642-16712-6_730) and Geng, H. et al. in Comput. Struct. Biotechnol. J. 2019, 17, 1162-1170 (doi: 10.1016/j.csbj.2019.07.010).

In a further preferred embodiment, alpha-amino acids located at positions 2 and/or 3 of a beta-turn are replaced by beta-turn inducing amino acids, wherein preferably said beta-turn inducing amino acids are selected from the following:

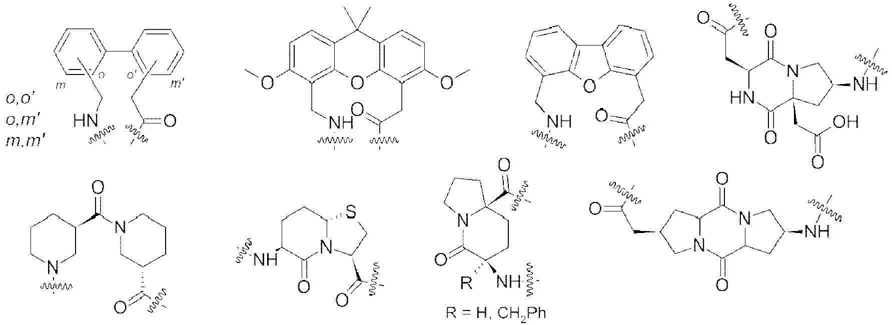

In the preferred embodiment above, use is made of beta-turn inducing moieties at one or both positions 2 and/or 3. A repertoire of beta-turn inducing moieties is available to the skilled person. This is reviewed for instance by Robinson, J. A. in Acc. Chem. Res. 2008, 41, 1278-1288 (doi.org/10.1021/ar700259k) and Nair, R. V. et al. in Chem. Commun. 2014, 50, 13874-13884 (doi.org/10.1039/C4CC03114H).

In a further preferred embodiment, said peptide or peptidomimetic is capable of interfering with ubiquitination.

As mentioned above, ubiquitination is a cellular process which targets proteins for degradation. In the course of cell division, for cell division to proceed, especially to anaphase, degradation of proteins involved in earlier stages of mitosis is key. Interfering with the ubiquitination function of APC/C is therefore a means of stopping mitosis.

Assays for determining such capability are known in the art and disclosed in the Examples. In a preferred embodiment, said interfering is determined by bringing said peptide or peptidomimetic into contact with APC/C, Cdc20, and E2 enzyme (UBE2C, UBE2D or UBE2S), ubiquitin, a substrate (e.g. securin or cyclin B1) and ATP, and quantifying the amount of ubiquitinated substrate in presence and absence of said peptide or peptidomimetic, wherein a decreased amount of ubiquitinated substrate in presence of said peptide or peptidomimetic is indicative of said peptide or peptidomimetic being capable of interfering with ubiquitination.

A preferred substrate is securin.

The amount of ubiquitinated substrate (in the above implementation securin or cyclin B1) may be determined by SDS-PAGE followed by fluorescence detection.

Alternatively or in addition, the capability of the compounds of the invention to bind and interfere with the ubiquitination machinery contained in APC/C may be assessed/predicted using in silico means and methods, e.g., by molecular dynamics simulation and associated calculation of free energies of binding as described by McCammon, J. A. and Harvey, S. C. in Dynamics of Proteins and Nucleic Acids, Cambridge University Press, USA (1987), Leach, A. R. in Molecular Modelling: Principles and Applications, 2nd Edition, Pearson Education Limited, UK (2001) and Wang, J. M. et al. J. Am. Chem. Soc. 2001, 123, 5221-5230 (doi:10.1021/ja003834q).

In a preferred embodiment, said interfering is reducing or abolishing the activity of an ubiquitinating enzyme and/or of a ubiquitin ligase.

In a particularly preferred embodiment, said enzyme is an E2 enzyme, preferably UBE2C, UbcH7, UBE2D or UBE2S; or said ligase is an E3 ligase, preferably APC11, Rbx1 or c-Cbl.

The protein names used in this disclosure are art-established. For example, the UniProt Consortium provides curated information about proteins (Nucl. Acids. Res. 47, D1: D506-D515 (2019)).

In a further preferred embodiment, said peptide or peptidomimetic stops cell division or induces cell death.

Assays for determining such capability are known in the art and disclosed in the Examples. In a preferred embodiment, stopping or inducing is determined by bringing cells into contact with said peptide or peptidomimetic and determining whether said cells proliferate, stop dividing, or undergo cell death, wherein cells which do not divide or cell death is indicative of said peptide or peptidomimetic stopping cell division or inducing cell death. Useful cells for such assay include those used in the Examples, i.e., HeLa cells and hTERT RPE-1 cells. Preferred are HeLa cells.

Also, an extension of the time required for mitosis is indicative of a beneficial effect of the compounds of the invention.

An extended duration of mitosis, arrest of cell division and cell death are all associated with morphological changes which are amenable to detection by live cell microscopy, which is a preferred means of determining these outcomes upon bringing compounds of the invention into contact with cells.

In a further preferred embodiment, said peptide or peptidomimetic is modified
(a) to improve its delivery, absorption, distribution, metabolism or excretion; and/or
(b) by
   a. esterification of a carboxyl group;
   b. esterification of a hydroxyl group;
   c. formation of a pharmaceutically acceptable salt or complexes;
   d. introduction of a hydrophilic moiety;
   e. introduction, exchange or removal of a substituent on an aromatic ring or an aliphatic chain;
   f. conversion of an alkyl group into a cyclic form thereof;
   g. derivatization of a hydroxyl group to give rise to an acetal or ketal;
   h. derivatization of a triazole group to an isoxazole group;
   i. acetylation, alkylation, introduction of aldehyde or sulfonyl groups of a nitrogen as well as its transformation to carbamate; and/or
   j. derivatization of an aldehyde or a ketone to give rise to a Schiff's base, an oxime, an acetal, a ketal, an enol-ester, an oxazolidine or a thiazolidine.

It is known in the art that once proof of principle is established for a compound (here the capabilities disclosed in the preceding preferred embodiments), further optimization of such compound (generally referred to as "lead compound") is, albeit not strictly necessary, preferred or desirable. In terms of optimizing, generally the key pharmacokinetic properties of delivery, absorption, distribution, metabolism or excretion, the latter four commonly abbreviated as "ADME", are of interest.

As disclosed further below, the present inventors explored and implemented a number of optimizations in that respect. Alternatively, or in addition, use may be made of the well-established modification recited in this preferred embodiment.

In a preferred embodiment of part (a) of the first aspect and all its preferred embodiments as defined above and below,
(a) the alpha-amino group of X1 is bound to a moiety selected from acetyl; benzoyl; Biotin-Ahx; methyl, Ac-Gly-Phe-Trp-Phe-Gly; Ac-Gly-Phe-Trp-Phe-Gly-NH-(CH₂)₂-O-CH₂-CO-; Ac-Gly-Phe-Trp-Phe-Gly-NH-(CH₂)₂-PEG-O-CH₂-CO-, PEG being polyethylene glycol with a polymerization degree from 1 to 6; and R-COO-(CH₂)ₙ-OCO-, R-COO-(CH₂)ₙ-OCO-N(CH₃)-CH₂-CO-, R-COO-(CH₂)ₙ-OCOO-(CH₂)₂-CO- or R-COO-(CH₂)ₙ-OCO-NH-(CH₂)₂-CO-, R being alkyl, cycloalkyl, cycloheteroalkyl, aryl or heteroaryl, alkyl having 1 to 6 carbon atoms, cycles having 4 to 10, preferably 5 or 6 ring atoms and 1 or 2 cycles, R preferably being methyl, ethyl, phenyl or benzyl, and n being 1 or 2;
(b) X4 is (*R*)-2-(7'-octenyl)Ala and X11 is (*S*)-2-(4'-pentenyl)Ala, and a side chain of X4 is bound to a side chain of X11 by a covalent bond;
(c) X4 and X11 are Asp or preferably Glu, and their side chains are bound through a diamine alkyl moiety consisting of -NH-CH₂-(CH₂)ₙ-CH₂-NH-, n being 1, 2 or 3;
(d) X4 is D-Cys and X11 is Cys or D-Cys, and a side chain of X4 is bound to a side chain of X11 via an aryl moiety, preferably 1,1'-biphenyl-4,4'-bis(methyl) or 3,3'-bipyridine,6,6'-bis(methyl);
(e) X4 and X11 are Cys, and a side chain of X4 is bound to a side chain of X11 via an azoaryl moiety, preferably *N,N*'-[(1Z)-azodi-4,1-phenylene]diacetamide or *cis*-3,3'-bis(sulfonato)-4,4'-bis(acetamide)azobenzene;
(f) X4 is Dap and X11 is Asp, and a side chain of X4 is bound to a side chain of X11 via the aryl moiety -CO-CH₂-*p*-C₆H₄-CH₂-NH-, or -CO-CH₂-*p*-C₆F₄-CH₂-NH; or
(g) X14 is amidated, esterified or bound to a moiety selected from Gly-Phe-Trp-Phe-Gly-NH₂, -NH-(CH₂)₂-O-CH₂-CO-Gly-Phe-Trp-Phe-Gly-NH₂ and -NH-(CH₂)₂-PEG-O-CH₂-CO-Gly-Phe-Trp-Phe-Gly-NH₂, PEG being polyethylene glycol with a polymerization degree from 1 to 6.

Items (a) and (g) define preferred modifications which increase the stability of said peptide or peptidomimetic towards enzymatic degradation from exopeptidases or useful for labelling to identify protein interactions and delivery. In particular for the last one, it includes endosome escape domains and functional moieties that target intracellular esterases.

Items (b) to (f) define alternative implementations of the X4-X11 cross-link. Each of these may be combined with (a) and/or (g).

In a preferred embodiment of part (b) of the above embodiment (said above embodiment in turn relating to part (a) of the first aspect), said bond is as follows:

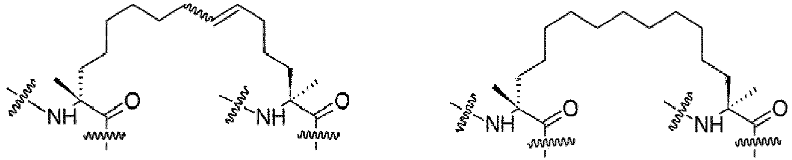

In a preferred embodiment of part (c) of the above embodiment, said bond is as follows:

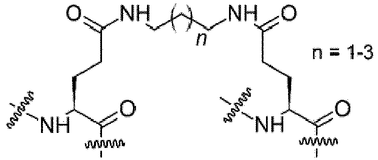

In a preferred embodiment of part (d) of the above embodiment, said bond is as follows:

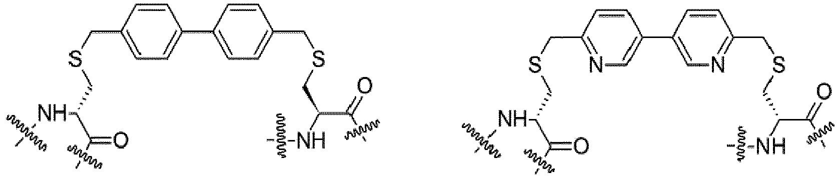

In a preferred embodiment of part (e) of the above embodiment, said bond is as follows:

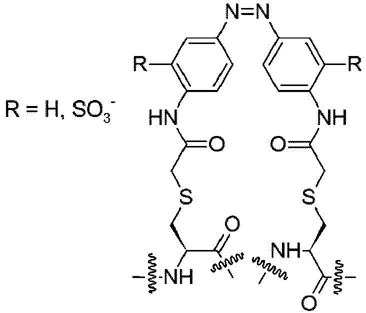

In a preferred embodiment of part (f) of the above embodiment, said bond is as follows:

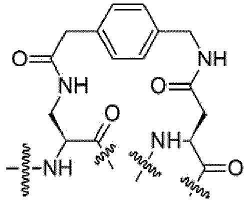

In a preferred embodiment of part (b) of the first aspect and all its preferred embodiments as defined above and below,
(a) the alpha-amino group of X1 is bound to a moiety selected from acetyl; benzoyl; Biotin-Ahx; methyl, Ac-Gly-Phe-Trp-Phe-Gly; Ac-Gly-Phe-Trp-Phe-Gly-NH-(CH₂)₂-O-CH₂-CO-; Ac-Gly-Phe-Trp-Phe-Gly-NH-(CH₂)₂-PEG-O-CH₂-CO-, PEG being polyethylene glycol with a polymerization degree from 1 to 6; and R-COO-(CH₂)ₙ-OCO, R-COO-(CH₂)ₙ-OCO-N(CH₃)-CH₂-CO-, R-COO-(CH₂)ₙ-OCOO-(CH₂)₂-CO- or R-COO-(CH₂)ₙ-OCO-NH-(CH₂)₂-CO-, R being alkyl, cycloalkyl, cycloheteroalkyl, aryl or heteroaryl, alkyl having 1 to 6 carbon atoms, cycles having 4 to 10, preferably 5 or 6 ring atoms and 1 or 2 cycles, R preferably being methyl, ethyl, phenyl or benzyl, and n being 1 or 2;
(b) wherein X2 and X11 are independently selected from Trp, Phe, Tyr, Ile and Leu;
(c) wherein a side chain of X2 is covalently connected to a side chain of X11;
   a. wherein if X2 and X11 are Agl or Crt, respectively, the covalent connection may comprise a carbon-carbon double bond;
   b. wherein if X2 is Cys and X11 is (S)-2-amino-4-bromobutyric acid, a C-S bond connects X2 with X11;
   c. wherein if X2 is Pra and X11 is 2Abu(γ-N3) or Nva(δ-N3), a triazole group connects X2 with X11;
   d. wherein if X2 and X11 are Cys, a disulfide bridge connects X2 with X11;
   e. wherein if X2 is (S)-2-amino-4-bromobutyric acid and X11 is Cys, a C-S bond connects X2 with X11;
   g. wherein if X2 is Hpg and X11 Dap(N3), a triazole group connects X2 with X11
(d) wherein X4 and X9 are independently selected from Trp and Phe;
(e) wherein a side chain of X4 is covalently connected to a side chain of X9;
   a. wherein if X4 and X9 are Cys, a disulfide bridge connects X4 with X9;
   b. wherein if X4 and X9 are Agl, the covalent connection may comprise a carbon-carbon double bond;
   c. wherein if X4 and X9 are Crt, the covalent connection may comprise a carbon-carbon double bond;
   d. wherein if X4 is Cys and X9 is (S)-2-amino-4-bromobutyric acid, a C-S bond connects X4 with X9;
   e. wherein if X4 is (S)-2-amino-4-bromobutyric acid and X9 is Cys, a C-S bond connects X4 with X9;
   f. wherein if X4 is Pra and X11 is 2Abu(γ-N3) or Nva(δ-N3), a triazole group connects X4 with X9;
   g. wherein if X4 is Hpg and X11 Dap(N3), a triazole group connects X4 with X9;
(f) wherein the alpha-carboxy group of X12 is amidated or bound to a moiety selected from -OEt, -OMe, -NHEt, -NHMe, -Gly-Phe-Trp-Phe-Gly-NH₂, -NH-(CH₂)₂-O-CH₂-CO-Gly-Phe-Trp-Phe-Gly-NH₂ and -NH-(CH₂)₂-PEG-O-CH₂-CO-Gly-Phe-Trp-Phe-Gly-NH₂, PEG being polyethylene glycol with a polymerization degree from 1 to 6;
wherein preferably the connections defined in parts (b) to (e) are implemented as follows:

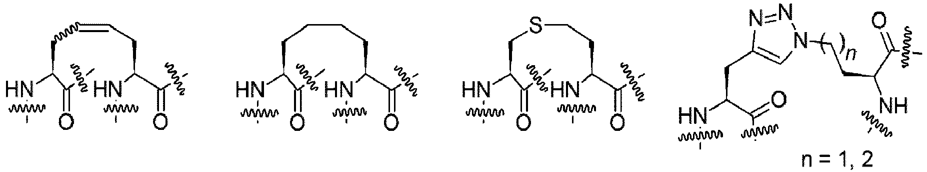

Items (b) and (c) are alternatives, as are sub-items a. to g. within item (c).
Items (d) and (e) are alternatives, as are sub-items a. to g. within item (d).
The most preferred connections are (c).a. and (c).d.

Any choice from these items may be combined with (a) and/or (f).

In the above preferred embodiment, there is recitation of the option that a cross-link comprises a carbon-carbon double bond. This is a result of a known process of introducing such cross-links; see, e.g., Robinson et al., Chem. Comm., 2009, 4293-4295. Such double bond may be reduced to give rise to the corresponding carbon-carbon single bond. Both options are embraced by the present invention. Preferred implementations are shown above, see the structural formulae at the end of the above embodiment.

Of note, in relation to item (b) of the above embodiment where X2 and X11 are independently selected from Trp, Phe, Tyr, Ile and Leu, such cross-link between X2 and X11 is not preferred.

In a preferred embodiment of part (c) of the first aspect and all its preferred embodiments as defined above and below,
(a) the alpha-amino group of X1 is bound to a moiety selected from acetyl; benzoyl; Biotin-Ahx; methyl, Ac-Gly-Phe-Trp-Phe-Gly; Ac-Gly-Phe-Trp-Phe-Gly-NH-(CH₂)₂-O-CH₂-CO-; Ac-Gly-Phe-Trp-Phe-Gly-NH-(CH₂)₂-PEG-O-CH₂-CO-, PEG being polyethylene glycol with a polymerization degree from 1 to 6; and R-COO-(CH₂)ₙ-OCO-, R-COO-(CH₂)ₙ-OCO-N(CH₃)-CH₂-CO-, R-COO-(CH₂)ₙ-OCOO-(CH₂)₂-CO- or R-COO-(CH₂)ₙ-OCO-NH-(CH₂)₂-CO-, R being alkyl, cycloalkyl, cycloheteroalkyl, aryl or heteroaryl, alkyl having 1 to 6 carbon atoms, cycles having 4 to 10, preferably 5 or 6 ring atoms and 1 or 2 cycles, R preferably being methyl, ethyl, phenyl or benzyl, and n being 1 or 2;
(b) X3 and X13 are Cys, and the covalent connection between X3 and X13 comprises a sulfur-sulfur bond;
(c) X3 and X13 are Agl, and the covalent connection between X3 and X13 may comprise a carbon-carbon double bond;
(d) X3 and X13 are Crt, and the covalent connection between X3 and X13 may comprise a carbon-carbon double bond;
(e) X3 is Cys and X13 is (S)-2-amino-4-bromobutyric acid, and a C-S bond connects X3 with X13;
(f) X3 is (S)-2-amino-4-bromobutyric acid and X13 is Cys, and a C-S bond connects X3 with X13;
(g) X3 is Pra and X13 is 2Abu(γ-N3) or Nva(δ-N3), and a triazole group connects X3 with X13;
(h) X3 is Hpg and X13 is Dap(N3), and a triazole group connects X3 with X13; or
(i) the alpha-carboxy group of X16 is amidated or bound to a moiety selected from-OEt, -OMe, -NHEt, -NHMe, -Gly-Phe-Trp-Phe-Gly-NH₂, -NH-(CH₂)₂-O-CH₂-CO-Gly-Phe-Trp-Phe-Gly-NH₂ and -NH-(CH₂)₂-PEG-O-CH₂-CO-Gly-Phe-Trp-Phe-Gly-NH₂, PEG being polyethylene glycol with a polymerization degree from 1 to 6.

Items (b) to (h) are alternatives. Any choice therefrom may be combined with (a) and/or (i).

In a preferred embodiment of part (d) of the first aspect and all its preferred embodiments as defined above and below,
(a) X4 and X14 are Cys, and the covalent connection between X4 and X14 comprises a sulfur-sulfur bond;
(b) X4 and X14 are Agl, and the covalent connection between X4 and X14 may comprise a carbon-carbon double bond;
(c) X4 and X14 are Crt, and the covalent connection between X4 and X14 may comprise a carbon-carbon double bond;
(d) X4 is Cys and X14 is (S)-2-amino-4-bromobutyric acid, and a C-S bond connects X4 with X14;
(e) X4 is (S)-2-amino-4-bromobutyric acid and X14 is Cys, and a C-S bond connects X4 with X14;
(f) X4 is Pra and X14 is 2Abu(γ-N3) or Nva(δ-N3), and a triazole group connects X4 with X14;
(g) X4 is Hpg and X14 is Dap(N3), and a triazole group connects X4 with X14; or
(h) the alpha-carboxy group of X17 is amidated or not, esterified, or bound to a moiety selected from Gly-Phe-Trp-Phe-Gly-NH₂, -NH-(CH₂)₂-O-CH₂-CO-Gly-Phe-Trp-Phe-Gly-NH₂ and -NH-(CH₂)₂-PEG-O-CH₂-CO-Gly-Phe-Trp-Phe-Gly-NH₂, PEG being polyethylene glycol with a polymerization degree from 1 to 6.

Items (a) to (g) define alternatives. Any of these may be combined with (h).

In a preferred embodiment of part (e) of the first aspect and all its preferred embodiments as defined above and below,
(a) the alpha-amino group of X1 is bound to a moiety selected from acetyl; benzoyl; Biotin-Ahx; methyl, Ac-Gly-Phe-Trp-Phe-Gly; Ac-Gly-Phe-Trp-Phe-Gly-NH-(CH₂)₂-O-CH₂-CO-; Ac-Gly-Phe-Trp-Phe-Gly-NH-(CH₂)₂-PEG-O-CH₂-CO-, PEG being polyethylene glycol with a polymerization degree from 1 to 6; and R-COO-(CH₂)ₙ-OCO-, R-COO-(CH₂)ₙ-OCO-N(CH₃)-CH₂-CO-, R-COO-(CH₂)ₙ-OCOO-(CH₂)₂-CO- or R-COO-(CH₂)ₙ-OCO-NH-(CH₂)₂-CO-, R being alkyl, cycloalkyl, cycloheteroalkyl, aryl or heteroaryl, alkyl having 1 to 6 carbon atoms, cycles having 4 to 10, preferably 5 or 6 ring atoms and 1 or 2 cycles, R preferably being methyl, ethyl, phenyl or benzyl, and n being 1 or 2;
(b) X1 and X10 are Cys, and the covalent connection between X1 and X10 comprises a sulfur-sulfur bond;
(c) X1 and X10 are Agl, and the covalent connection between X1 and X10 may comprise a carbon-carbon double bond;
(d) X1 and X10 are Crt, and the covalent connection between X1 and X10 may comprise a carbon-carbon double bond;
(e) X1 is Cys and X10 is (S)-2-amino-4-bromobutyric acid, and a C-S bond connects X1 with X10;
(f) X1 is (S)-2-amino-4-bromobutyric acid and X10 is Cys, and a C-S bond connects X1 with X10;
(g) X1 is Pra and X10 is 2Abu(γ-N3) or Nva(δ-N3), and a triazole group connects X1 with X10;
(h) X1 is Hpg and X10 is Dap(N3), and a triazole group connects X1 with X10;
   or
(i) X14 is amidated, esterified or functionalized with Gly-Phe-Trp-Phe-Gly-NH₂, -NH-(CH₂)₂-O-CH₂-CO-Gly-Phe- Trp-Phe-Gly- NH₂ and -NH-(CH₂)₂-PEG-O-CH₂-CO-Gly-Phe-Trp-Phe-Gly-NH₂, PEG being polyethylene glycol with a polymerization degree from 1 to 6.

Items (b) to (h) are alternatives. Any of these may be combined with (a) and/or (i).

In a preferred embodiment of part (f) of the first aspect and all its preferred embodiments as defined above and below
(a) X13 is allylamine or cysteamine, wherein the amino group of X13 is bound to a moiety selected from -CH₂CONH₂, -CH₂CO-Gly-Phe-Trp-Phe-Gly-NH₂, -CH₂COOEt, and CH₂COOMe;
(b) X14 comprises a 4-pentenoyl, bromoacetyl or 3-bromopropionyl moiety, wherein the carboxy group of said moiety is covalently bound to the alpha-amino group of X1; or
(c) wherein a side chain of X13 is covalently connected to a side chain of X14;
   i. if X13 is allylamine and X14 is 4-pentenoic acid, the covalent connection may comprise a carbon-carbon double bond;
   ii. if X13 is cysteamine and X14 is 3-bromopropionyl, a carbon-sulfur bond or a sulfur-sulfur bond is comprised in the covalent connection;
wherein preferably said covalent connection in accordance with (c) is selected from the following:

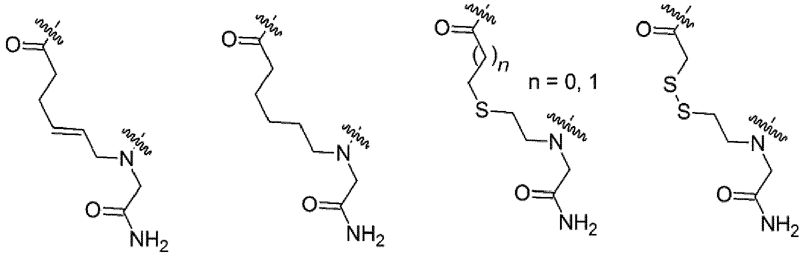

In a preferred embodiment of part (g) of the first aspect and all its preferred embodiments as defined above and below
(a) the amino group of X1 is bound to a moiety selected from acetyl; benzoyl; Biotin-Ahx; methyl, Ac-Gly-Phe-Trp-Phe-Gly; Ac-Gly-Phe-Trp-Phe-Gly-NH-(CH₂)₂-O-CH₂-CO-; Ac-Gly-Phe-Trp-Phe-Gly-NH-(CH₂)₂-PEG-O-CH₂-CO-, PEG being polyethylene glycol with a polymerization degree from 1 to 6; and R-COO-(CH₂)ₙ-OCO-, R-COO-(CH₂)ₙ-OCO-N(CH₃)-CH₂-CO-, R-COO-(CH₂)ₙ-OCOO-(CH₂)₂-CO- or R-COO-(CH₂)ₙ-OCO-NH-(CH₂)₂-CO-, R being alkyl, cycloalkyl, cycloheteroalkyl, aryl or heteroaryl, alkyl having 1 to 6 carbon atoms, cycles having 4 to 10, preferably 5 or 6 ring atoms and 1 or 2 cycles, R preferably being methyl, ethyl, phenyl or benzyl, and n being 1 or 2;
(b) X5 and X9 are (S)-2-(4'-pentenyl)Ala, wherin a side chain of X5 is covalently connected to a side chain of X9, and wherein the covalent connection may comprise a carbon-carbon double bond;
(c) wherein X5 and X9 are Ser, and their side chains are bound through the adipoyl moiety -CO-(CH₂)₄-CO-;
(d) X5 is Lys and X9 is Asp, or X5 is Glu and X9 is Lys, wherein a side chain of X5 is covalently connected to a side chain of X9;
   X5 is Lys(N3) and X9 is Pra or D-Pra, wherein a side chain of X5 is covalently connected to a side chain of X9 through a triazole group; and/or
(e) X14 is amidated, esterified or bound to a moiety selected from Gly-Phe-Trp-Phe-Gly-NH₂, -NH-(CH₂)₂-O-CH₂-CO-Gly-Phe-Trp-Phe-Gly-NH₂ and -NH-(CH₂)₂-PEG-O-CH₂-CO-Gly-Phe-Trp-Phe-Gly-NH₂, PEG being polyethylene glycol with a polymerization degree from 1 to 6.

In a preferred embodiment of part (h) of the first aspect and all its preferred embodiments as defined above and below
(a) the alpha-amino group of X1 is bound to a moiety selected from acetyl; benzoyl; Biotin-Ahx; methyl, Ac-Gly-Phe-Trp-Phe-Gly; Ac-Gly-Phe-Trp-Phe-Gly-NH-(CH₂)₂-O-CH₂-CO-; Ac-Gly-Phe-Trp-Phe-Gly-NH-(CH₂)₂-PEG-O-CH₂-CO-, PEG being polyethylene glycol with a polymerization degree from 1 to 6; and R-COO-(CH₂)ₙ-OCO-, R-COO-(CH₂)ₙ-OCO-N(CH₃)-CH₂-CO-, R-COO-(CH₂)ₙ-OCOO-(CH₂)₂-CO- or R-COO-(CH₂)ₙ-OCO-NH-(CH₂)₂-CO-, R being alkyl, cycloalkyl, cycloheteroalkyl, aryl or heteroaryl, alkyl having 1 to 6 carbon atoms, cycles having 4 to 10, preferably 5 or 6 ring atoms and 1 or 2 cycles, R preferably being methyl, ethyl, phenyl or benzyl, and n being 1 or 2;
(b) wherein X3 is Lys and X7 is Asp, or X3 is Glu and X7 is Lys;
(c) wherein X8 is Lys and X12 is Asp, or X8 is Glu and X12 is Lys;
(d) wherein X3 and X7 are (S)-2-(4'-pentenyl)Ala;
(e) wherein X8 and X12 are (S)-2-(4'-pentenyl)Ala;
(f) wherein X3 is Lys(N3) and X7 is Pra or D-Pra;
(g) wherein X8 is Lys(N3) and X12 is Pra or D-Pra;
(h) wherein X3 and X7 are Ser, and their side chains are bound through the adipoyl moiety -CO-(CH₂)₄-CO-;
(i) wherein X8 and X12 are Ser, and their side chains are bound through the adipoyl moiety -CO-(CH₂)₄-CO-; and/or
(j) the alpha-carboxy group of X14 is amidated, esterified or bound to a moiety selected from Gly-Phe-Trp-Phe-Gly-NH₂, -NH-(CH₂)₂-O-CH₂-CO-Gly-Phe-Trp-Phe-Gly-NH₂ and -NH-(CH₂)₂-PEG-O-CH₂-CO-Gly-Phe-Trp-Phe-Gly-NH₂, PEG being polyethylene glycol of different polymerization degrees, preferably from 1 to 6.

In a preferred embodiment of part (i) of the first aspect and all its preferred embodiments as defined above and below
(a) the alpha-amino group of X1 is bound to a moiety selected from acetyl; benzoyl; Biotin-Ahx; methyl, Ac-Gly-Phe-Trp-Phe-Gly; Ac-Gly-Phe-Trp-Phe-Gly-NH-(CH₂)₂-O-CH₂-CO-; Ac-Gly-Phe-Trp-Phe-Gly-NH-(CH₂)₂-PEG-O-CH₂-CO-, PEG being polyethylene glycol with a polymerization degree from 1 to 6; and R-COO-(CH₂)ₙ-OCO-, R-COO-(CH₂)ₙ-OCO-N(CH₃)-CH₂-CO-, R-COO-(CH₂)ₙ-OCOO-(CH₂)₂-CO- or R-COO-(CH₂)ₙ-OCO-NH-(CH₂)₂-CO-, R being alkyl, cycloalkyl, cycloheteroalkyl, aryl or heteroaryl, alkyl having 1 to 6 carbon atoms, cycles having 4 to 10, preferably 5 or 6 ring atoms and 1 or 2 cycles, R preferably being methyl, ethyl, phenyl or benzyl, and n being 1 or 2;
(b) wherein X4 is Lys and X8 is Asp, or X4 is Glu and X8 is Lys;
(c) wherein X11 is Lys and X15 is Asp, or X11 is Glu and X15 is Lys;
(d) wherein X4 and X8 are (S)-2-(4'-pentenyl)Ala;
(e) wherein X11 and X15 are (S)-2-(4'-pentenyl)Ala;
(f) wherein X4 is Lys(N3) and X8 is Pra or D-Pra;
(g) wherein X11 is Lys(N3) and X15 is Pra or D-Pra;
(h) wherein X4 and X8 are Ser, and their side chains are bound through the adipoyl moiety -CO-(CH₂)₄-CO-;
(i) wherein X11 and X15 are Ser, and their side chains are bound through the adipoyl moiety -CO-(CH₂)₄-CO-; and/or
(j) the corresponding carboxy group X15 is amidated, esterified or bound to a moiety selected from Gly-Phe-Trp-Phe-Gly-NH₂, -NH-(CH₂)₂-O-CH₂-CO-Gly-Phe-Trp-Phe-Gly-NH₂, and -NH-(CH₂)₂-PEG-O-CH₂-CO-Gly-Phe-Trp-Phe-Gly-NH₂, PEG being polyethylene glycol of different polymerization degrees, preferably from 1 to 6.

Where cross-links are implemented either as carbon-carbon double bond or involving a triazole group, a carbon-carbon double bond, e.g. as formed in a first step during preparation of the respective compound, may be reduced to a carbon-carbon single bond.

In a preferred embodiment, said peptide or peptidomimetic is conjugated, preferably via a linker, to a ligand of an E3 ligase. Generally speaking, such E3 ligase may be freely chosen. A compound of the invention, when conjugated with a ligand of an E3 ligase, provides a heterobifunctional molecule. Conjugation is preferably via main chain peptide bonds or isopeptide bonds. Conjugation may be in any orientation. Preferably, such conjugates consist of the three specified elements: peptide or peptidomimetic of the invention, linker, and E3 ligand. The location of the linker is between the other two elements.
In an alternative approach, a compound of the invention and said ligand of an E3 ligase are administered to a cell or formulated as a medicine (including the option of separate, i.e., subsequent in any order, administration). In such a case, both the compound of the invention and the ligand are modified by the addition of functional groups which, once compound and ligand are in the cell, react to form a covalent bond (e.g. click chemistry).

This preferred embodiment combines the capability of peptides and peptidomimetics of the invention to bind to APC/C with the capability of E3 ligands to recruit an E3 ligase. Such E3 ligase in turn recruits a cognate E2 enzyme. This provides for ubiquitination and subsequent degradation of APC/C. This effect is also a means of providing the beneficial and therapeutic effects in accordance with this invention which are described in more detail further below.

The general notion of conjugating an E3 ligand to a compound (here the peptide or peptidomimetic of the invention) known to bind a target (here APC/C) for the purpose of triggering degradation of said target via the ubiquitination pathway has been described, for example, in An and Fu, EBioMedicine 36: 553-562 (2018). Implementations of E3 ligands can also be found in this publication. Exemplary E3 ligands include Pomalidomide, Thalidomide, Lenalidomide and VHL-1 (the latter being a ligand of Von Hippel Lindau (VHL) E3 ligase); see Table 1 of An and Fu, loc. cit. Examplary E3 ligases are MDM2, IAP, VHL and cereblon. Useful linkers include short, peptidic and/or flexible linkers; see, e.g. Chen et al., Adv Drug Deliv Rev. 2013 Oct 15; 65(10): 1357-1369. An example is (Gly4Ser)3.

In a second aspect, the present invention relates to a peptide or peptidomimetic of the first aspect and of any of its embodiments for use in medicine.

Given that the compounds of the invention are capable of interfering with cell division, their medical use is evident. Preferred medical use is the subject of further aspects disclosed below.

In a third aspect, the present invention provides a medicament comprising or consisting of the peptide or peptidomimetic of any one of the preceding aspects and embodiments thereof.

The medicament (also referred to as "pharmaceutical composition") may further comprise pharmaceutically acceptable carriers, excipients and/or diluents. Examples of suitable pharmaceutical carriers, excipients and/or diluents are well known in the art and include phosphate buffered saline solutions, water, emulsions, such as oil/water emulsions, various types of wetting agents, sterile solutions etc. Compositions comprising such carriers can be formulated by well-known conventional methods.

These pharmaceutical compositions can be administered to the subject at a suitable dose. Administration of the suitable compositions may be effected by different ways, e.g., by intravenous, intraperitoneal, subcutaneous, intramuscular, topical, intradermal, intranasal or intrabronchial administration. It is particularly preferred that said administration is carried out by injection. The compositions may also be administered directly to the target site, e.g., by biolistic delivery to an external or internal target site, like the site of a tumour.

The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently.

In a preferred embodiment,
(a) one or more peptides or peptidomimetics of any one of the preceding claims is/are the only pharmaceutically active agents comprised in said medicament;
(b) said medicament comprises one or more further pharmaceutically active agents, preferably selected from
   a. agents which interfere with or stop cell division such as TAME, proTAME, apcin, Emi1, or GLMN;
   b. agents which induce DNA damage, such as Topoisomerase II inhibitors, preferably etoposide or doxorubicin; and
   c. agents which interfere with microtubule assembly such as paclitaxel, vincristine, and PLK1 inhibitors including BI6727; or
(c) said medicament is to be administered to a patient which is undergoing, has undergone, or will undergo radiation therapy.

In accordance with part (b) of this preferred embodiment, a combination therapy is provided. Preferred agents to be combined with compounds of the invention are those which directly or indirectly interfere with APC/C function and/or related systems (see in this respect the introductory section of this disclosure above) or with cell division in general. These agents are reviewed and defined in the introductory part of this disclosure.

Given that the agents of part (b) on the one hand and the compounds of the invention on the other hand have distinct molecular mechanisms of action, such combinations are characterized by a synergistic effect on cell division, cell death and mitigating or curing hyperproliferative disorders.

In a fourth aspect, the present invention provides a peptide or peptidomimetic of any one of the preceding aspects and embodiments thereof for use in a method of treating, ameliorating or curing a hyperproliferative disease.

In a preferred embodiment, said hyperproliferative disease is a benign or malign tumour, a metastasis or cancer.

Preferably, said cancer is B-cell non-Hodgkin lymphoma, bladder cancer, breast tumour or breast cancer, cancer of the stomach, cervical cancer, colorectal cancer, esophageal squamous cell carcinoma, glioblastoma, incasive ductal carcinoma, leukemia such as chronic myeloid leukemia, liver cancer such as hepatocellular carcinoma, lung cancer such as lung adenocarcinoma and non-small cell lung cancer, melanoma, ovary cancer, pancreatic cancer such as pancreatic ductal adenocarcinoma, or prostate cancer.

Such preferred medical indications are evident in view of the function of APC/C and the information given in the introductory section above. Having said that, and in line with the disclosure above, beneficial effects are expected for all types of hyperproliferation or instances or disorders where stopping cell division is desirable.

In a fifth aspect, the invention provides an in vitro or ex vivo method of interfering with or stopping cell division, said method comprising bringing one or more peptides or peptidomimetics of any one of the preceding aspects and embodiments thereof in contact with a cell.

In a preferred embodiment, the cells are cells in culture or comprised in a tissue.

In a sixth aspect, the invention relates to a method of purifying APC/C, said method comprising
(a) bringing into contact a sample comprising APC/C with a peptide or peptidomimetic of any one of the preceding aspects and embodiments thereof; and
(b) separating a complex comprising APC/C and said peptide or peptidomimetic formed in step (a) from the remainder of the constituents of said sample.

In a preferred embodiment, said peptide or peptidomimetic is immobilized on a carrier or bead. Beads may be magnetic, e.g. paramagnetic beads which facilitates their handling when purifying in accordance with the method of the sixth aspect. Such carriers or beads with compounds of the invention being covalently or non-covalently immobilized thereon may be viewed as affinity matrices.

Once APC/C is bound to such carrier, bead or matrix, it may be detected by any downstream detection method such as ELISA or Biacore.

In a further preferred embodiment, said sample is a total cell extract.

In an alternative, any solution or suspension comprising APC/C may be used as sample.

In a seventh aspect, the invention provides a method of detecting APC/C, said method comprising
(a) bringing a sample comprising or suspected to comprise APC/C in contact with a peptide or peptidomimetic of any one of the preceding aspects and embodiments thereof, wherein said peptide or peptidomimetic carries a detectable label; and
(b) detecting a complex comprising APC/C and said peptide or peptidomimetic.

For the purpose of detecting, labelled forms of the compounds of the invention may be employed which labelled forms are disclosed herein further above.

In an eighth aspect, the invention provides a kit comprising or consisting of one or more peptides or peptidomimetics of the first aspect and its embodiments.

In a preferred embodiment, said kit further comprises or further consists of
(a) an agent which interferes with or stops cell division such as TAME, proTAME, apcin, Emi1 or GLMN;
(b) an agent which induces DNA damage, such as Topoisomerase II inhibitors, preferably etoposide or doxorubicin;
(c) an agent which interfers with microtubule assembly such as paclitaxel, vincristine, and PLK1 inhibitors including BI6727;
   and/ or
(d) a manual comprising instructions for performing the method of any one of the fifth, sixth and seventh aspect.

In a further preferred embodiment of said kit, said peptide or peptidomimetic
(a) carries a detectable label; and/or
(b) is conjugated, preferably via a linker, to a ligand of an E3 ligase.

In a ninth aspect, the present invention relates to a method of treating, ameliorating, curing or preventing a hyperproliferative disease, said method comprising or consisting of administering one or more peptides or peptidomimetics of the first or second aspect or a medicament of the third aspect to a patient suffering from or being at risk of developing said disease.

Preferred embodiments of said disease are those disclosed further above.

In a tenth aspect, the invention provides a use of a peptide or peptidomimetic of the first aspect and of any one of its embodiments as a lead for developing a pharmaceutically active agent, wherein preferably said pharmaceutically active agent has anti-proliferative activity.

While it is understood that the compounds of the invention themselves are useful as therapeutic agents, it will in many instances be possible to fine-tune pharmacokinetic properties thereof.

Related thereto, and in an eleventh aspect, the invention provides an in vitro or ex vivo method of developing a lead for the treatment of a hyperproliferative disease, said method comprising:
(a) Chemically modifying a peptide or peptidomimetic of the first aspect or any one of its embodiments;
(b)
   a. Comparing anti-proliferative activity of the result of (a) to the activity of said peptide or peptidomimetic prior to performing step (a);
      and/ or
   b. Comparing toxicity of the result of (a) to the toxicity of said peptide or peptidomimetic prior to performing step (a);
(c) Wherein an improved activity determined in (b)a. and/or a decreased toxicity determined in (b)b. is indicative of the result of (a) being an improved lead.

In a preferred embodiment, said modifying is as defined in relation to the optimization of pharmacokinetic properties including delivery and ADME.

In the following, especially preferred compounds of the invention are disclosed. These compounds are not only especially preferred implementations of the first aspect, but at the same time define preferred embodiments of any of the other aspects of this invention.

Of note, the respective SEQ ID NOs define the core part of the peptide or peptidomimetic, i.e., without N- and C-terminal modifications and without information about cross-links (or, equivalently, cyclic structure). Generally speaking, and if not otherwise indicated herein above, such terminal modifications and cross-links are preferred features. Indeed, the terminal modifications are optional features. They generally provide improved delivery. Yet, sufficient activity is observed also in absence of said terminal modifications. Further information about the cross-links can be found in the description of the first aspect of the invention above.

It is evident from the numerous references to the Examples in the following that substantially all claimed classes of compounds have been reduced to practice.

Especially preferred peptides and peptidomimetics in accordance with part (a) of the first aspect:
SEQ. ID. NO. 31. (designated G3-4 in the Examples) Ac-cyclo(4,11)-EWWZKLEIQRXRRA-NH₂ (Z refers to (*R*)-2-(7'-octenyl)Ala, X refers to (*S*)-2-(4'-pentenyl)Ala, underlined Z and X denote hydrocarbon-stapled C=C bond between the side chains of Z and X). The term "hydrocarbon-stapled" as used herein generally refers to a chain of carbon atoms (for a definition of the term "chain of atoms" see further above) connecting two alpha-carbon atoms. Preferably this is implemented as -C-C=C-C- or in the reduced from thereof (-C-C-C-C-).
SEQ. ID. NO. 32. (designated G3-5 in the Examples) Ac-cyclo(4,11)-EWWZKLELQRXRRA-NH₂ (Z refers to (*R*)-2-(7'-octenyl)Ala, X refers to (*S*)-2-(4'-pentenyl)Ala, underlined Z and X denote hydrocarbon-stapled C=C bond between the side chains of Z and X)
SEQ. ID. NO. 33. (designated G3-6 in the Examples) Ac-cyclo(4,11)-EWWZIKEIQRXRRA-NH₂ (Z refers to (*R*)-2-(7'-octenyl)Ala, X refers to (*S*)-2-(4'-pentenyl)Ala, underlined Z and X denote hydrocarbon-stapled C=C bond between the side chains of Z and X)
SEQ. ID. NO. 37. (designated G3-6Pra in the Examples) Ac-cyclo(4,11)-EWPraZIKEIQRXRRA-NH₂ (Z refers to (R)-2-(7'-octenyl)Ala, X refers to (*S*)-2-(4'-pentenyl)Ala, underlined Z and X denote hydrocarbon-stapled C=C bond between the side chains of Z and X)
SEQ. ID. NO. 36. (designated G3-6_mod1_miniPEG in the Examples) Ac-GFWFG-NH(CH₂)₂OCH₂CO-cyclo(4,11)-EWWZIKEIQRXRRA-NH₂ (Z refers to (*R*)-2-(7'-octenyl)Ala, X refers to (*S*)-2-(4'-pentenyl)Ala, underlined Z and X denote hydrocarbon-stapled C=C bond between the side chains of Z and X)
SEQ. ID. NO. 34. Ac-GFWFG-PEG₆-cyclo(4,11)-EWWZIKEIQRXRRA-NH₂ (Z refers to (R)-2-(7'-octenyl)Ala, X refers to (*S*)-2-(4'-pentenyl)Ala, underlined Z and X denote hydrocarbon-stapled C=C bond between the side chains of Z and X)
SEQ. ID. NO. 35. Ac-GFWFG-PEG₂-cyclo(4,11)-EWWZIKEIQRXRRA-NH₂ (Z refers to (R)-2-(7'-octenyl)Ala, X refers to (*S*)-2-(4'-pentenyl)Ala, underlined Z and X denote hydrocarbon-stapled C=C bond between the side chains of Z and X)
SEQ. ID. NO. 38. Ac-GFWFG-NH(CH₂)₂OCH₂CO-cyclo(4,11)-EWWZₛIKEIQRXₛRRA-NH₂ (Z refers to (*R*)-2-(7'-octenyl)Ala, X refers to (*S*)-2-(4'-pentenyl)Ala, underlined Zₛ and Xₛ denote hydrocarbon-stapled C-C bond between the side chains of Z and X followed by hydrogenation reaction)
SEQ. ID. NO. 39. CH₃COOCH₂OCO-cyclo(4,11)-EWWZIKEIQRXRRA-NH₂ (Z refers to (*R*)-2-(7'-octenyl)Ala, X refers to (*S*)-2-(4'-pentenyl)Ala, underlined Z and X denote hydrocarbon-stapled C=C bond between the side chains of Z and X)
SEQ. ID. NO. 90. CH₃CH₂COOCH₂OCO-cyclo(4,11)-EWWZIKEIQRXRRA-NH₂ (Z refers to (*R*)-2-(7'-octenyl)Ala, X refers to (*S*)-2-(4'-pentenyl)Ala, underlined Z and X denote hydrocarbon-stapled C=C bond between the side chains of Z and X)
SEQ. ID. NO. 91. CH₃CH₂COOCH₂OCO-Sar-cyclo(4,11)-EWWZIKEIQRXRRA-NH₂ (Z refers to (*R*)-2-(7'-octenyl)Ala, X refers to (*S*)-2-(4'-pentenyl)Ala, underlined Z and X denote hydrocarbon-stapled C=C bond between the side chains of Z and X)
SEQ. ID. NO. 92. CH₃CH₂COOCH₂0CO-NH(CH₂)₂CO-cyclo(4,11)-EWWZIKEIQRXRRA-NH₂ (Z refers to (R)-2-(7'-octenyl)Ala, X refers to (S)-2-(4'-pentenyl)Ala, underlined Z and X denote hydrocarbon-stapled C=C bond between the side chains of Z and X)
SEQ. ID. NO. 93. CH₃CH₂COOCH₂OCOO-(CH₂)₂CO-cyclo(4,11)-EWWZIKEIQRXRRA-NH₂ (Z refers to (*R*)-2-(7'-octenyl)Ala, X refers to (*S*)-2-(4'-pentenyl)Ala, underlined Z and X denote hydrocarbon-stapled C=C bond between the side chains of Z and X)
SEQ. ID. NO. 40. C₆H₅CH₂COOCH₂OCO-cyclo(4,11)-EWWZIKEIQRXRRA-NH₂ (Z refers to (*R*)-2-(7'-octenyl)Ala, X refers to (*S*)-2-(4'-pentenyl)Ala, underlined Z and X denote hydrocarbon-stapled C=C bond between the side chains of Z and X)

Most preferred in accordance with part (a) of the first aspect is the above compound comprising the sequence of SEQ ID NO: 33.

Especially preferred peptides and peptidomimetics in accordance with part (b) of the first aspect:
SEQ. ID. NO. 1 (designated G1-1 in the Examples) Ac-cyclo(2,11)-RCHWGPETFWCR-NH₂ (underlined C denotes disulfide bridge)
SEQ. ID. NO. 2 (designated G1-2 in the Examples) Ac-cyclo(2,11)-RCSWGPETFWCR-NH₂ (underlined C denotes disulfide bridge)
SEQ. ID. NO. 3 (designated G1-3 in the Examples) Ac-cyclo(2,11)-RCHWGPRTFWCR-NH₂ (underlined C denotes disulfide bridge)
SEQ. ID. NO. 4. Ac-cyclo(2,11)-RAglSWGPETFWAglR-NH₂ (underlined Agl denotes stapled C=C bond between Agl side chains)
SEQ. ID. NO. 41. Ac-cyclo(2,11)-RAglₛSWGPETFWAglₛR-NH₂ (underlined Aglₛ denotes stapled C-C bond between the precursor Agl side chains followed by hydrogenation reaction)
SEQ. ID. NO. 5. Ac-cyclo(2,11)-RAglHWGPRTFWAglR-NH₂ (underlined Agl denotes stapled C=C bond between Agl side chains)
SEQ. ID. NO. 42. Ac-cyclo(2,11)-RAglₛHWGPRTFWAglₛR-NH₂ (underlined Aglₛ denotes stapled C-C bond between the precursor Agl side chains followed by hydrogenation reaction)
SEQ. ID. NO. 43. Ac-cyclo(2,11)-RPraHWGPRTFWNva(δ-N3)R-NH₂ (underlined Pra and Nva(δ-N3) denotes that a triazole group connect the side chains of Pra and Nva(δ-N3))
SEQ. ID. NO. 44. Ac-cyclo(2,11)(4,9)-RAglSCrtGPETCrtWAglR-NH₂ (underlined Agl denotes stapled C=C bond between Agl side chains, underlined Crt denotes stapled C=C bond between Crt side chains)
SEQ. ID. NO. 45. Ac-cyclo(2,11)(4,9)-RAglHCrtGPRTCrtWAglR-NH₂ (underlined Agl denotes stapled C=C bond between Agl side chains, underlined Crt denotes stapled C=C bond between Crt side chains)
SEQ. ID. NO. 46. Ac-cyclo(2,11)(4,9)-RAglₛHCrtₛGPRTCrtₛWAglₛR-NH₂ (underlined Aglₛ denotes stapled C-C bond between the precursor Agl side chains followed by hydrogenation reaction, underlined Crtₛ denotes stapled C-C bond between the precursor Crt side chains followed by hydrogenation reaction)
SEQ. ID. NO. 47. Ac-cyclo(2,11)(4,9)-RPraHPraGPRT2Abu(γ-N3)WNva(δ-N3)R-NH₂ (underlined Pra at position X2 and Nva(δ-N3) denotes that a triazole group connect the side chains of Pra and Nva(δ-N3), underlined Pra at position X4 and 2Abu(γ-N3) denotes that a triazole group connects the side chains of Pra and 2Abu(γ-N3))
SEQ. ID. NO. 87. Ac-cyclo(2,11)(4,9)-RPraHAglGPRTAglWNva(δ-N3)R-NH₂ (underlined Pra at position X2 and Nva(δ-N3) denotes that a triazole group connects the side chains of Pra and Nva(δ-N3), underlined Agl at positions X4 and X9 denotes stapled C=C bond between Agl side chains)
SEQ. ID. NO. 88. Ac-cyclo(2,11)(4,9)-RPraHAglₛGPRTAglₛWNva(δ-N3)R-NH₂ (underlined Pra at position X2 and Nva(δ-N3) denotes that a triazole group connects the side chains of Pra and Nva(δ-N3), underlined Agl at positions X4 and X9 denotes stapled C-C bond between the precursor Agl side chains followed by hydrogenation reaction)
SEQ. ID. NO. 89. Ac-cyclo(2,11)(4,9)-RWHHpgGPRTDap(N3)WFR-NH₂ (underlined Hpg and Dap(N3) denotes that a triazole group connects the side chains of Hpg and Dap(N3))
SEQ. ID. NO. 48 (designated G1-4 in the Examples) cyclo(3,12)-GRCHWGPETFWCRT (underlined C denotes disulfide bridge, N- and C-term free).
SEQ. ID. NO. 49. Ac-GFWFG-NH(CH₂)₂OCH₂CO-cyclo(2,11)-RCHWGPRTFWCR-NH₂ (underlined C denotes disulfide bridge)
SEQ. ID. NO. 50. C₆H₅CH₂COOCH₂OCO-cyclo(2,11)-RCHWGPRTFWCR-NH₂ (underlined C denotes disulfide bridge)
SEQ. ID. NO. 51. CH₃COOCH₂OCO-cyclo(2,11)-RCHWGPRTFWCR-NH₂ (underlined C denotes disulfide bridge)
SEQ. ID. NO. 52. Ac-GFWFG-NH(CH₂)₂OCH₂CO-cyclO(2,11)-RAglHWGPRTFWAglR-NH₂ (underlined Agl denotes stapled C=C bond between Agl side chains)
SEQ. ID. NO. 53. C₆H₅CH₂COOCH₂OCO-cyclo(2,11)-RAglHWGPRTFWAglR-NH₂ (underlined Agl denotes stapled C=C bond between Agl side chains)
SEQ. ID. NO. 54. CH₃COOCH₂OCO-cyclo(2,11)-RAglHWGPRTFWAglR-NH₂ (underlined Agl denotes stapled C=C bond between Agl side chains)
SEQ. ID. NO. 94 CH₃CH₂COOCH₂OCO-Sar-cyclo(2,11)-RAglₛSWGPETFWAglₛR-NH₂ (underlined Aglₛ denotes stapled C-C bond between the precursor Agl side chains followed by hydrogenation reaction)

Most preferred in accordance with part (b) of the first aspect is the above compound comprising the sequence of SEQ ID NO: 3 and 42.

Especially preferred peptides and peptidomimetics in accordance with part (c) of the first aspect:
SEQ. ID. NO. 6 (designated G1-7 in the Examples) Ac-cyclo(3,13)-THCDDPETWRWNCQRV-NH₂ (underlined C denotes disulfide bridge)
SEQ. ID. NO. 7. Ac-cyclo(3,13)-TSCDDPETWRWNCQRV-NH₂ (underlined C denotes disulfide bridge)
SEQ. ID. NO. 8. Ac-cyclo(3,13)-THAglDDPETWRWNAglQRV-NH₂ (underlined Agl denotes stapled C=C bond between Agl side chains)
SEQ. ID. NO. 9. Ac-cyclo(3,13)-TSAglDDPETWRWNAglQRV-NH₂ (underlined Agl denotes stapled C=C bond between Agl side chains)
SEQ. ID. NO. 55. Ac-cyclo(3,13)-THAglₛDDPETWRWNAglₛQRV-NH₂ (underlined Aglₛ denotes stapled C-C bond between the precursor Agl side chains followed by hydrogenation reaction)
SEQ. ID. NO. 56. Ac-GFWFG-NH(CH₂)₂OCH₂CO-cyclo(3,13)-THCDDPETWRWNCQRV-NH₂ (underlined C denotes disulfide bridge)
SEQ. ID. NO. 57. Ac-GFWFG-NH(CH₂)₂OCH₂CO-cyclo(3,13)- THAglₛDDPETWRWNAglₛQRV-NH₂ (underlined Aglₛ denotes stapled C-C bond between the precursor Agl side chains followed by hydrogenation reaction)
SEQ. ID. NO. 58. C₆H₅CH₂COOCH₂OCO-cyclo(3,13)-THCDDPETWRWNCQRV-NH₂ (underlined C denotes disulfide bridge)
SEQ. ID. NO. 59. C₆H₅CH₂COOCH₂OCO-cyclo(3,13)-THAglₛDDPETWRWNAglₛQRV-NH₂ (underlined Aglₛ denotes stapled C-C bond between the precursor Agl side chains followed by hydrogenation reaction)
SEQ. ID. NO. 95. CH₃CH₂COOCH₂OCO-Sar-cyclo(3,13)-THAglₛDDPETWRWNAAglₛQRV-NH₂ (underlined Aglₛ denotes stapled C-C bond between the precursor Agl side chains followed by hydrogenation reaction)

Most preferred in accordance with part (c) of the first aspect is the above compound comprising the sequence of SEQ ID NO: 6 and 55.

Especially preferred peptides and peptidomimetics in accordance with part (d) of the first aspect:
SEQ. ID. NO. 10 (designated G1-8 in the Examples) cyclo(4,14)-GDTCDDPETWRWNCQRH (underlined C denotes disulfide bridge)
SEQ. ID. NO. 11. cyclo(4,14)-GDTAglDDPETWRWNAglQRH (underlined Agl denotes stapled C=C bond between Agl side chains)
SEQ. ID. NO. 60. cyclo(4,14)-GDTAglₛDDPETWRWNAglₛQRH (underlined Aglₛ denotes stapled C-C bond between the precursor Agl side chains followed by hydrogenation reaction)
SEQ. ID. NO. 61. Ac-GFWFG-NH(CH₂)₂OCH₂CO-cyclo(4,14)-GDTCDDPETWRWNCQRH (underlined C denotes disulfide bridge)
SEQ. ID. NO. 62. Ac-GFWFG-NH(CH₂)₂OCH₂CO-cyclo(4,14)-GDTAglDDPETWRWNAglQRH (underlined Agl denotes stapled C=C bond between Agl side chains)
SEQ. ID. NO. 63. C₆H₅CH₂COOCH₂OCO-cyclo(4,14)-GDTCDDPETWRWNCQRH (underlined C denotes disulfide bridge)
SEQ. ID. NO. 64. CH₃COOCH₂OCO-cyclo(4,14)-GDTCDDPETWRWNCQRH (underlined C denotes disulfide bridge)
SEQ. ID. NO. 65. C₆H₅CH₂COOCH₂OCO-cyclo(4,14)-GDTAglₛDDPETWRWNAglₛQRH (underlined Aglₛ denotes stapled C-C bond between the precursor Agl side chains followed by hydrogenation reaction)
SEQ. ID. NO. 66. CH₃COOCH₂OCO-cyclo(4,14)-GDTAglₛDDPETWRWNAglₛQRH (underlined Aglₛ denotes stapled C-C bond between the precursor Agl side chains followed by hydrogenation reaction)

Most preferred in accordance with part (d) of the first aspect is the above compound comprising the sequence of SEQ ID NO: 10.

Especially preferred peptides and peptidomimetics in accordance with part (e) of the first aspect:
SEQ. ID. NO. 12 (designated G1-5 in the Examples) Ac-cyclo(1,10)-CEAGPSQWHCTWRY-NH₂ (C denotes disulfide bridge)
SEQ. ID. NO. 13. (designated G1-6 in the Examples) Ac-cyclo(1,10)-CEAGPSQWHCEWRY-NH₂ (C denotes disulfide bridge)
SEQ. ID. NO. 67. Ac-cyclo(1,10)-AglₛEAGPSQWHAglₛTWRY-NH₂ (underlined Aglₛ denotes stapled C-C bond between the precursor Agl side chains followed by hydrogenation reaction)
SEQ. ID. NO. 68. Ac-GFWFG-NH(CH₂)₂OCH₂CO-cyclo(1,10)-AglₛEAGPSQWHAglₛTWRY-NH₂ (underlined Aglₛ denotes stapled C-C bond between the precursor Agl side chains followed by hydrogenation reaction)
SEQ. ID. NO. 69. C₆H₅CH₂COOCH₂OCO-cyclo(1,10)-AglₛEAGPSQWHAglₛTWRY-NH₂ (underlined Aglₛ denotes stapled C-C bond between the precursor Agl side chains followed by hydrogenation reaction)

Especially preferred peptides and peptidomimetics in accordance with part (f) of the first aspect:
SEQ. ID. NO. 14 (designated G2-1 in the Examples) cyclo(1,14)-[L 2-NaI L W K t E W E K S K p P] (small caps single letter AA code denote D-amino acid)
SEQ. ID. NO. 15 (designated G2-2 in the Examples) cyclo(1,14)-[L 2-NaI L W K W E W E K S K p P] (small caps single letter AA code denote D-amino acid)
SEQ. ID. NO. 16 (designated G2-3 in the Examples) cyclo(1,14)-[L 2-NaI L W K l E W E K Cit K p P] (small caps single letter AA code denote D-amino acid)
SEQ. ID. NO. 17 (designated G2-4 in the Examples) cyclo(1,14)-[L 2-NaI L W K l E W E K R K p P] (small caps single letter AA code denote D-amino acid)
SEQ. ID. NO. 18 (designated G2-5 in the Examples) cyclo(1,14)-[L 2-NaI L W K t E W E K Cit K p P] ((small caps single letter AA code denote D-amino acid)
SEQ. ID. NO. 19 (designated G2-6 in the Examples) cyclo(1,14)-[L 2-NaI L W K W E W E K R K p P] (small caps single letter AA code denote D-amino acid)
SEQ. ID. NO. 20 (designated G2-6Pra in the Examples) cyclo(1,14)-[L 2-NaI L W K W E W E K R Pra p P] (small caps single letter AA code denote D-amino acid)
SEQ. ID. NO. 21 (designated G2-7 in the Examples) cyclo(1,14)-[L 2-NaI L W K e E W E K R K p P] (small caps single letter AA code denote D-amino acid)
SEQ. ID. NO. 22 (designated G2-8 in the Examples) cyclo(1,14)-[T F L W K W E W T K R K p P] (small caps single letter AA code denote D-amino acid)
SEQ. ID. NO. 23. cyclo(1,14)-[T F L W K W E W E K R K p P] (small caps single letter AA code denote D-amino acid)
SEQ. ID. NO. 24. cyclo(1,14)-[T F K W K W E W E K R L p P] (small caps single letter AA code denote D-amino acid)
SEQ. ID. NO. 25. cyclo(1,14)-[T F L W K A E W T K S K p P] (small caps single letter AA code denote D-amino acid)
SEQ. ID. NO. 26. cyclo(1,14)-[ L 2-NaI L W K t E W E K hSer K p P] (small caps single letter AA code denote D-amino acid)
SEQ. ID. NO. 70. cyclo(1,14)-[L 2-NaI L W K W E W E K R K X Z] (X refers to NH₂COCH₂NH-Allylamine, Z refers to 4-pentenoic acid, underlined X and Z denote hydrocarbon stapled C=C bond between their side chains as shown in Figure below) 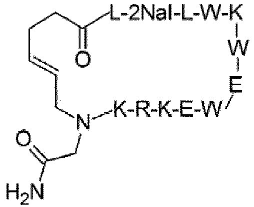
SEQ. ID. NO. 71. cyclo(1,13)-[L 2NaI L W K W E W E K R K ^{δ}Orn] (as shown in Figure below)

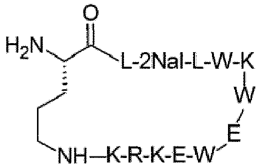

Most preferred in accordance with part (f) of the first aspect is the above compound comprising the sequences of SEQ ID NO: 19 and 22.

Especially preferred peptides and peptidomimetics in accordance with part (g) of the first aspect:
SEQ. ID. NO. 27 (designated G3-1 in the Examples) Ac-cyclo(5,9)-RWPQXKWDXQVRRW-NH₂ (X refers to (*S*)-2-(4'-pentenyl)Ala, underlined X denotes hydrocarbon-stapled C=C bond between the side chains of X)
SEQ. ID. NO. 28 (designated G3-1Pra in the Examples) Ac-cyclo(5,9)-RWPPraXKWDXQVRRW-NH₂ (X refers to (S)-2-(4'-pentenyl)Ala, underlined X denotes hydrocarbon-stapled C=C bond between the side chains of X)
SEQ. ID. NO. 29 (designated G3-2 in the Examples) Ac-cyclo(5,9)-REPQXIKDXQVRRW-NH₂ (X refers to (*S*)-2-(4'-pentenyl)Ala, underlined X denotes hydrocarbon-stapled C=C bond between the side chains of X)
SEQ. ID. NO. 30 (designated G3-3 in the Examples) Ac-cyclo(5,9)-RWPQXIKDXQVRWW-NH₂ (X refers to (*S*)-2-(4'-pentenyl)Ala, underlined X denotes hydrocarbon-stapled C=C bond between the side chains of X)
SEQ. ID. NO. 72. Ac-cyclo(5,9)-RWPQXₛIKDXₛQVRWW-NH₂ (X refers to (*S*)-2-(4'-pentenyl)Ala, underlined Xₛ denotes hydrocarbon-stapled C-C bond between the side chains of X followed by hydrogenation reaction)
SEQ. ID. NO. 73. Ac-GFWFG-NH(CH₂)₂OCH₂CO-cyclo(5,9)-RWPQXIKDXQVRWW-NH₂ (X refers to (S)-2-(4'-pentenyl)Ala, underlined X denotes hydrocarbon-stapled C=C bond between the side chains of X)
SEQ. ID. NO. 74. C₆H₅CH₂COOCH₂OCO-cyclo(5,9)-RWPQXIKDXQVRWW-NH₂ (X refers to (*S*)-2-(4'-pentenyl)Ala, underlined X denotes hydrocarbon-stapled C=C bond between the side chains of X)
SEQ. ID. NO. 96. CH₃CH₂COOCH₂OCO-Sar-cyclo(5,9)-RWPQXIKDXQVRWW-NH₂ (X refers to (*S*)-2-(4'-pentenyl)Ala, underlined X denotes hydrocarbon-stapled C=C bond between the side chains of X)
SEQ. ID. NO. 75 Ac-cyclo(5,9)-RWPQPraIKDK(N3)QVRWW-NH₂ (underlined Pra and K(N3) denotes that a triazole group connects the side chains of Pra and K(N3))
SEQ. ID. NO. 99. Ac-RWPQAibKWDAibQVRRW-NH₂ (Aib means α-aminoisobutyric acid)

Most preferred in accordance with part (g) of the first aspect is the above compound comprising the sequence of SEQ ID NO: 30.

Especially preferred peptides and peptidomimetics in accordance with part (h) of the first aspect:
SEQ. ID. NO. 76. Ac-cyclo(3,7)(8,12)-EWKWWIDKIQVDRA-NH₂ (underlined K and D denote lactam bridge between their side chains)
SEQ. ID. NO. 77. Ac-cyclo(3,7)(8,12)-EWKWWIDKIQVDWA-NH₂ (underlined K and D denote lactam bridge between their side chains)
SEQ. ID. NO. 78. Ac-cyclo(3,7)(8,12)-EWKWRIDKIQVDRA-NH₂ (underlined K and D denote lactam bridge between their side chains)
SEQ. ID. NO. 79. Ac-GFWFG-NH(CH₂)₂OCH₂CO-cyclo(3,7)(8,12)-EWKWWIDKIQVDRA-NH₂ (underlined K and D denote lactam bridge between their side chains)
SEQ. ID. NO. 80. C₆H₅CH₂COOCH₂OCO-cyclo(3,7)(8,12)-EWKWWIDKIQVDRA-NH₂ (underlined K and D denote lactam bridge between their side chains)
SEQ. ID. NO. 97. CH₃CH₂COOCH₂OCO-Sar-cyclo(3,7)(8,12)-EWKWWIDKIQVDRA-NH₂ (underlined K and D denote lactam bridge between their side chains)

Most preferred in accordance with part (h) of the first aspect are the above compounds comprising the sequences of SEQ ID NO: 76, 77 and 78, respectively.

Especially preferred peptides and peptidomimetics in accordance with part (i) of the first aspect:
SEQ. ID. NO. 81. Ac-cyclo(4,8)(11,15)-EWWKRIKDIQKWRRD-NH₂ (underlined K and D denote lactam bridge between their side chains)
SEQ. ID. NO. 82. Ac-cyclo(4,8)(11,15)-EWEKRIKDIQKWRRD-NH₂ (underlined K and D denote lactam bridge between their side chains)
SEQ. ID. NO. 83. Ac-cyclo(4,8)(11,15)-EWWKRIKDIQKWWRD-NH₂ (underlined K and D denote lactam bridge between their side chains)
SEQ. ID. NO. 84. Ac-GFWFG-NH(CH₂)₂OCH₂CO-cyclo(4,8)(11,15)-EWWKRIKDIQKWRRD-NH₂ (underlined K and D denote lactam bridge between their side chains)
SEQ. ID. NO. 85. C₆H₅CH₂COOCH₂OCO-cyclo(4,8)(11,15)-EWWKRIKDIQKWRRD-NH₂ (underlined K and D denote lactam bridge between their side chains)
SEQ. ID. NO. 86. CH₃COOCH₂OCO-cyclo(4,8)(11,15)-EWWKRIKDIQKWRRD-NH₂ (underlined K and D denote lactam bridge between their side chains)
SEQ. ID. NO. 98. CH₃CH₂COOCH₂OCO-Sar-cyclo(4,8)(11,15)-EWWKRIKDIQKWRRD-NH₂ (underlined K and D denote lactam bridge between their side chains)

Most preferred in accordance with part (i) of the first aspect are the above compounds comprising the sequences of SEQ ID NO: 81, 82 and 83, respectively.

The present invention furthermore relates to the following items:
1. A peptide or peptidomimetic comprising or consisting of the following sequence
   (a) X1-X2-X3-X4-X5-X6-X7-X8-X9-X10-X11-X12-X13-X14
      wherein
      a. X1 is an alpha-amino acid, preferably a proteinogenic amino acid, more preferably Glu or Nmglu;
      b. X2 is an alpha-amino acid, preferably a proteinogenic amino acid, or preferably selected from Trp, 2-NaI and 1-NaI;
      c. X3 is an alpha-amino acid, preferably a proteinogenic amino acid, or preferably selected from Trp, 2-NaI, 1-NaI and Pra;
      d. X4 is an alpha-amino acid, preferably a proteinogenic amino acid, or preferably selected from Lys, Cys, D-Cys, Asp, Glu, Dap, Aib and (R)-2-(7' -octenyl)Ala;
      e. X5 is an alpha-amino acid, preferably a proteinogenic amino acid, more preferably Ile or Lys;
      f. X6 is an alpha-amino acid, preferably a proteinogenic amino acid, more preferably Leu or Lys;
      g. X7 is an alpha-amino acid, preferably a proteinogenic amino acid, more preferably Asp or Glu;
      h. X8 is an alpha-amino acid, preferably a proteinogenic amino acid, more preferably Ile or Leu;
      i. X9 is an alpha-amino acid, preferably a proteinogenic amino acid, more preferably Gln;
      j. X10 is an alpha-amino acid, preferably a proteinogenic amino acid, more preferably Arg;
      k. X11 is an alpha-amino acid, preferably a proteinogenic amino acid, or preferably Asp, Glu, Cys, D-Cys, Aib or (S)-2-(4'-pentenyl)Ala;
      l. X12 is an alpha-amino acid, preferably a proteinogenic amino acid, more preferably Arg;
      m. X13 is an alpha-amino acid, preferably a proteinogenic amino acid, more preferably Arg;
      n. X14 is an alpha-amino acid, preferably a proteinogenic amino acid, more preferably Ala;
   (b) X1-X2-X3-X4-X5-X6-X7-X8-X9-X10-X11-X12
      wherein
      a. X1 is an amino acid, preferably an alpha-amino acid, more preferably a proteinogenic amino acid, or preferably selected from Arg, Phe(2-guanidino) and Phe(3-guanidino);
      b. X2 is an alpha-amino acid, preferably a proteinogenic amino acid, or preferably selected from Trp, Phe, Tyr, Ile, Leu, Cys, L-propargylglycine (Pra), L-homopropargylglycine (Hpg), allylglycine (Agl), prenylglycine (Pre), crotylglycine (Crt) and (S)-2-amino-4-bromobutyric acid;
      c. X3 is an alpha-amino acid, preferably a proteinogenic amino acid, or preferably selected from Ser, His and Dab;
      d. X4 is an alpha-amino acid, preferably a proteinogenic amino acid, or preferably selected from Trp, Cys, Pra, Hpg, Agl, Pre, Crt and (S)-2-amino-4-bromobutyric acid;
      e. X5 is an alpha-amino acid, preferably a proteinogenic amino acid, or preferably Gly or D-Ala;
      f. X6 is an alpha-amino acid, preferably a proteinogenic amino acid, or preferably Pro or D-Pro;
      g. X7 is an alpha-amino acid, preferably a proteinogenic amino acid, or preferably selected from Glu, Arg, Trp, Cit, Orn and pSer;
      h. X8 is an alpha-amino acid, preferably a proteinogenic amino acid, more preferably Thr;
      i. X9 is an alpha-amino acid, preferably a proteinogenic amino acid, or preferably selected from Phe, Cys, Agl, Pre, Crt, 3-azido-L-alanine (Dap(N3)), (S)-2-amino-4-azido-butyric acid (2Abu(y-N3), 5-azido-noravline (Nva(δ-N3), and (S)-2-amino-4-bromobutyric acid;
      j. X10 is an alpha-amino acid, more preferably a proteinogenic amino acid, or preferably selected from Trp, 2-NaI and 1-NaI;
      k. X11 is an alpha-amino acid, preferably a proteinogenic amino acid, or preferably selected from Trp, Phe, Tyr, Ile, Leu, Cys, (S)-2-amino-4-bromobutyric acid, Dap(N3), 2Abu(γ-N3), Nva(δ-N3), Agl and Crt;
      l. X12 is an alpha-amino acid, preferably a proteinogenic amino acid, or preferably selected from Arg, Phe(3-guanidino), Phe(4-guanidino), Cit and Orn;
   (c) X1-X2-X3-X4-X5-X6-X7-X8-X9-X10-X11-X12-X13-X14-X15-X16
      wherein
      a. X1 is an alpha-amino acid, preferably a proteinogenic amino acid, more preferably Thr;
      b. X2 is an alpha-amino acid, preferably a proteinogenic amino acid, or preferably selected from Ser, His and hSer;
      c. X3 is an alpha-amino acid, preferably a proteinogenic amino acid, or preferably selected from Cys, Pra, Hpg, Agl, Crt and (S)-2-amino-4-bromobutyric acid;
      d. X4 is an alpha-amino acid, preferably a proteinogenic amino acid, more preferably Asp;
      e. is an alpha-amino acid, preferably a proteinogenic amino acid, more preferably Asp;
      f. X6 is an alpha-amino acid, preferably a proteinogenic amino acid, more preferably Pro;
      g. is an alpha-amino acid, preferably a proteinogenic amino acid, or preferably Glu, Trp or Aad;
      h. X8 is an alpha-amino acid, preferably a proteinogenic amino acid, more preferably Thr;
      i. X9 is an alpha-amino acid, preferably a proteinogenic amino acid, or preferably selected from Trp, 2-NaI and 1-NaI;
      j. X10 is a group consisting of any natural amino acid, preferably a proteinogenic amino acid, more preferably Arg;
      k. X11 is alpha-amino acid, preferably a proteinogenic amino acid, or preferably selected from Trp, 2-NaI and 1-NaI;
      l. X12 is an alpha-amino acid, preferably a proteinogenic amino acid, more preferably Asn;
      m. X13 is an alpha-amino acid, preferably a proteinogenic amino acid, or preferably selected from Cys, (S)-2-amino-4-bromobutyric acid, Dap(N3), 2Abu(γ-N3), Nva(δ-N3), Agl and Crt;
      n. X14 is a group consisting of any natural amino acid, preferably a proteinogenic amino acid, more preferably Gln;
      o. X15 is alpha-amino acid, preferably a proteinogenic amino acid, or preferably selected from Arg, Phe(3-guanidino), Phe(4-guanidino) and Cit;
      p. X16 is an alpha-amino acid, preferably a proteinogenic amino acid, more preferably Val;
      wherein a side chain of X3 is covalently connected to a side chain of X13;
   (d) X1-X2-X3-X4-X5-X6-X7-X8-X9-X10-X11-X12-X13-X14-X25-X16-X17
      wherein
      a. X1 is an alpha-amino acid, preferably a proteinogenic amino acid, more preferably Gly;
      b. X2 is an alpha-amino acid, preferably a proteinogenic amino acid, more preferably Asp;
      c. X3 is an alpha-amino acid, preferably a proteinogenic amino acid, or preferably Thr or hSer;
      d. X4 is an alpha-amino acid, preferably a proteinogenic amino acid, or preferably selected from Cys, Pra, Hpg, Agl, Crt and (S)-2-amino-4-bromobutyric acid;
      e. X5 is an alpha-amino acid, preferably a proteinogenic amino acid, more preferably Asp;
      f. X6 is an alpha-amino amino acid, preferably a proteinogenic amino acid, more preferably Asp;
      g. X7 is an alpha-amino amino acid, preferably a proteinogenic amino acid, more preferably Pro;
      h. X8 is an alpha-amino acid, preferably a proteinogenic amino acid, or preferably Glu, Trp or Aad;
      i. X9 is an alpha-amino acid, preferably a proteinogenic amino acid, more preferably Thr;
      j. X10 is an alpha-amino acid, preferably a proteinogenic amino acid, or preferably selected from Trp, 2-NaI and 1-NaI;
      k. X11 is an alpha-amino acid, preferably a proteinogenic amino acid, more preferably Arg;
      l. X12 is an alpha-amino acid, preferably a proteinogenic amino acid, or preferably selected from Trp, 2-NaI and 1-NaI;
      m. X13 is an alpha-amino acid, preferably a proteinogenic amino acid, more preferably Asn;
      n. X14 is an alpha-amino acid, preferably a proteinogenic amino acid, or preferably selected from Cys, (S)-2-amino-4-bromobutyric acid, Dap(N3), 2Abu(γ-N3), Nva(δ-N3), Pra, Agl and Crt;
      o. X15 is an alpha-amino acid, preferably a proteinogenic amino acid, more preferably Gln;
      p. X16 is an alpha-amino acid, preferably a proteinogenic amino acid, or preferably Arg or Cit;
      q. X17 is an alpha-amino acid, preferably a proteinogenic amino acid, more preferably His;
      wherein a side chain of X4 is covalently connected to a side chain of X14;
   (e) X1-X2-X3-X4-X5-X6-X7-X8-X9-X10-X11-X12-X13-X14
      wherein
      a. X1 is an alpha-amino acid, preferably a proteinogenic amino acid, or preferably selected from Cys, Pra, Hpg, Agl, Crt and (S)-2-amino-4-bromobutyric acid;
      b. X2 is an alpha-amino acid, preferably a proteinogenic amino acid, more preferably Glu;
      c. X3 is an alpha-amino acid, preferably a proteinogenic amino acid, or preferably Ala or Aib;
      d. X4 is an alpha-amino acid, preferably a proteinogenic amino acid, more preferably Gly;
      e. X5 is an alpha-amino acid, preferably a proteinogenic amino acid, more preferably Pro;
      f. X6 is an alpha-amino acid, preferably a proteinogenic amino acid, more preferably Ser;
      g. X7 is an alpha-amino acid, preferably a proteinogenic amino acid, more preferably Gln;
      h. X8 is an alpha-amino acid, preferably a proteinogenic amino acid, or preferably selected from Trp, 2-NaI and 1-NaI;
      i. X9 is an alpha-amino acid, preferably a proteinogenic amino acid, more preferably selected from His, Asn and Gln;
      j. X10 is an alpha-amino acid, preferably a proteinogenic amino acid, or preferably selected from Cys, (S)-2-amino-4-bromobutyric acid, Dap(N3), 2Abu(γ-N3), Nva(δ-N3), Agl and Crt;
      k. X11 is an alpha-amino acid, preferably a proteinogenic amino acid, more preferably Thr or Glu;
      l. X12 is an alpha-amino acid, preferably a proteinogenic amino acid, or preferably selected from Trp, 2-NaI and 1-NaI;
      m. X13 is an alpha-amino acid, preferably a proteinogenic amino acid, or preferably Arg or Cit;
      n. X14 is an alpha-amino acid, preferably a proteinogenic amino acid, more preferably Tyr;
      wherein a side chain of X1 is covalently connected to a side chain of X10;
   (f) X1-X2-X3-X4-X5-X6-X7-X8-X9-X10-X11-X12-X13-X14
      wherein
      a. X1 is an alpha-amino acid, preferably a proteinogenic amino acid, more preferably Leu or Thr;
      b. X2 is an alpha-amino acid, preferably a proteinogenic amino acid, or preferably selected from Trp, Phe and 2-NaI;
      c. X3 is an alpha-amino acid, preferably a proteinogenic amino acid, more preferably Leu;
      d. X4 is an alpha-amino acid, preferably a proteinogenic amino acid, more preferably Trp;
      e. X5 is an alpha-amino acid, preferably a proteinogenic amino acid, more preferably Lys;
      f. X6 is an alpha-amino acid, preferably a proteinogenic amino acid, or preferably selected from Ala, Trp, D-Leu, D-Thr and D-Glu;
      g. X7 is an alpha-amino acid, preferably a proteinogenic amino acid, more preferably Glu;
      h. X8 is an alpha-amino acid, preferably a proteinogenic amino acid, more preferably Trp;
      i. X9 is an alpha-amino acid, preferably a proteinogenic amino acid, more preferably Glu or Thr;
      j. X10 is an alpha-amino acid, preferably a proteinogenic amino acid, more preferably Lys;
      k. X11 is an alpha-amino acid, preferably a proteinogenic amino acid, or preferably selected from Arg, Ser, Cit and hSer;
      l. X12 is an alpha-amino acid, preferably a proteinogenic amino acid, more preferably Lys or Pra;
      m. X13 is an alpha-amino acid, preferably a non-proteinogenic amino acid, or preferably selected from D-Pro, allylamine, cysteamine and Orn;
      n. X14 is an alpha-amino acid, preferably a proteinogenic amino acid, more preferably selected from Pro, Aib or an acid comprising 4-pentenoyl or 3-bromopropionyl moiety; wherein
         1) the alpha-amino group of X1 is bound to the a carboxy group of X14; or
         2) if X13 is Orn, the alpha-amino group of X1 is bound to the alpha-carboxy group of X13; and the side chain of X13 is bound to the alpha-carboxy group of X12;
   (g) X1-X2-X3-X4-X5-X6-X7-X8-X9-X10-X11-X12-X13-X14,
      wherein
      a. X1 is an alpha-amino acid, preferably a proteinogenic amino acid, more preferably Arg or Nmarg;
      b. X2 is an alpha-amino acid, preferably a proteinogenic amino acid, or preferably selected from Trp, Glu, 2-NaI and 1-NaI;
      c. X3 is an alpha-amino acid, preferably a proteinogenic amino acid, more preferably selected from Pro, Trp and Glu;
      d. X4 is an alpha-amino acid, preferably a proteinogenic amino acid, or preferably selected from Gln, Leu, Arg, and Pra;
      e. X5 is an alpha-amino acid, preferably a proteinogenic amino acid, or preferably selected from Lys, Glu, Ser, Lys(N3), (S)-2-(4'-pentenyl)Ala and Aib;
      f. X6 is an alpha-amino acid, preferably a proteinogenic amino acid, more preferably Ile or Lys;
      g. X7 is an alpha-amino acid, preferably a proteinogenic amino acid, or preferably selected from Lys, Trp, 2-NaI and 1-NaI;
      h. X8 is an alpha-amino acid, preferably a proteinogenic amino acid, more preferably Asp or Glu;
      i. X9 is an alpha-amino acid, preferably a proteinogenic amino acid, or preferably selected from Asp, Lys, Ser, (S)-2-(4'-pentenyl)Ala, Pra, D-Pra and Aib;
      j. X10 is an alpha-amino acid, preferably a proteinogenic amino acid, more preferably Gln;
      k. X11 is an alpha-amino acid, preferably a proteinogenic amino acid, more preferably Arg or Val;
      l. X12 is an alpha-amino acid, preferably a proteinogenic amino acid, more preferably Arg or Thr;
      m. X13 is an alpha-amino acid, preferably a proteinogenic amino acid, more preferably Arg or Trp;
      n. X14 is an alpha-amino acid, preferably a proteinogenic amino acid, more preferably Trp or Arg;
   (h) X1-X2-X3-X4-X5-X6-X7-X8-X9-X10-X11-X12-X13-X14
      wherein
      a. X1 is an alpha-amino acid, preferably a proteinogenic amino acid, more preferably Glu or Nmglu;
      b. X2 is an alpha-amino acid, preferably a proteinogenic amino acid, or preferably selected from Trp, 2-NaI, and 1-NaI;
      c. X3 is an alpha-amino acid, preferably a proteinogenic amino acid, more preferably Lys, Glu, Ser, Lys(N3) or (S)-2-(4'-pentenyl)Ala;
      d. X4 is an alpha-amino acid, preferably a proteinogenic amino acid, more preferably selected from Trp, 2-NaI, and 1-NaI;
      e. X5 is an alpha-amino acid, preferably a proteinogenic amino acid, more preferably Ile, Trp or Arg;
      f. X6 is an alpha-amino acid, preferably a proteinogenic amino acid, more preferably selected from Ile, Leu and Lys;
      g. X7 is an alpha-amino acid, preferably a proteinogenic amino acid, more preferably Asp, Lys, Ser, Pra, D-Pra or (S)-2-(4'-pentenyl)Ala;
      h. X8 is an alpha-amino acid, preferably a proteinogenic amino acid, more preferably Lys, Glu, Ser, Lys(N3) or (S)-2-(4'-pentenyl)Ala;
      i. X9 is an alpha-amino acid, preferably a proteinogenic amino acid, more preferably Ile;
      j. X10 is an alpha-amino acid, preferably a proteinogenic amino acid, more preferably Gln;
      k. X11 is an alpha-amino acid, preferably a proteinogenic amino acid, more preferably Ala or Val;
      l. X12 is an alpha-amino acid, preferably a proteinogenic amino acid, more preferably Asp, Lys, Ser, Pra, D-Pra or (S)-2-(4'-pentenyl)Ala;
      m. X13 is an alpha-amino acid, preferably a proteinogenic amino acid, more preferably selected from Arg, Lys, and Trp;
      n. X14 is an alpha-amino acid, preferably a proteinogenic amino acid, more preferably Ala or Trp;
      wherein a side chain of X3 is covalently connected to a side chain of X7; and
      wherein a side chain of X8 is covalently connected to a side chain of X12;
      or
   (i) X1-X2-X3-X4-X5-X6-X7-X8-X9-X10-X11-X12-X13-X14-X15
      wherein
      a. X1 is an alpha-amino acid, preferably a proteinogenic amino acid, more preferably Glu, Arg, Nmglu or Nmarg;
      b. X2 is an alpha-amino acid, preferably a proteinogenic amino acid, or preferably selected from Trp, 2-NaI and 1-NaI;
      c. X3 is an alpha-amino acid, preferably a proteinogenic amino acid, or preferably selected from Trp, 2-NaI, 1-NaI and Glu;
      d. X4 is an alpha-amino acid, preferably a proteinogenic amino acid, more preferably Lys, Glu, Ser, Lys(N3) or (S)-2-(4'-pentenyl)Ala;
      e. X5 is an alpha-amino acid, preferably a proteinogenic amino acid, more preferably Ile or Arg;
      f. X6 is an alpha-amino acid, preferably a proteinogenic amino acid, more preferably selected from Ile, Leu and Lys;
      g. X7 is an alpha-amino acid, preferably a proteinogenic amino acid, more preferably Lys;
      h. X8 is an alpha-amino acid, preferably a proteinogenic amino acid, more preferably Asp, Lys, Ser, Pra, D-Pra or (S)-2-(4'-pentenyl)Ala;
      i. X9 is an alpha-amino acid, preferably a proteinogenic amino acid, more preferably Ile;
      j. X10 is an alpha-amino acid, preferably a proteinogenic amino acid, more preferably Gln;
      k. X11 is an alpha-amino acid, preferably a proteinogenic amino acid, more preferably Lys, Glu, Ser, Lys(N3) or (S)-2-(4'-pentenyl)Ala;
      l. X12 is an alpha-amino acid, preferably a proteinogenic amino acid, more preferably Trp or Ile;
      m. X13 is an alpha-amino acid, preferably a proteinogenic amino acid, more preferably selected from Arg, Lys and Trp;
      n. X14 is an alpha-amino acid, preferably a proteinogenic amino acid, more preferably Arg or Trp;
      o. X15 is an alpha-amino acid, preferably a proteinogenic amino acid, more preferably Asp, Lys, Ser, Pra, D-Pra or (S)-2-(4'-pentenyl)Ala;
      wherein a side chain of X4 is covalently connected to a side chain of X8; and
      wherein a side chain of X11 is covalently connected to a side chain of X15;.
2. The peptide or peptidomimetic of item 1(a), wherein
   X2 is selected from Trp, 2-NaI and 1-NaI, being preferred Trp;
   X4 is selected from Lys, Cys, D-Cys, Asp, Glu, Dap, Aib and (*R*)-2-(7'-octenyl)Ala, being preferred (*R*)-2-(7'-octenyl)Ala;
   X5 is Ile or Lys, being preferred Ile;
   X8 is Ile or Leu, being preferred Ile;
   X9 is Gln;
   X11 is Asp, Glu, Cys, D-Cys, Aib or (*S*)-2-(4'-pentenyl)Ala, being preferred (*S*)-2-(4'-pentenyl)Ala;
   and
   X12 is Arg.
3. The peptide or peptidomimetic of item 1 (b), wherein
   X1 is selected from Arg, Phe(2-guanidino) and Phe(3-guanidino), being preferred Arg;
   X2 is selected from Trp, Phe, Tyr, Ile, Leu, Cys, Pra, Hpg, Agl, Pre, Crt and (S)-2-amino-4-bromobutyric acid, being preferred Cys and Agl;
   X3 is selected from Ser, His and Dab, being preferred His;
   X6 is Pro or D-Pro, being preferred Pro;
   X7 is selected from Glu, Arg, Trp, Cit, Orn and pSer, being preferred Arg;
   X8 is Thr;
   X10 is selected from Trp, 2-NaI and 1-NaI, being preferred Trp;
   X11 is selected from Trp, Phe, Tyr, Ile, Leu, Cys, (S)-2-amino-4-bromobutyric acid, Dap(N3), 2-amino-4-azido-butyric acid (2Abu(γ-N3)), 5-azido-norvaline (Nva(δ-N3)), Agl and Crt, being preferred Cys and Agl;
   and
   X12 is selected from Arg, Phe(3-guanidino), Phe(4-guanidino), Cit and Orn, being preferred Arg.
4. The peptide or peptidomimetic of item 1(c), wherein
   X2 is selected from Ser, His and hSer, being preferred His;
   X3 is selected from Cys, Pra, Hpg, Agl, Crt and (S)-2-amino-4-bromobutyric acid, being preferred Cys and Agl;
   X6 is Pro;
   X7 is Glu, Trp or Aad, being preferred Glu;
   X8 is Thr;
   X9 is selected from Trp, 2-NaI and 1-NaI, being preferred Trp;
   X11 is selected from Trp, 2-NaI and 1-NaI, being preferred Trp;
   X12 is Asn;
   X13 is selected from Cys, (S)-2-amino-4-bromobutyric acid, Dap(N3), 2Abu(γ-N3), Nva(δ-N3), Agl and Crt, being preferred Cys and Agl;
   and
   X15 is selected from Arg, Phe(3-guanidino), Phe(4-guanidino) and Cit, being preferred Arg.
5. The peptide or peptidomimetic of item 1(d), wherein
   X3 is Thr or hSer, being preferred Thr;
   X4 is selected from Cys, Pra, Hpg, Agl, Crt and (S)-2-amino-4-bromobutyric acid, being preferred Cys and Agl;
   X8 is Glu, Trp, or Aad, being preferred Glu;
   X9 is Thr;
   X10 is selected from Trp, 2-NaI and 1-NaI, being preferred Trp;
   X12 is selected from Trp, 2-NaI and 1-NaI, being preferred Trp;
   X13 is Asn;
   X14 is selected from Cys, (S)-2-amino-4-bromobutyric acid, Dap(N3), 2Abu(γ-N3), Nva(δ-N3), Agl and Crt, being preffered Cys and Agl;
   and
   X16 is Arg or Cit, being preferred Arg.
6. The peptide or peptidomimetic of item 1(e), wherein
   X1 is selected from Cys, Pra, Hpg, Agl, Crt and (S)-2-amino-4-bromobutyric acid, being preferred Cys and Agl;
   X2 is Glu;
   X5 is Pro;
   X8 is selected from Trp, 2-NaI and 1-NaI, being preferred Trp;
   X9 is selected from His, Asn and Gln, being preferred His;
   X10 is selected from Cys, (S)-2-amino-4-bromobutyric acid, Dap(N3), 2Abu(γ-N3), Nva(δ-N3), Agl and Crt, being preferred Cys and Agl;
   X12 is selected from Trp, 2-NaI and 1-NaI, being preferred Trp;
   and
   X13 is Arg or Cit, being preferred Arg.
7. The peptide or peptidomimetic of item 1(f), wherein
   X2 is selected from Trp, Phe and 2-NaI, being preferred Phe and 2-NaI;
   X4 is Trp;
   X6 is selected from Ala, Trp, D-Leu, D-Thr and D-Glu, being preferred Trp;
   X7 is Glu;
   X9 is Glu or Thr;
   X11 is selected from Arg, Ser, Cit and hSer, being preferred Arg;
   X13 is selected from D-Pro, allylamine, cysteamine and Orn, being preferred D-Pro;
   and
   X14 is selected from Pro, Aib, 4-pentenoic acid and 3-bromopropionic acid, being preferred Pro.
8. The peptide or peptidomimetic of item 1(g), wherein
   X2 is selected from Trp, Glu, 2-NaI and 1-NaI, being preferred Trp;
   X5 is selected from Lys, Glu, Lys(N3), (S)-2-(4'-pentenyl)Ala and Aib, being preferred (S)-2-(4'-pentenyl)Ala;
   X6 is Ile or Lys, being preferred Ile;
   X9 is selected from Asp, Lys, (S)-2-(4'-pentenyl)Ala, Pra, D-Pra and Aib, being preferred (S)-2-(4'-pentenyl)Ala;
   X10 is Gln;
   X13 is Arg or Trp, being preferred Trp;
   and
   X14 is Trp or Arg, being preferred Trp.
9. The peptide or peptidomimetic of item 1(h), wherein
   X2 is selected from Trp, 2-NaI and 1-NaI, being preferred Trp;
   X3 is Lys, Glu, Ser, Lys(N3) or (S)-2-(4'-pentenyl)Ala, being preferred Lys;
   X5 is Ile, Trp or Arg, being preferred Trp;
   X6 is selected from Ile, Leu and Lys, being preferred Ile;
   X7 is Asp, Lys, Ser, Pra, D-Pra or (S)-2-(4'-pentenyl)Ala, being preferred Asp;
   X8 is Lys, Glu, Ser, Lys(N3) or (S)-2-(4'-pentenyl)Ala, being preferred Lys;
   X9 is Ile;
   X10 is Gln;
   X12 is Asp, Lys, Ser, Pra, D-Pra or (S)-2-(4'-pentenyl)Ala being preferred Asp;
   and
   X13 is selected from Arg, Lys, and Trp, being preferred Arg.
10. The peptide or peptidomimetic of item 1(i), wherein
   X2 is selected from Trp, 2-NaI and 1-NaI, being preferered Trp;
   X4 is Lys, Glu, Ser, Lys(N3) or (S)-2-(4'-pentenyl)Ala, being preferred Lys;
   X6 is selected from Ile, Leu and Lys, being preferred Ile;
   X8 is Asp, Lys, Ser, Pra, D-Pra or (S)-2-(4'-pentenyl)Ala, being preferred Asp;
   X9 is Ile;
   X10 is Gln;
   X11 is Lys, Glu, Ser, Lys(N3) or (S)-2-(4'-pentenyl)Ala, being preferred Lys;
   X13 is selected from Arg, Lys, and Trp;
   and
   X15 is Asp, Lys, Ser, Pra, D-Pra or (S)-2-(4'-pentenyl)Ala, being preferred Asp.
11. The peptide or peptidomimetic of any one of the preceding items, wherein
   (a) X2 to X12 of said peptide or peptidomimetic of item 1 (a) assume an alpha-helix structure under physiological conditions;
   (b) said peptide or peptidomimetic of item 1 (b) assumes a beta-hairpin structure under physiological conditions, wherein preferably X1 to X4 and X9 to X12 are in beta-sheet conformation and/or a turn is formed by residues X5 to X8;
   (c) said peptide or peptidomimetic of item 1 (c) assumes a helix-turn-helix structure under physiological conditions, wherein preferably X1 to X4 and X10 to X15 are in alpha-helical conformation and/or a turn is formed by X5 to X9;
   (d) said peptide or peptidomimetic of item 1 (d) assumes a helix-turn-helix structure under physiological conditions, wherein preferably X2 to X5 and X11 to X16 are in alpha-helical conformation and/or a turn is formed by X6 to X10;
   (e) said peptide or peptidomimetic of item 1 (e) assumes a helix-turn structure under physiological conditions, wherein preferably X7 to X14 are in alpha-helical conformation and/or a turn is formed by X1 to X6;
   (f) said peptide or peptidomimetic of item 1 (f) assumes a beta-hairpin structure under physiological conditions, wherein X2 to X4 and X9 to X11 are in beta-sheet conformation and/or turns are formed by X5 to X8, and X12 to X14 and X1;
   (g) X2 to X13 of said peptide or peptidomimetic of item 1 (g) assume an alpha-helix structure under physiological conditions;
   (h) X1 to X12 of said peptide or peptidomimetic of item 1 (h) assume an alpha-helix structure under physiological conditions;
   (i) X1 to X15 of said peptide or peptidomimetic of item 1 (i) assume an alpha-helix structure under physiological conditions.
12. The peptide or peptidomimetic of any one of the preceding items, wherein alpha-amino acids located at positions 2 and/or 3 of a beta-turn are replaced with beta-turn inducing amino acids, wherein preferably said beta-turn inducing amino acids are selected from the following: 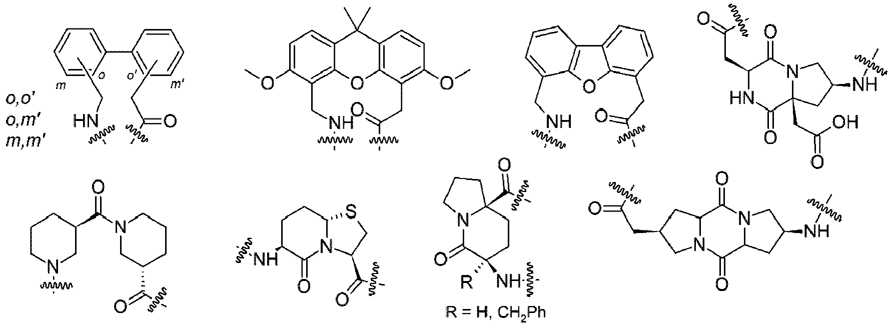
13. The peptide or peptidomimetic of any one of the preceding items, wherein said peptide or peptidomimetic is capable of interfering with ubiquitination.
14. The peptide or peptidomimetic of item 13, wherein said interfering is determined by bringing said peptide or peptidomimetic into contact with APC/C, Cdc20, and E2 enzyme (UBE2C, UBE2D or UBE2S), ubiquitin, a substrate (e.g. securin or cyclin B1) and ATP, and quantifying the amount of ubiquitinated susbtrate in presence and absence of said peptide or peptidomimetic, wherein a decreased amount of ubiquitinated substrate in presence of said peptide or peptidomimetic is indicative of said peptide or peptidomimetic being capable of interfering with ubiquitination.
15. The peptide or peptidomimetic of item 13 or 14, wherein said interfering is reducing or abolishing the activity of an ubiquitinating enzyme and/or of an ubiquitin ligase.
16. The peptide or peptidomimetic of item 15, wherein said enzyme is an E2 enzyme, preferably UBE2C, UbcH7, UBE2D or UBE2S; or said ligase is an E3 ligase, preferably APC11, Rbx1 or c-Cbl.
17. The peptide or peptidomimetic of any one of the preceding items, wherein said peptide or peptidomimetic stops cell division or induces cell death.
18. The peptide or peptidomimetic of item 17, wherein stopping or inducing is determined by bringing cells into contact with said peptide or peptidomimetic and determining whether said cells proliferate, stop dividing, or undergo cell death, wherein cells which do not divide or cell death is indicative of said peptide or peptidomimetic stopping cell division or inducing cell death.
19. The peptide or peptidomimetic of any one of the preceding items, wherein said peptide or peptidomimetic is modified
   (a) to improve its delivery, absorption, distribution, metabolism or excretion; and/or
   (b) by
      a. esterification of a carboxyl group;
      b. esterification of a hydroxyl group;
      c. formation of a pharmaceutically acceptable salt or complexes;
      d. introduction of a hydrophilic moiety;
      e. introduction, exchange or removal of a substituent on an aromatic ring or an aliphatic chain;
      f. conversion of an alkyl group into a cyclic form thereof;
      g. derivatization of a hydroxyl group to give rise to an acetal or ketal;
      h. derivatization of a triazole group to an isoxazole group;
      i. acetylation, alkylation, introduction of aldehyde or sulfonyl groups of a nitrogen as well as its transformation to carbamate; and/or
      j. derivatization of an aldehyde or a ketone to give rise to a Schiff's base, an oxime, an acetal, a ketal, an enol-ester, an oxazolidine, or a thiazolidine.
20. The peptide or peptidomimetic of item 1(a) and any one of items 2 to 19, to the extent they refer back to item 1(a), wherein
   (a) the alpha-amino group of X1 is bound to a moiety selected from acetyl; benzoyl; Biotin-Ahx; methyl, Ac-Gly-Phe-Trp-Phe-Gly; Ac-Gly-Phe-Trp-Phe-Gly-NH-(CH₂)₂-O-CH₂-CO-; Ac-Gly-Phe-Trp-Phe-Gly-NH-(CH₂)₂-PEG-O-CH₂-CO-, PEG being polyethylene glycol with a polymerization degree from 1 to 6; and R-COO-(CH₂)ₙ-OCO-, R-COO-(CH₂)ₙ-OCO-N(CH₃)-CH₂-CO-, R-COO-(CH₂)ₙ-OCOO-(CH₂)₂-CO-, or R-COO-(CH₂)ₙ-OCO-NH-(CH₂)₂-CO-, R being alkyl, cycloalkyl, cycloheteroalkyl, aryl or heteroaryl, alkyl having 1 to 6 carbon atoms, cycles having 4 to 10, preferably 5 or 6 ring atoms and 1 or 2 cycles, R preferably being methyl, ethyl, phenyl or benzyl, and n being 1 or 2;
   (b) X4 is (R)-2-(7'-octenyl)Ala and X11 is (S)-2-(4'-pentenyl)Ala, and a side chain of X4 is bound to a side chain of X11 by a covalent bond;
   (c) X4 and X11 are Asp or preferably Glu, and their side chains are bound through a diamine alkyl moiety consisting of -NH-CH₂-(CH₂)ₙ-CH₂-NH-, n being 1, 2 or 3;
   (d) X4 is D-Cys and X11 is Cys or D-Cys, and a side chain of X4 is bound to a side chain of X11 via an aryl moiety, preferably 1,1'-biphenyl-4,4'-bis(methyl) or 3,3'-bipyridine,6,6' -bis(methyl);
   (e) X4 and X11 are Cys, and a side chain of X4 is bound to a side chain of X11 via an azoaryl moiety, preferably N,N'-[(1Z)-azodi-4,1-phenylene]diacetamide or *cis*-3,3'-bis(sulfonato)-4,4'-bis(acetamide)azobenzene;
   (f) X4 is Dap and X11 is Asp, and a side chain of X4 is bound to a side chain of X11 via the aryl moiety -CO-CH₂-*p*-C₆H₄-CH₂-NH-, or -CO-CH₂-*p*-C₆F₄-CH₂-NH; or
   (g) X14 is amidated, esterified or bound to a moiety selected from Gly-Phe-Trp-Phe-Gly-NH₂, -NH-(CH₂)₂-O-CH₂-CO-Gly-Phe-Trp-Phe-Gly-NH₂ and -NH-(CH₂)₂-PEG-O-CH₂-CO-Gly-Phe-Trp-Phe-Gly-NH₂, PEG being polyethylene glycol with a polymerization degree from 1 to 6.
21. The peptide or peptidomimetic of item 20 (b), wherein said bond is as follows: 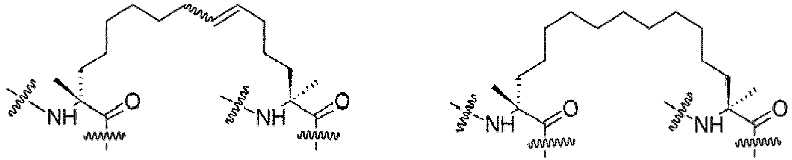
22. The peptide or peptidomimetic of item 20 (c), wherein said bond is as follows: 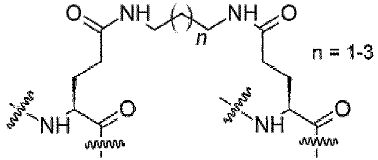
23. The peptide or peptidomimetic of item 20 (d), wherein said bond is as follows: 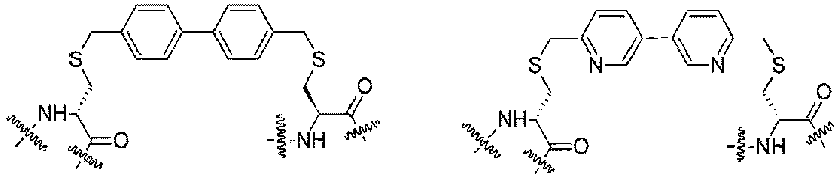
24. The peptide or peptidomimetic of item 20 (e), wherein said bond is as follows: 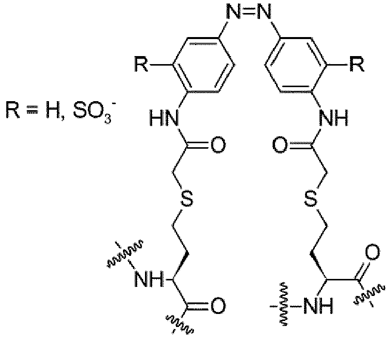
25. The peptide or peptidomimetic of item 20 (f), wherein said bond is as follows: 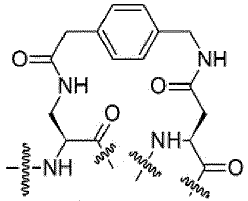
26. The peptide or peptidomimetic of item 1(b) and any one of items 2 to 19, to the extent they refer back to item 1(b), wherein
   (a) the alpha-amino group of X1 is bound to a moiety selected from acetyl; benzoyl; Biotin-Ahx; methyl, Ac-Gly-Phe-Trp-Phe-Gly; Ac-Gly-Phe-Trp-Phe-Gly-NH-(CH₂)₂-O-CH₂-CO-; Ac-Gly-Phe-Trp-Phe-Gly-NH-(CH₂)₂-PEG-O-CH₂-CO-, PEG being polyethylene glycol with a polymerization degree from 1 to 6; and R-COO-(CH₂)ₙ-OCO-, R-COO-(CH₂)ₙ-OCO-N(CH₃)-CH₂-CO-, R-COO-(CH₂)ₙ-OCOO-(CH₂)₂-CO-, or R-COO-(CH₂)ₙ-OCO-NH-(CH₂)₂-CO-, R being alkyl, cycloalkyl, cycloheteroalkyl, aryl or heteroaryl, alkyl having 1 to 6 carbon atoms, cycles having 4 to 10, preferably 5 or 6 ring atoms and 1 or 2 cycles, R preferably being methyl, ethyl, phenyl or benzyl, and n being 1 or 2;
   (b) wherein X2 and X11 are independently selected from Trp, Phe, Tyr, Ile and Leu;
   (c) wherein a side chain of X2 is covalently connected to a side chain of X11;
      a. wherein if X2 and X11 are Agl or Crt, respectively, the covalent connection may comprise a carbon-carbon double bond;
      b. wherein if X2 is Cys and X11 is (S)-2-amino-4-bromobutyric acid, a C-S bond connects X2 with X11;
      c. wherein if X2 is Pra and X11 is 2Abu(γ-N3) or Nva(δ-N3), a triazole group connects X2 with X11;
      d. wherein if X2 and X11 are Cys, a disulfide bridge connects X2 with X11;
      e. wherein if X2 is (S)-2-amino-4-bromobutyric acid and X11 is Cys, a C-S bond connects X2 with X11;
      f. wherein if X2 is Hpg and X11 Dap(N3), a triazole group connects X2 with X11
   (d) wherein X4 and X9 are independently selected from Trp and Phe;
   (e) wherein a side chain of X4 is covalently connected to a side chain of X9;
      a. wherein if X4 and X9 are Cys, a disulfide bridge connects X4 with X9;
      b. wherein if X4 and X9 are Agl, the covalent connection may comprise a carbon-carbon double bond;
      c. wherein if X4 and X9 are Crt, the covalent connection may comprise a carbon-carbon double bond;
      d. wherein if X4 is Cys and X9 is (S)-2-amino-4-bromobutyric acid, a C-S bond connects X4 with X9;
      e. wherein if X4 is (S)-2-amino-4-bromobutyric acid and X9 is Cys, a C-S bond connects X4 with X9;
      f. wherein if X4 is Pra and X11 is 2Abu(γ-N3) or Nva(δ-N3), a triazole group connects X4 with X9;
      g. wherein if X4 is Hpg and X11 Dap(N3), a triazole group connects X4 with X9; or
   (f) wherein the alpha-carboxy group of X12 is amidated or bound to a moiety selected from -OEt, -OMe, -NHEt, -NHMe, -Gly-Phe-Trp-Phe-Gly-NH₂, -NH-(CH₂)₂-O-CH₂-CO-Gly-Phe-Trp-Phe-Gly-NH₂, and -NH-(CH₂)₂-PEG-O-CH₂-CO-Gly-Phe-Trp-Phe-Gly-NH₂, PEG being polyethylene glycol with a polymerization degree from 1 to 6;
      wherein preferably the connections defined in parts (b) to (e) are implemented as follows: 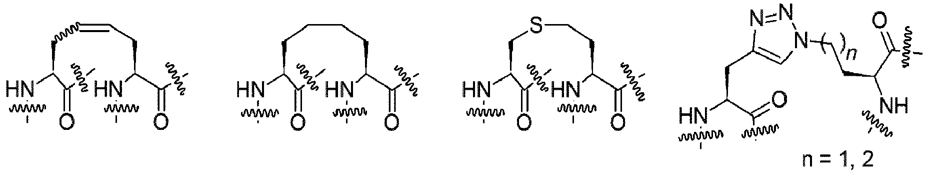
27. The peptide or peptidomimetic of item 1(c) and any one of items 2 to 19, to the extent they refer back to item 1(c), wherein
   (a) the alpha-amino group of X1 is bound to a moiety selected from acetyl; benzoyl; Biotin-Ahx; methyl, Ac-Gly-Phe-Trp-Phe-Gly; Ac-Gly-Phe-Trp-Phe-Gly-NH-(CH₂)₂-O-CH₂-CO-; Ac-Gly-Phe-Trp-Phe-Gly-NH-(CH₂)₂-PEG-O-CH₂-CO-, PEG being polyethylene glycol with a polymerization degree from 1 to 6; and R-COO-(CH₂)ₙ-OCO-, R-COO-(CH₂)ₙ-OCO-N(CH₃)-CH₂-CO-, R-COO-(CH₂)ₙ-OCOO-(CH₂)₂-CO-, or R-COO-(CH₂)ₙ-OCO-NH-(CH₂)₂-CO-, R being alkyl, cycloalkyl, cycloheteroalkyl, aryl or heteroaryl, alkyl having 1 to 6 carbon atoms, cycles having 4 to 10, preferably 5 or 6 ring atoms and 1 or 2 cycles, R preferably being methyl, ethyl, phenyl or benzyl, and n being 1 or 2;
   (b) X3 and X13 are Cys, and the covalent connection between X3 and X13 comprises a sulfur-sulfur bond;
   (c) X3 and X13 are Agl, and the covalent connection between X3 and X13 may comprise a carbon-carbon double bond;
   (d) X3 and X13 are Crt, and the covalent connection between X3 and X13 may comprise a carbon-carbon double bond;
   (e) X3 is Cys and X13 is (S)-2-amino-4-bromobutyric acid, and a C-S bond connects X3 with X13;
   (f) X3 is (S)-2-amino-4-bromobutyric acid and X13 is Cys, and a C-S bond connects X3 with X13;
   (g) X3 is Pra and X13 is 2Abu(γ-N3) or Nva(δ-N3), and a triazole group connects X3 with X13;
   (h) X3 is Hpg and X13 is Dap(N3), and a triazole group connects X3 with X13; or
   (i) the alpha-carboxy group of X16 is amidated or bound to a moiety selected from-OEt, -OMe, -NHEt, -NHMe, -Gly-Phe-Trp-Phe-Gly-NH₂, -NH-(CH₂)₂-O-CH₂-CO-Gly-Phe-Trp-Phe-Gly-NH₂ and -NH-(CH₂)₂-PEG-O-CH₂-CO-Gly-Phe-Trp-Phe-Gly-NH₂, PEG being polyethylene glycol with a polymerization degree from 1 to 6.
28. The peptide or peptidomimetic of item 1(d) and any one of items 2 to 19, to the extent they refer back to item 1(d), wherein
   (a) X4 and X14 are Cys, and the covalent connection between X4 and X14 comprises a sulfur-sulfur bond;
   (b) X4 and X14 are Agl, and the covalent connection between X4 and X14 may comprise a carbon-carbon double bond;
   (c) X4 and X14 are Crt, and the covalent connection between X4 and X14 may comprise a carbon-carbon double bond;
   (d) X4 is Cys and X14 is (S)-2-amino-4-bromobutyric acid, and a C-S bond connects X4 with X14;
   (e) X4 is (S)-2-amino-4-bromobutyric acid and X14 is Cys, and a C-S bond connects X4 with X14;
   (f) X4 is Pra and X14 is 2Abu(γ-N3) or Nva(δ-N3), and a triazole group connects X4 with X14;
   (g) X4 is Hpg and X14 is Dap(N3), and a triazole group connects X4 with X14; or
   (h) the alpha-carboxy group of X17 is amidated, esterified or bound to a moiety selected from Gly-Phe-Trp-Phe-Gly-NH₂, -NH-(CH₂)₂-O-CH₂-CO-G|y-Phe-Trp-Phe-Gly-NH₂ and -NH-(CH₂)₂-PEG-O-CH₂-CO-Gly-Phe-Trp-Phe-Gly-NH₂, PEG being polyethylene glycol with a polymerization degree from 1 to 6.
29. The peptide or peptidomimetic of item 1(e) and any one of items 2 to 19, to the extent they refer back to item 1(e), wherein
   (a) the alpha-amino group of X1 is bound to a moiety selected from acetyl; benzoyl; Biotin-Ahx; methyl, Ac-Gly-Phe-Trp-Phe-Gly; Ac-Gly-Phe-Trp-Phe-Gly-NH-(CH₂)₂-O-CH₂-CO-; Ac-Gly-Phe-Trp-Phe-Gly-NH-(CH₂)₂-PEG-O-CH₂-CO-, R-COO-(CH₂)ₙ-OCO-N(CH₃)-CH₂-CO-, R-COO-(CH₂)ₙ-OCOO-(CH₂)₂-CO- or R-COO-(CH₂)ₙ-OCO-NH-(CH₂)₂-CO-, PEG being polyethylene glycol with a polymerization degree from 1 to 6; and R-COO-(CH₂)ₙ-OCO-, R being alkyl, cycloalkyl, cycloheteroalkyl, aryl or heteroaryl, alkyl having 1 to 6 carbon atoms, cycles having 4 to 10, preferably 5 or 6 ring atoms and 1 or 2 cycles, R preferably being methyl, ethyl, phenyl or benzyl and n being 1 or 2;
   (b) X1 and X10 are Cys, and the covalent connection between X1 and X10 comprises a sulfur-sulfur bond;
   (c) X1 and X10 are Agl, and the covalent connection between X1 and X10 may comprise a carbon-carbon double bond;
   (d) X1 and X10 are Crt, and the covalent connection between X1 and X10 may comprise a carbon-carbon double bond;
   (e) X1 is Cys and X10 is (S)-2-amino-4-bromobutyric acid, and a C-S bond connects X1 with X10;
   (f) X1 is (S)-2-amino-4-bromobutyric acid and X10 is Cys, and a C-S bond connects X1 with X10;
   (g) X1 is Pra and X10 is 2Abu(γ-N3) or Nva(δ-N3), and a triazole group connects X1 with X10;
   (h) X1 is Hpg and X10 is Dap(N3), and a triazole group connects X1 with X10;
      or
   (i) X14 is amidated, esterified or functionalized with Gly-Phe-Trp-Phe-Gly-NH₂, -NH-(CH₂)₂-O-CH₂-CO-Gly-Phe- Trp-Phe-Gly-NH₂ and -NH-(CH₂)₂-PEG-O-CH₂-CO-Gly-Phe-Trp-Phe-Gly-NH₂, PEG being polyethylene glycol with a polymerization degree from 1 to 6.
30. The peptide or peptidomimetic of item 1(f) and any one of items 2 to 19, to the extent they refer back to item 1(f), wherein
   (a) X13 is allylamine or cysteamine, wherein the alpha-amino group of X13 is bound to a moiety selected from -CH₂CONH₂, -CH₂CO-Gly-Phe-Trp-Phe-Gly-NH₂, -CH₂COOEt and CH₂COOMe;
   (b) X14 comprises a 4-pentenoyl, bromoacetyl or 3-bromopropionyl moiety, wherein the carboxy group of said moiety is covalently bound to the alpha-amino group of X1; or
   (c) wherein a side chain of X13 is covalently connected to a side chain of X14;
      i. if X13 is allylamine and X14 is 4-pentenoic acid, the covalent connection may comprise a carbon-carbon double bond;
      ii. if X13 is cysteamine and X14 is 3-bromopropionyl, a carbon-sulfur bond or a sulfur-sulfur bond is comprised in the covalent connection;
         wherein preferably said covalent connection in accordance with (c) is selected from the following: 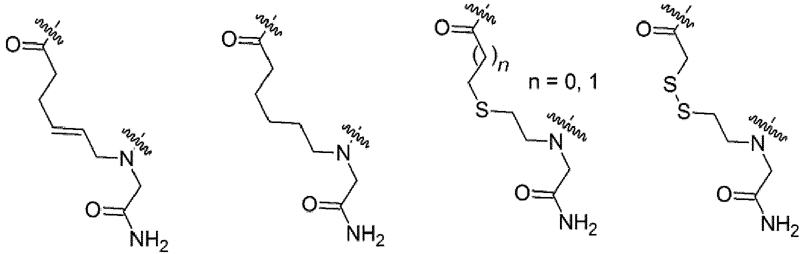
31. The peptide or peptidomimetic of item 1(g) and any one of items 2 to 19, to the extent they refer back to item 1(g), wherein
   (a) the alpha-amino group of X1 is bound to a moiety selected from acetyl; benzoyl; Biotin-Ahx; methyl, Ac-Gly-Phe-Trp-Phe-Gly; Ac-Gly-Phe-Trp-Phe-Gly-NH-(CH₂)₂-O-CH₂-CO-; Ac-Gly-Phe-Trp-Phe-Gly-NH-(CH₂)₂-PEG-O-CH₂-CO-, PEG being polyethylene glycol with a polymerization degree from 1 to 6; and R-COO-(CH₂)ₙ-OCO-, R-COO-(CH₂)ₙ-OCO-N(CH₃)-CH₂-CO-, R-COO-(CH₂)ₙ-OCOO-(CH₂)₂-CO- or R-COO-(CH₂)ₙ-OCO-NH-(CH₂)₂-CO-, R being alkyl, cycloalkyl, cycloheteroalkyl, aryl or heteroaryl, alkyl having 1 to 6 carbon atoms, cycles having 4 to 10, preferably 5 or 6 ring atoms and 1 or 2 cycles, R preferably being methyl, ethyl, phenyl or benzyl and n being 1 or 2;
   (b) if neither X5 not X9 are Aib, a side chain of X5 is covalently connected to a side chain of X9, wherein the covalent connection may comprise a carbon-carbon double bond;
   (c) X5 and X9 are Ser, and their side chains are bound through the adipoyl moiety - CO-(CH₂)₄-CO-;
      or
   (d) X14 is amidated, esterified or bound to a moiety selected from Gly-Phe-Trp-Phe-Gly-NH₂, -NH-(CH₂)₂-O-CH₂-CO-Gly-Phe-Trp-Phe-Gly-NH₂, and -NH-(CH₂)₂-PEG-O-CH₂-CO-Gly-Phe-Trp-Phe-Gly-NH₂, PEG being polyethylene glycol with a polymerization degree from 1 to 6.
32. The peptide or peptidomimetic of item 1(h) and any one of items 2 to 19, to the extent they refer back to item 1(h), wherein
   (a) the alpha-amino group of X1 is bound to a moiety selected from acetyl; benzoyl; Biotin-Ahx; methyl, Ac-Gly-Phe-Trp-Phe-Gly; Ac-Gly-Phe-Trp-Phe-Gly-NH-(CH₂)₂-O-CH₂-CO-; Ac-Gly-Phe-Trp-Phe-Gly-NH-(CH₂)₂-PEG-O-CH₂-CO-, PEG being polyethylene glycol with a polymerization degree from 1 to 6; and R-COO-(CH₂)ₙ-OCO-, R-COO-(CH₂)ₙ-OCO-N(CH₃)-CH₂-CO-, R-COO-(CH₂)ₙ-OCOO-(CH₂)₂-CO- or R-COO-(CH₂)ₙ-OCO-NH-(CH₂)₂-CO-, R being alkyl, cycloalkyl, cycloheteroalkyl, aryl or heteroaryl, alkyl having 1 to 6 carbon atoms, cycles having 4 to 10, preferably 5 or 6 ring atoms and 1 or 2 cycles, R preferably being methyl, ethyl, phenyl or benzyl and n being 1 or 2;
   (b) wherein X3 is Lys and X7 is Asp, or X3 is Glu and X7 is Lys;
   (c) wherein X8 is Lys and X12 is Asp, or X8 is Glu and X12 is Lys;
   (d) wherein X3 and X7 are Ser, and their side chains are bound through the adipoyl moiety -CO-(CH₂)₄-CO-;
   (e) wherein X8 and X12 are Ser, and their side chains are bound through the adipoyl moiety -CO-(CH₂)₄-CO-; or
   (f) the alpha-carboxy group of X14 is amidated, esterified or bound to a moiety selected from Gly-Phe-Trp-Phe-Gly-NH₂, -NH-(CH₂)₂-O-CH₂-CO-Gly-Phe-Trp-Phe-Gly-NH₂ and -NH-(CH₂)₂-PEG-O-CH₂-CO-Gly-Phe-Trp-Phe-Gly-NH₂, PEG being polyethylene glycol of different polymerization degrees, preferably from 1 to 6.
33. The peptide or peptidomimetic of item 1(i) and any one of items 2 to 19, to the extent they refer back to item 1(i), wherein
   (a) the alpha-amino group of X1 is bound to a moiety selected from acetyl; benzoyl; Biotin-Ahx; methyl, Ac-Gly-Phe-Trp-Phe-Gly; Ac-Gly-Phe-Trp-Phe-Gly-NH-(CH₂)₂-O-CH₂-CO-; Ac-Gly-Phe-Trp-Phe-Gly-NH-(CH₂)₂-PEG-O-CH₂-CO-, PEG being polyethylene glycol with a polymerization degree from 1 to 6; and R-COO-(CH₂)ₙ-OCO-, R-COO-(CH₂)ₙ-OCO-N(CH₃)-CH₂-CO-, R-COO-(CH₂)ₙ-OCOO-(CH₂)₂-CO- or R-COO-(CH₂)ₙ-OCO-NH-(CH₂)₂-CO-, R being alkyl, cycloalkyl, cycloheteroalkyl, aryl or heteroaryl, alkyl having 1 to 6 carbon atoms, cycles having 4 to 10, preferably 5 or 6 ring atoms and 1 or 2 cycles, R preferably being methyl, ethyl, phenyl or benzyl and n being 1 or 2;
   (b) wherein X4 is Lys and X8 is Asp, or X4 is Glu and X8 is Lys;
   (c) wherein X11 is Lys and X15 is Asp, or X11 is Glu and X15 is Lys;
   (d) wherein X4 and X8 are Ser, and their side chains are bound through the adipoyl moiety -CO-(CH₂)₄-CO-;
   (e) wherein X11 and X15 are Ser, and their side chains are bound through the adipoyl moiety -CO-(CH₂)₄-CO-; or
   (f) the corresponding carboxy group X15 is amidated, esterified or bound to a moiety selected from Gly-Phe-Trp-Phe-Gly-NH₂, -NH-(CH₂)₂-O-CH₂-CO-Gly-Phe-Trp-Phe-Gly-NH₂, and -NH-(CH₂)₂-PEG-O-CH₂-CO-Gly-Phe-Trp-Phe-Gly-NH₂, PEG being polyethylene glycol of different polymerization degrees, preferably from 1 to 6.
34. The peptide or peptidomimetic of any one of the preceding items, wherein said peptide or peptidomimetic is conjugated, preferably via a linker, to a ligand of an E3 ligase.
35. A peptide or peptidomimetic of any one of the preceding items for use in medicine.
36. A medicament comprising or consisting of the peptide or peptidomimetic of any one of the preceding items.
37. The medicament of item 36, wherein
   (a) one or more peptides or peptidomimetics of any one of the preceding items is/are the only pharmaceutically active agents comprised in said medicament;
   (b) said medicament comprises one or more further pharmaceutically active agents, preferably selected from
      a. agents which interfere with or stop cell division such as TAME, proTAME, apcin, GLMN or Emi1;
      b. agents which induce DNA damage, such as Topoisomerase II inhibitors, preferably etoposide or doxorubicin; and
      c. agents which interfere with microtubule assembly such as paclitaxel, vincristine, and PLK1 inhibitors including BI6727; or
   (c) said medicament is to be administered to a patient which is undergoing, has undergone, or will undergo radiation therapy.
38. A peptide or peptidomimetic of any one of items 1 to 35 for use in a method of treating, ameliorating or curing a hyperproliferative disease.
39. The peptide or peptidomimetic for use of item 38, wherein said hyperproliferative disease is a benign or malign tumour, a metastasis or cancer.
40. The peptide or peptidomimetic for use of item 39, wherein said cancer is B-cell non-Hodgkin lymphoma, bladder cancer, breast tumour or breast cancer, cancer of the stomach, cervical cancer, colorectal cancer, esophageal squamous cell carcinoma, glioblastoma, incasive ductal carcinoma, leukemia such as chronic myeloid leukemia, liver cancer such as hepatocellular carcinoma, lung cancer such as lung adenocarcinoma and non-small cell lung cancer, melanoma, ovary cancer, pancreatic cancer such as pancreatic ductal adenocarcinoma, or prostate cancer.
41. An in vitro or ex vivo method of interfering with or stopping cell division, said method comprising bringing one or more peptides or peptidomimetics of any one of items 1 to 34 in contact with a cell.
42. The method of item 41, wherein the cells are cells in culture or comprised in a tissue.
43. A method of purifying APC/C, said method comprising
   (a) bringing into contact a sample comprising APC/C with a peptide or peptidomimetic of any one of items 1 to 34; and
   (b) separating a complex comprising APC/C and said peptide or peptidomimetic from the remainder of the constituents of said sample.
44. The method of item 43, wherein said peptide or peptidomimetic is immobilized on a carrier or bead.
45. The method of item 43 or 44, wherein said sample is a total cell extract.
46. A method of detecting APC/C, said method comprising
   (a) bringing a sample comprising or suspected to comprise APC/C in contact with a peptide or peptidomimetic of any one of items 1 to 34, wherein said peptide or peptidomimetic carries a detectable label; and
   (b) detecting a complex comprising APC/C and said peptide or peptidomimetic.
47. A kit comprising or consisting of one or more peptides or peptidomimetics of any one of items 1 to 34.
48. The kit of item 47, wherein said kit further comprises or further consists of
   (a) an agent which interferes with or stops cell division such as TAME, proTAME, apcin, Emi1 or GLMN;
   (b) an agent which induce DNA damage, such as Topoisomerase II inhibitors, preferably etoposide or doxorubicin;
   (c) an agent which interfere with microtubule assembly such as paclitaxel, vincristine, and PLK1 inhibitors including BI6727;
   and/or
   a manual comprising instructions for performing the method of any one of items 41 to 46.
49. The kit of item 47 or 48, wherein said peptide or peptidomimetic
   (a) carries a detectable label; and/or
   (b) is conjugated, preferably via a linker, to a ligand of an E3 ligase.
50. A method of treating, ameliorating, curing or preventing a hyperproliferative disease, said method comprising or consisting of administering one or more peptides or peptidomimetics of any one of items 1 to 35 to a patient suffering from or being at risk of developing said disease.
51. Use of a peptide or peptidomimetic of any one of items 1 to 34 as a lead for developing a pharmaceutically active agent, wherein preferably said pharmaceutically active agent has anti-proliferative activity.
52. An in vitro or ex vivo method of developing a lead for the treatment of a hyperproliferative disease, said method comprising:
   (a) Chemically modifying a peptide or peptidomimetic of any one of items 1 to 34;
   (b)
      a. Comparing anti-proliferative activity of the result of (a) to the activity of said peptide or peptidomimetic prior to performing step (a);
         and/or
      b. comparing toxicity of the result of (a) to the toxicity of said peptide or peptidomimetic prior to performing step (a);
   (c) Wherein an improved activity determined in (b)a. and/or a decreased toxicity determined in (b)b. is indicative of the result of (a) being an improved lead.
53. The method of item 52, wherein said modifying is as defined in item 19.

The Figures show:

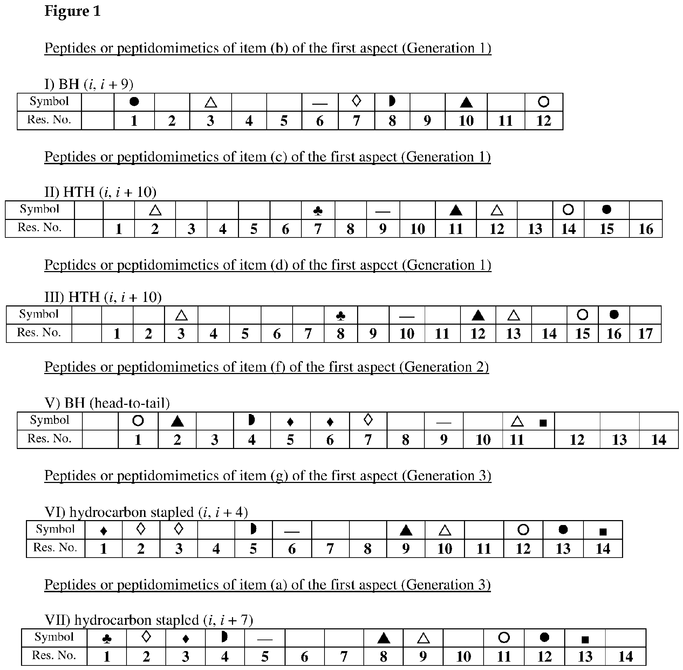

- **Figure 1:**: Chemical equivalences of functionalities in 3D space among the different scaffolds- Chemical equivalences (resembling similar physicochemical features in 3D space) are depicted with the same symbol.

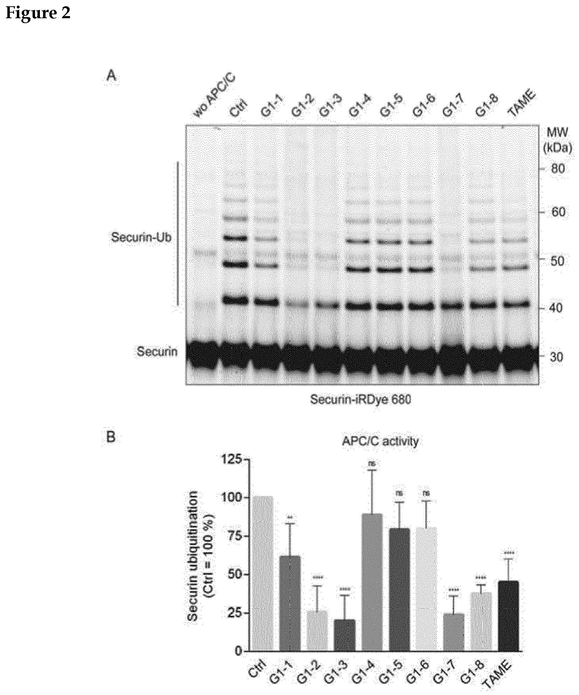

- **Figure 2:**: (A) Comparison of the APC/C activity in the presence of 100 µM of molecules of items (b)-(e) of the first aspect (generation 1) or TAME as a previously described APC/C inhibitor. In vitro APC/C ubiquitination assay driven by the E2 enzyme UBE2C was employed to test the APC/C activity, the reaction time was 45 min. A reaction without the APC/C was used as a negative control and as a substrate, fluorophore-labelled securin was used and monitored by SDS-PAGE followed by fluorescence detection.
(B) The amount of ubiquitinated securin, as shown in (A) was quantified and normalised to the amount of product formed by control (100%) and the mean and SD from 5 independent experiments were plotted. Statistical significance was tested using one-way Anova test, ns (p > 0.05); * (p < 0.05); ** (p ≤ 0.01); *** (p ≤ 0.001); **** (p ≤ 0.0001).

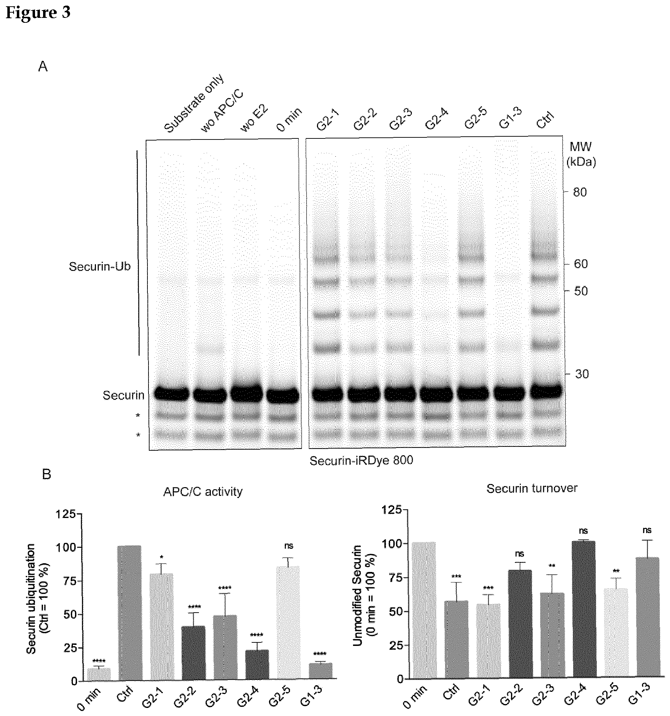

- **Figure 3:**: (A) Comparison of the APC/C activity in the presence of 100 |jM of molecules of item (f) of the first aspect (generation 2) or G1-3 (SEQ. ID. NO. 3) as the most potent molecule from items (b)-(e) of the first aspect (generation 1). In vitro APC/C ubiquitination assay driven by the E2 enzyme UBE2C was employed to test the APC/C activity, the reaction time was 45 min. Reactions without the APC/C or without the E2 were used as a negative control. As a substrate, fluorophore-labelled securin was used and monitored by SDS-PAGE followed by fluorescence detection. Asterisks indicate truncated forms of securin.
(B) The amount of ubiquitinated securin, as shown in (A) was quantified and normalized to the amount of product formed by control (100%) and the mean and SD from 3 independent experiments were plotted (left). Analogous quantification of the amount of unmodified securin was done (right), time point 0 min was set to 100%. Statistical significance was tested using one-way Anova test, ns (p > 0.05); * (p ≤ 0.05); ** (p ≤ 0.01); *** (p ≤ 0.001); **** (p ≤ 0.0001).

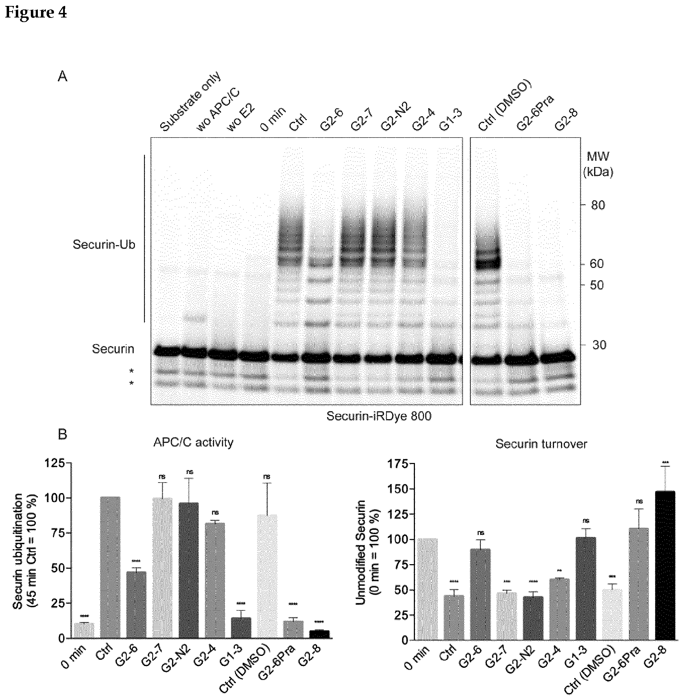

- **Figure 4:**: (A) Comparison of the APC/C activity in the presence of 100 µM of molecules of of item (f) of the first aspect (generation 2), including as references G2-4 (SEQ. ID. NO. 17) and G1-3 (SEQ. ID. NO. 3) from item (b) (generation 1) of the first aspect. In vitro APC/C ubiquitination assay driven by the E2 enzyme UBE2C was employed to test the APC/C activity, the reaction time was 45 min. Reactions without the APC/C or without the E2 were used as a negative control, further, a new negative control G2-N2 derived from the inhibitor molecules, was used. Note, G2-6Pra and G2-8 molecules were dissolved in 40% DMSO. As a substrate, fluorophore-labelled securin was used and monitored by SDS-PAGE followed by fluorescence detection. Asterisks indicate truncated forms of securin.
(B) The amount of ubiquitinated securin, as shown in (A) was quantified and normalized to the amount of product formed by control (100%) and the mean and SD from 3 independent experiments were plotted (left). Analogous quantification of the amount of unmodified securin was done (right), time point 0 min was set to 100%. Statistical significance was tested using one-way Anova test, ns (p > 0.05); * (p ≤ 0.05); ** (p ≤ 0.01); *** (p ≤ 0.001); **** (p ≤ 0.0001).

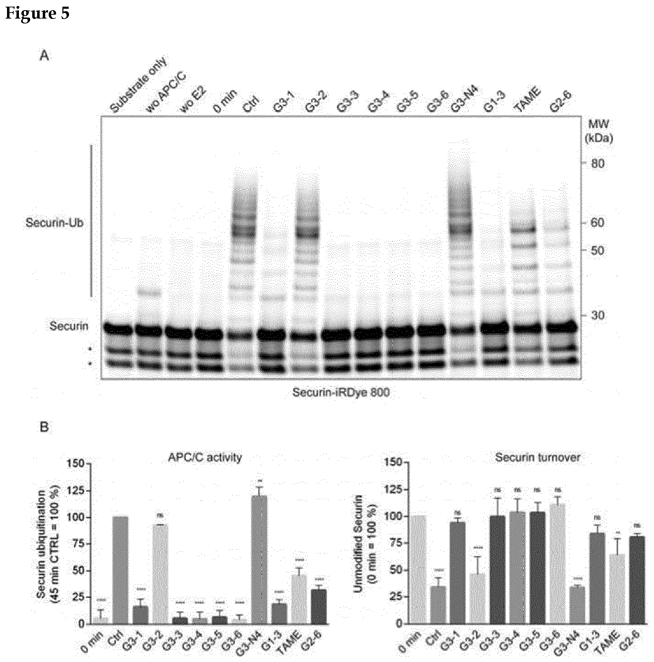

- **Figure 5:**: (A) Comparison of the APC/C activity in the presence of 100 µM of molecules of items (a) and (g) of the first aspect (generation 3), including G2-6 (SEQ. ID. NO. 19) as the most potent molecule from item (f) (generation 2) of the first aspect, G1-3 (SEQ. ID. NO. 3) as the most potent molecule from item (b) of the first aspect and TAME as a previously described APC/C inhibitor. In vitro APC/C ubiquitination assay driven by the E2 enzyme UBE2C was employed to test the APC/C activity, the reaction time was 45 min. Reactions without the APC/C or without the E2 were used as a negative control, further, a negative control G3-N4 derived from the generation 3 molecules was used. As a substrate, fluorophore-labelled securin was used and monitored by SDS-PAGE followed by fluorescence detection. Asterisks indicate truncated forms of securin.
(B) The amount of ubiquitinated securin, as shown in (A) was quantified and normalized to the amount of product formed by control (100%) and the mean and SD from 3 independent experiments were plotted (left). Analogous quantification of the amount of unmodified securin was done (right), time point 0 min was set to 100%. Statistical significance was tested using one-way Anova test, ns (p > 0.05); * (p ≤ 0.05); ** (p ≤ 0.01); *** (p ≤ 0.001); **** (p ≤ 0.0001).

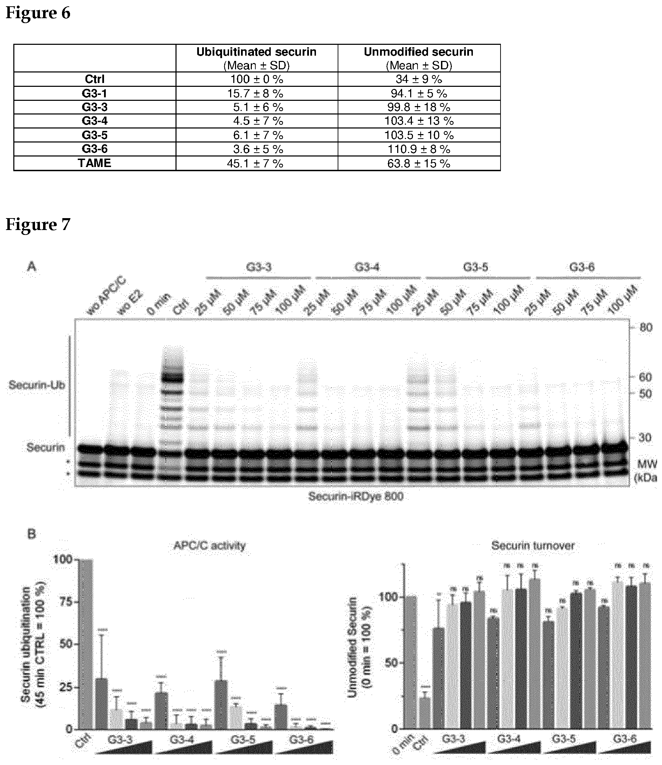

- **Figure 6:**: Comparison of the APC/C activity in vitro in the presence of 100 µM of G3-1 (SEQ. ID. NO. 27), G3-3 (SEQ. ID. NO. 30), G3-4 (SEQ. ID. NO. 31), G3-5 (SEQ. ID. NO. 32), G3-6 (SEQ. ID. NO. 33) and TAME as a previously described APC/C inhibitor. The data represents results of 3 independent in vitro APC/C ubiquitination assay shown in Figure 5.
- **Figure 7:**: (A) Comparison of the APC/C activity in the presence of 25, 50, 75 and 100 µM of G3-3 (SEQ. ID. NO. 30), G3-4 (SEQ. ID. NO. 31), G3-5 (SEQ. ID. NO. 32) or G3-6 (SEQ. ID. NO. 33). In vitro APC/C ubiquitination assay driven by the E2 enzyme UBE2C was employed to test the APC/C activity, the reaction time was 45 min. Reactions without the APC/C or without the E2 were used as a negative control, as a substrate, fluorophore-labelled securin was used and monitored by SDS-PAGE followed by fluorescence detection. Asterisks indicate truncated forms of securin.
(B) The amount of ubiquitinated securin, as shown in (A) was quantified and normalized to the amount of product formed by control (100%) and the mean and SD from 3 independent experiments were plotted (left). Analogous quantification of the amounts of unmodified securin was done (right), time point 0 min was set to 100%. Statistical significance was tested using one-way Anova test, ns (p > 0.05); * (p ≤ 0.05); ** (p ≤ 0.01); *** (p ≤ 0.001); **** (p ≤ 0.0001).

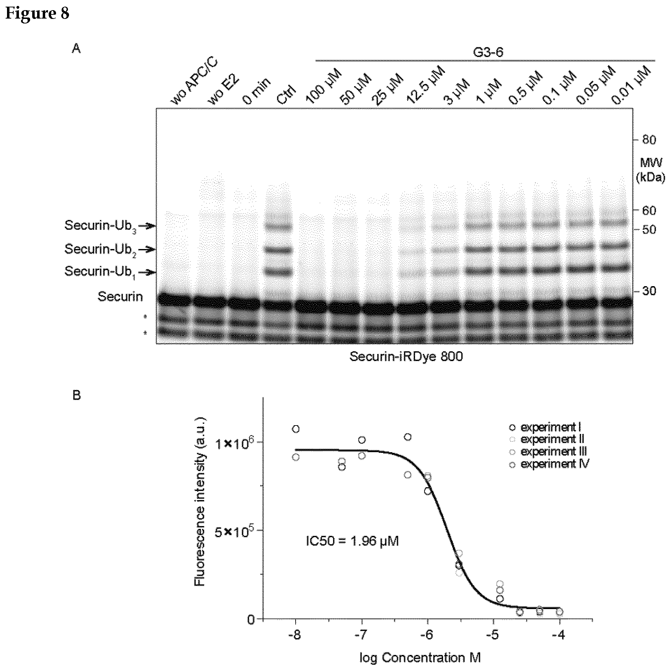

- **Figure 8:**: (A) IC₅₀ determination of the lead molecule G3-6 (SEQ. ID. NO. 33) based on monitoring APC/C activity in the presence of 0.01-100 µM G3-6 (SEQ. ID. NO. 33). In vitro APC/C ubiquitination assay driven by the E2 enzyme UBE2C was employed to test the APC/C activity, the reaction time was 10 min. Reactions without the APC/C or without the E2 were used as a negative control, as a substrate, fluorophore-labelled securin was used and monitored by SDS-PAGE followed by fluorescence detection. Asterisks indicate truncated forms of securin. To prevent ubiquitin chain elongation, methylated ubiquitin was used.
(B) The amount of ubiquitinated securin (Securin-Ub1, Securin-Ub2 and Securin-Ub3), as shown in (A), was quantified and plotted, different colours of circles represent data from 4 independent experiments. Non-linear curve was fitted and IC₅₀ of the G3-6 (SEQ. ID. NO. 33) was determined to 1.96 µM.

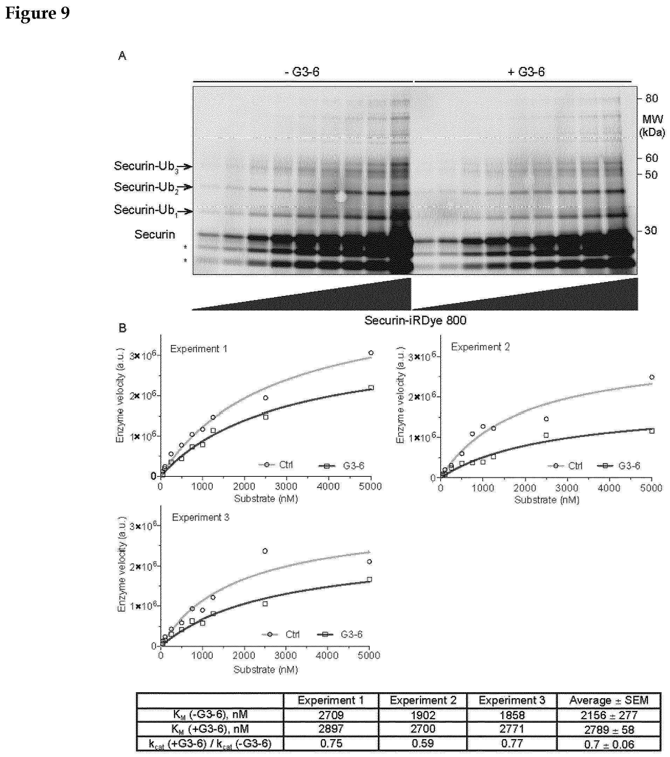

- **Figure 9:**: (A) K_{M} and k_{cat} determination of the lead molecule G3-6 (SEQ. ID. NO. 33) based on monitoring APC/C activity in the presence or absence of 3 µM G3-6 APC11 inhibitor and different substrate concentrations. In vitro APC/C ubiquitination assay driven by the E2 enzyme UBE2C was employed to monitor the APC/C activity, the reaction time was 2 min. As a substrate 50, 100, 250, 500, 750, 1000, 1250, 2500 and 5000 nM fluorophore-labelled securin was used and monitored by SDS-PAGE followed by fluorescence detection. To prevent ubiquitin chain elongation, methylated ubiquitin was used. Asterisks indicate truncated forms of securin.
(B) The amount of ubiquitinated securin (Securin-Ub1, Securin-Ub2 and Securin-Ub3), as shown in (A), was quantified and plotted and non-linear curves were fitted. Quantification of 3 independent experiments and a summary of K_{M} and k_{cat} are shown. Note, difference between K_{M} (-G3-6 (SEQ. ID. NO. 33)) and K_{M} (+G3-6 (SEQ. ID. NO. 33)) is not significant (p=0.0886) using unpaired t test.
Based on the results of this experiment inhibitory constant (Kᵢ) was calculated using a model for non-competitive inhibition in Prism 6.0. Kᵢ of the lead molecule G3-6 (SEQ. ID. NO. 33) is 5 µM.

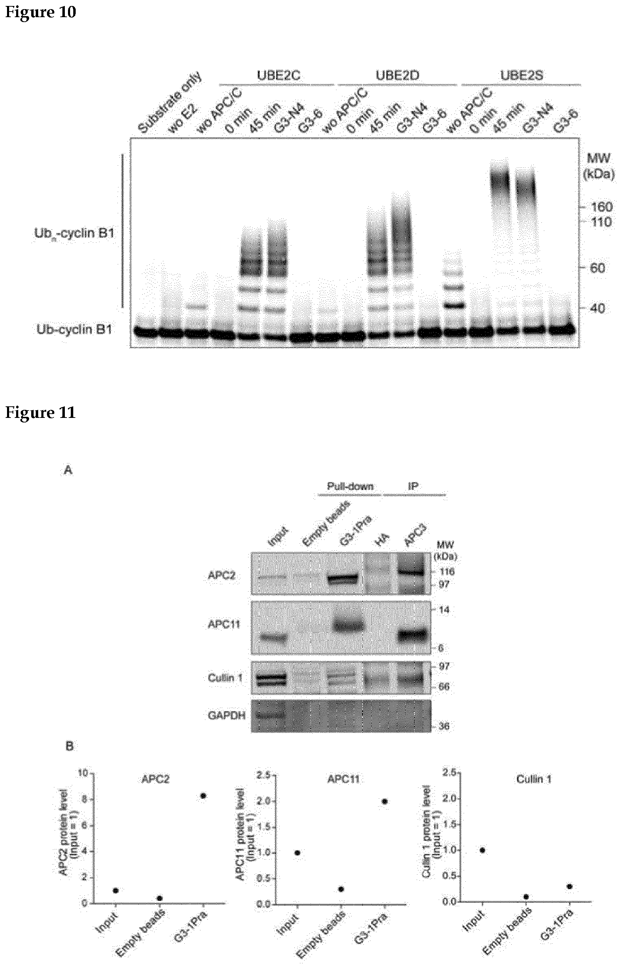

- **Figure 10:**: Comparison of the capability of the lead molecule G3-6 (SEQ. ID. NO. 33) to inhibit in vitro APC/C activity driven by different E2 enzymes, specifically UBE2C, UBE2D and UBE2S. In vitro APC/C ubiquitination assay was employed to test the APC/C activity, G3-6 and G3-N4 molecules were used in 100 µM concentration and the reaction time was 40 min. Reactions without the APC/C or without the E2 were used as a negative control, further, a negative control G3-N4 derived from the generation 3 molecules was used. As a substrate, fluorophore-labelled ubiquitin-cyclin B1 fusion (Ub-cyclin B1) was used and monitored by SDS-PAGE followed by fluorescence detection. The use of the ubiquitin-cyclin B1 fusion allows UBE2S activity to be monitored without the chain-initiating E2 enzymes UBE2S or UBE2D. Representative image from 3 independent experiments is shown.
- **Figure 11:**: G3-1Pra (SEQ. ID. NO. 28) has the Propargyl (Pra) chemical group that can be employed for click chemistry. G3-1Pra was clicked to azide agarose beads and used for the pull-down in HeLa K cell extract. As a control, empty azide agarose beads, that were treated in the same manner like G3-1Pra beads were used. Another control was magnetic beads coupled with the APC3 antibody or the HA antibody. (A) Western blot analysis was performed and indicated proteins were detected using near-infrared fluorescence imaging. GAPDH serves as a control for unspecific binding. (B) Indicated proteins were quantified. G3-1Pra binds to the APC/C (APC2 and APC11), however, it almost does not bind to Cullin 1, the subunit of E3 ubiquitin ligases related to the APC/C, suggesting G3-1Pra specificity towards the APC/C.

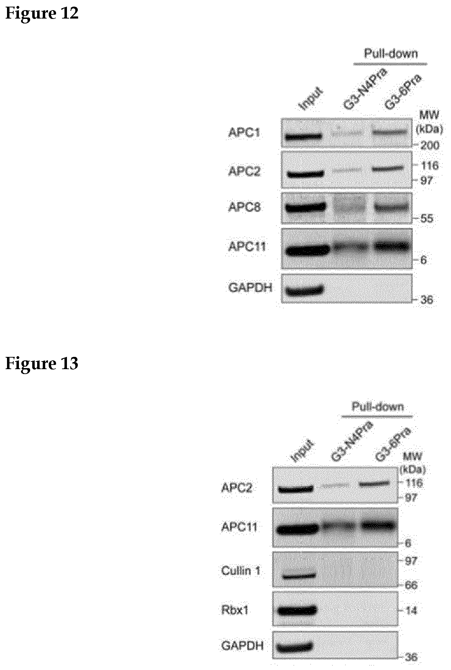

- **Figure 12:**: G3-6Pra (SEQ. ID. NO. 37) has the Propargyl (Pra) chemical group that can be employed for Click chemistry. G3-6Pra peptides were clicked to azide magnetic beads and used for the pull-down in HeLa K cell extract. As a control, G3-N4Pra negative control molecule was used. Western blot analysis was performed and indicated APC/C subunits were detected using near-infrared fluorescence imaging. GAPDH serves as a control for unspecific binding.
- **Figure 13:**: G3-6Pra (SEQ. ID. NO. 37) was clicked to azide magnetic beads and used for the pull-down in HeLa K cell extract. As a control, G3-N4Pra negative control molecule was used. Western blot analysis was performed and indicated proteins were detected using near-infrared fluorescence imaging. GAPDH serves as a control for unspecific binding. G3-6Pra molecule binds to the APC/C, however, does not bind to Cullin 1 and Rbx1, subunits of E3 ubiquitin ligases related to the APC/C suggesting G3-6Pra specificity towards the APC/C.

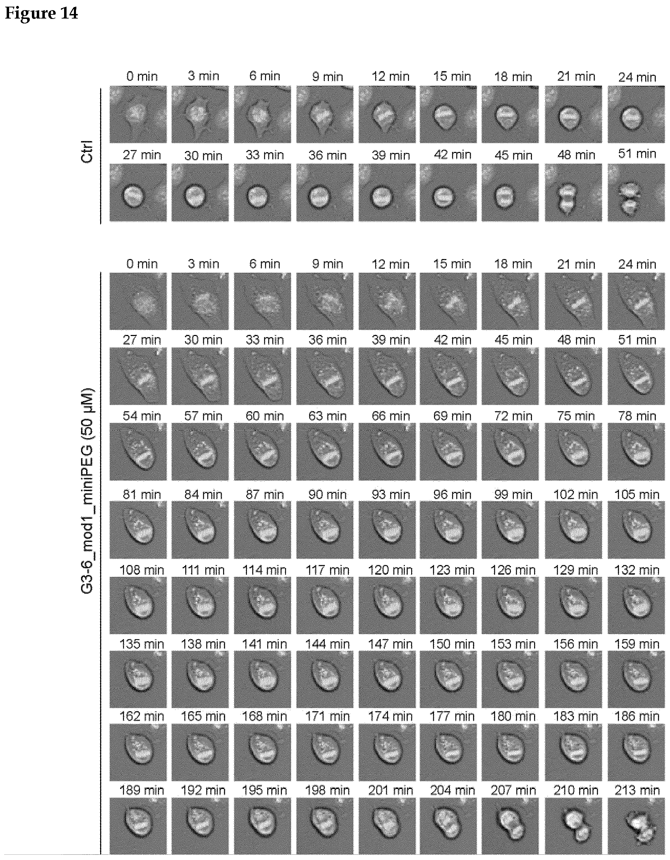

- **Figure 14:**: G3-6_mod1_miniPEG (SEQ. ID. NO. 36) prolongs mitosis by arresting cells in metaphase. HeLa K cells were treated with Ctrl (DMSO) or 50 µM G3-6_mod1_miniPEG (in DMSO) imaged by live bright-field and fluorescence microscopy for 48 hours. DNA stained by SiR-Hoechst is shown in white. Images show representative control and G3-6_mod1_miniPEG-treated cells. Images are aligned to the entry into mitosis determined by the breakdown of the nuclear envelope (t= 0 min).

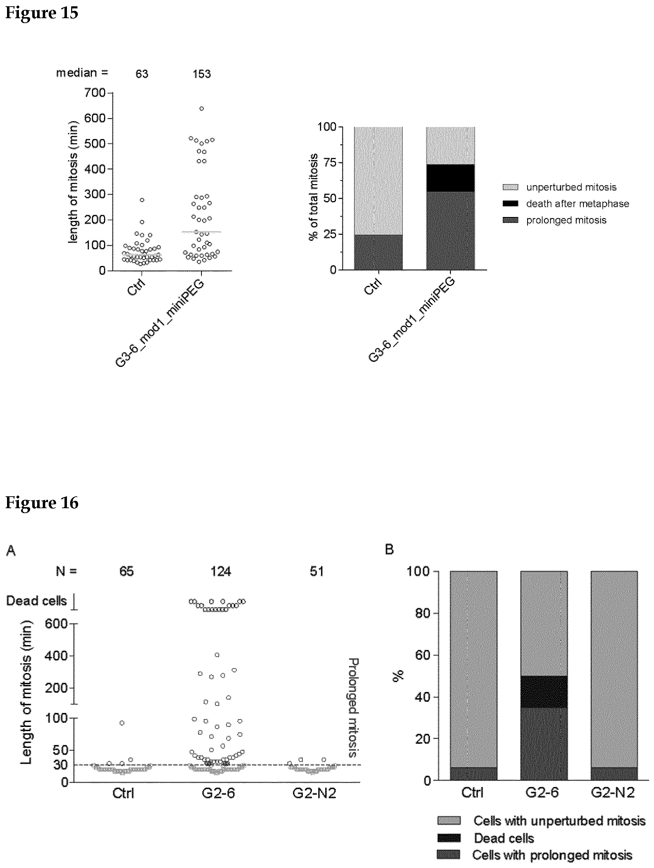

- **Figure 15:**: G3-6_mod1_miniPEG (SEQ. ID. NO. 36) prolongs mitosis and cause cell death after metaphase. Left panel: Scatterplots showing the length of mitosis in cells treated and analysed as in Figure 14. Gray lines indicate the median duration of mitosis. Note, only cells after 10 hours of treatment were analysed. Right panel: Cell fate analysis of mitotic cells analysed in the left panel. Cells that required longer than the 75% percentile duration of mitosis are considered as "prolonged".
- **Figure 16:**: G2-6 (SEQ. ID. NO. 19) prolongs mitosis and causes cell death. hTERT RPE-1 cells were treated with 100 µM G2-6, G2-N2 or untreated as Ctrl and imaged by bright-field microscopy for 12-24 hours. Note, G2-N2 is a negative control molecule. (A) Scatterplots showing the length of mitosis. Cells that required longer than the 95% percentile duration of mitosis in Ctrl (30 min) are considered as "prolonged" and are above a dashed line. N indicates total number of analysed cells obtained from 3 independent experiments (in case of G2-N2, 2 independent experiments). (B) Cell fate analysis of mitotic cells analysed in (A).

The Examples illustrate the invention.

### EXAMPLE 1

### Materials and Methods

Peptides or peptidomimetics were purchased from GenScript Biotech (Netherlands) B. V. with a > 95% purity determined by analytical rpHPLC.

### General synthetic procedure of preferred sequences of items of the first aspect.

Peptides or peptidomimetics are preferably prepared by standard Fmoc vs. standard Boc solid-phase synthesis by using Rink Amide MBHA resin for items (a)-(e) and (g)-(i) (Scheme 1), and 2-chlorotritylchloride resin for item (f). Coupling steps involve 4 equiv. of amino acid, 4 equiv. of 2-(1*H*-benzotriazol-1-yl)-1,1,3,3-tetramethyluroniumhexafluorophosphate (HBTU) as coupling reagent and 5 equiv. of diisopropylethylamine (DIPEA) in DMF. Coupling progress is monitored via Kaiser (ninhydrin) test and coupling of amino acids is repeated when results suggest yields below than 99%. Fmoc deprotections are carried out by 2 × 1 min treatments with excess (1:1) piperidine: DMF and the resin is sequentially washed with DMF, DCM:MeOH (1:1, v/v), and DCM upon completation. The N-terminus is acetylated by standar methods with Ac₂O for 10 minutes. The resulting peptides or peptidomimetics are cleaved from resin by treatment with TFA/H₂O/EDT/TIPS (94:2.5:2.5:1) for 2 h followed by precipitation in cold Et₂O. Peptides or peptidomimetics are redisolved in water/acetonitrile and lyophilized. After freeze-drying, the peptides are purified by rpHPLC. Purity of compounds is assessed via analytical rpHPLC (solvent A is 0.065% TFA in 100% water (v/v) and solvent B is 0.05% TFA in 100% acetonitrile (v/v), Table 4).

### Additional synthetic steps of preferred items (a) and (g) of the first aspect.

Crosslinking reaction of unnatural olefininc amino acids is carried out by ring-closing metathesis (RCM) (Scheme 1). The resin is swollen in dry DCM for 30 min and a solution of 4 mg/mL of Grubbs 1^{st} generation catalyst in dry DCM is added to the resin under inert atmosphere three times for 2 h. The reaction is keeped under inert atmosphere.

### Additional synthetic steps of preferred items (b) - (d) of the first aspect.

Peptides or peptidomimetics containing Cys are prepared through Fmoc synthesis by using DIC/Oxyma or DIC/HOBt as carboxyl activators. Disulfide bridge formation can be attempted through the two following strategies. First, the Fmoc-Cys(Mmt)-OH is used. The residue is incorporated in the respective positions following the general synthetic procedure described above. The resin is washed with DCM and treated with 2% TFA in DCM (5 x 2 min). Cyclization is carried out on-resin using 25 mM solution of N-chlorosuccinimide (NCS) in DMF. In a second strategy, the linear peptides or peptidomimetics are converted to cyclic disulfides by dropwise addition of a saturated solution of I₂ in acetic acid and repurified by rpHPLC.

### Additional synthetic steps of preferred item (f) of the first aspect.

Once the linear peptide is fully synthesized, the peptide is cleaved from resin with cold 0.8% TFA in DCM 5 times for one minute. The eluate is treated with DIPEA (1 mL) immediately after TFA treatment. The resin is further washed with DCM and MeOH and the collected solvents in the eluate containing the linear peptide are evaporated in high vacumm. Cyclization is performed by treatment of the resulting crude with 3 equiv. 2-(7-aza-1H-benzotriazole-1-yl)-1,1,3,3,-tetramethyluronium (HATU), 3 equiv. 1-hydroxy-7-azabenzotriazole (HOAt) and 6 equiv. of DIPEA in DMF and stirred for 18 h. Then DMF is removed under high vacuum and the crude is further disosolved in DCM and extracted with 10% acetonitrile in water. After solvent evaporation, the crude peptide is cooled and treated with pre-cooled TFA:H₂O (9:1) for 5 h at 5 ºC followed by precipitation in cold Et₂O.

### Additional synthetic steps of preferred item (h) and (i) of the first aspect.

Fmoc-Lys(Mtt)-OH and Fmoc-Asp(OPip)-OH are used for lactam cyclization. These residues are incorporated in the respective positions following the general synthetic procedure described above. The resin is washed with DCM and treated with 2% TFA in DCM (5 x 2 min). Cyclization is carried out on-resin using 2.5 equiv. BOP, 2.5 equiv. DIPEA in DMF 0.5 M.

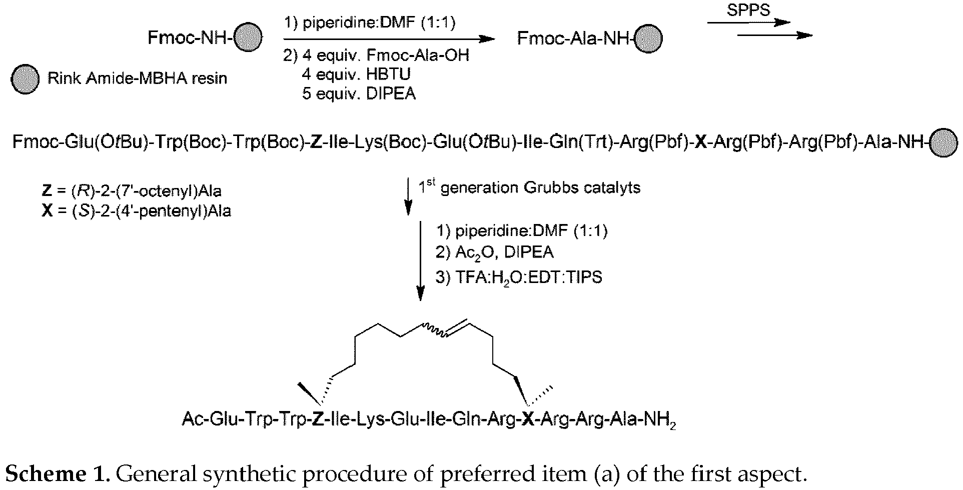

**Table 4. rpHPLC retention times (Rt) and mass spectrometry data of preferred items (a) and (g) of the first aspect.**

| SEQ. ID. NO. | Designation in the Figures | Rt (min) | [M + XH]^{x+} | | | Calculated mass |
|---|---|---|---|---|---|---|
| | | | x=2 | x = 3 | x=4 | |
| 27 | G3-1 | 11.7^{a} | 1017.0 | 678.3 | 509.0 | 2032.20 |
| 29 | G3-2 | 15.4^{b} | 951.9 | 635.0 | 476.5 | 1902.27 |
| 30 | G3-3 | 17.7^{b} | 995.5 | 664.0 | - | 1989.39 |
| 31 | G3-4 | 13.8^{a} | 1003.2 | 669.4 | 502.2 | 2004.37 |
| 32 | G3-5 | 19.2^{b} | 1003.1 | 669.0 | 502.0 | 2004.37 |
| 33 | G3-6 | 21.7^{b} | 1003.0 | 669.0 | 502.0 | 2004.37 |

| | | | | | | |
|---|---|---|---|---|---|---|
| ^{a}Linear gradient from 15% to 75% solvent B over 25 min. ^{b}Linear gradient from 5% to 65% solvent B over 25 min. | | | | | | |

### Antibodies

### Primary antibodies

| **Antibody target** | **Product no.** | **Company** | **Species** | **Dilution** |
|---|---|---|---|---|
| APC11 | N/A | Moravian Biotechnology | mouse monoclonal | WB 1:1000 |
| APC11 | 14090S | Cell Signalling | rabbit monoclonal | WB 1:1000 |
| APC2 | 12301S | Cell Signalling | rabbit monoclonal | WB 1:1000 |
| APC1 | A301-653a | Bethyl | rabbit polyclonal | WB 1:1000 |
| APC8 | A301-181A | Bethyl | rabbit polyclonal | WB 1:1000 |
| Cullin 1 | 32-2400 | Thermo Fisher Scientific | mouse monoclonal | WB 1:1000 |
| Rbx1 | 11922S | Cell Signalling | rabbit monoclonal | WB 1:1000 |
| GAPDH | 2118 | Cell Signalling | rabbit polyclonal | WB 1:5000 |

### Secondary antibodies

| **Antibody target** | **Product no.** | **Company** | **Species** | **Dilution** |
|---|---|---|---|---|
| anti-rabbit IgG, IRDye 800CW conjugated antibody | 926-32213 | LI-COR Biosciences | donkey | 1:20000 |
| anti-mouse IgG, IRDye 800CW conjugated antibody | 926-32212 | LI-COR Biosciences | donkey | 1:20000 |
| anti-rabbit IgG, IRDye 680RD conjugated antibody | 926-68073 | LI-COR Biosciences | donkey | 1:20000 |
| anti-mouse IgG, IRDye 680RD conjugated antibody | 926-68072 | LI-COR Biosciences | donkey | 1:20000 |

### Cell lines

Cells were cultured according to the standard mammalian tissue culture protocol and sterile technique at 37 °C in 5% CO₂ and tested in regular intervals for mycoplasma.

HeLa K cells were maintained in DMEM (Gibco) supplemented with 10% (v/v) fetal bovine serum (FBS) (Gibco), 1% (v/v) penicillin-streptomycin (Sigma-Aldrich), 1% (v/v) Glutamax (Gibco), and 0.5 µg/ml amphotericin B (Sigma-Aldrich).

hTERT RPE-1 cells were maintained in DMEM/F12 (Sigma-Aldrich,) supplemented with 10% (v/v) FBS, 1% (v/v) penicillin-streptomycin, 1% (v/v) Glutamax, 0.26% (v/v) sodium bicarbonate (Gibco) and 0.5 µg/mL amphotericin B.

### Protein Expression and Purification

### APC/C

The bacmids encoding the human APC/C were a kind gift from David Barford (MRC, Cambridge, UK). SF9 cells (Expression Systems) were co-infected with two recombinant baculoviruses (ratio 2:5; the first corresponding to the virus containing Strep-tagged APC4) encoding the APC/C at a multiplicity of infection (MOI) of ∼ 1 and a cell density of 1 million cells/ml. SF9 cells were incubated at 27 °C for 72 h. All steps of APC/C purification were performed at 4 °C. Cell pellets were re-suspended in APC/C lysis buffer (50 mM Tris-HCl pH 8.3, 250 mM NaCl, 5% Glycerol, 1 mM EDTA, 2 mM DTT, 0.1 mM PMSF, 2 mM Benzamidine, 5 units/ml benzonase, cOmplete protease inhibitor cocktail (Roche)) and disrupted by nitrogen cavitation in a 4639 Cell Disruption Vessel (Parr Instrument Company). Cell extract was cleared by centrifugation at 48 000 g at 4 °C for 1 h. Strep-tagged APC/C was captured on Strep-Tactin Superflow resin (IBA Life Sciences), washed with APC/C wash buffer (50 mM Tris-HCl pH 8, 250 mM NaCl, 5% Glycerol, 1 mM EDTA, 2 mM DTT, 2 mM Benzamidine) and eluted with Buffer E (IBA Life Sciences) supplied with additional NaCl (final concentration 250 mM), 2 mM DTT and 2 mM Benzamidine. Peak fractions were concentrated using Vivaspin 6 centrifugal concentrator (VivaProducts) and purified by size-exclusion chromatography (Superose 6 Increase 10/300 GL column; GE healthcare) in APC/C size-exclusion buffer (20 mM Hepes-NaOH pH 8, 200 mM NaCl, 2 mM DTT, 5% Glycerol). Finally, APC/C was flash-frozen in liquid nitrogen and stored at -80 °C.

### Cdc20

The bacmid encoding SBP-tagged Cdc20 was a kind gift from Jonathon Pines (ICR, London, UK). SF9 cells were infected with the recombinant baculovirus encoding Cdc20 at a multiplicity of infection (MOI) of ∼1 and a cell density of 1 million cells/ml. SF9 cells were incubated at 27 °C for 72 h. All steps of Cdc20 purification were performed at 4 °C. Cell pellets were re-suspended in Cdc20 lysis buffer (250 mM NaCl, 50 mM Tris-HCl pH 8, 1 mM DTT, 5% Glycerol, cOmplete protease inhibitor cocktail, 1 mM PMSF) and disrupted by nitrogen cavitation in a 4639 Cell Disruption Vessel. Cell extract was cleared by centrifugation at 48 000 g at 4 °C for 1 h. SBP-tagged Cdc20 was captured to Strep-Tactin Superflow resin, washed with IX Buffer W (IBA Life Sciences) and eluted with Buffer E supplied with additional NaCl (final concentration 250 mM). Finally, glycerol was added to a final concentration of 10% and Cdc20 was flash-frozen in liquid nitrogen and stored at -80 °C.

### Protein analysis

### SDS-PAGE

Proteins were separated by SDS-PAGE electrophoresis using precast Bolt 4-12% Bis-Tris Plus protein gels (Thermo Fisher Scientific) and Criterion XT Bis-Tris Midi Protein Gels (Bio-Rad). In different experiments, various conditions were used: in vitro APC/C ubiquitination assays - 165 V, 40 min, SDS-MES running buffer (50 mM MES, 50 mM Tris base, 0.1% SDS, 1 mM EDTA, pH 7.3; Thermo Fisher Scientific); in vitro APC/C ubiquitination assays determining K_{M} - 165V, 1 h, SDS-MOPS running buffer (50mM MOPS, 50mM Tris base, 0.1% SDS, 1 mM EDTA, pH 7.7; Thermo Fisher Scientific); pull-down binding assay -165 V, 35 min, SDS-MES running buffer.

### Western blot analysis

For the protein transfer, the 0.45 µm Immobilon-FL PVDF membrane (Merck Millipore) was used. The membrane was activated in ethanol and then transferred to the Blotting buffer (50 mM SDS-MOPS, 50 mM Tris base, 0.1% SDS, 1 mM EDTA, 20% EtOH). The proteins were transferred using a wet transfer for 1.5 h at 400 mA. Subsequently, the membrane was incubated with the blocking solution, 10% milk in PBS-T (0.02% Tween-20 in PBS), for 1 h at room temperature. To detect proteins of the interest, the membrane was incubated with primary antibodies overnight at 4°C. Followed by washing the membrane 3 times 10 min with PBS-T and incubation with IRDye fluorescently labelled secondary antibodies for 1 h. Prior detection, the membrane was twice washed with PBS-T for 10 min and once with PBS for 10 min. The detection was done using the quantitative near-infrared scanning system Odyssey (LI-COR Biosciences).

### In vitro APC/C ubiquitination assay

APC/C-dependent ubiquitination reactions were performed at 30 °C in 30 mM HEPES pH 7.4, 175 mM NaCl, 8 mM MgCl₂, 0.05% Tween-20, 1 mM DTT and 5% glycerol and contained 20 nM recombinant APC/C (note, for testing the first generation of inhibitors, APC/C and Cdc20 immunoprecipitated from mitotic HeLa K cells was used), 340 nM Cdc20, 46 nM GST-UBA1, 340 nM UBE2C (alternatively 400 nM UBE2D, 280 nM UBE2S), 21 µM His₆-ubiquitin or if it is indicated 21 µM methylated-ubiquitin (BostonBiochem), 2.6 mM ATP, 10 mM phosphocreatine and 11 µM creatine kinase. As substrates, 35 nM fluorophore-labeled ubiquitin-cyclin B and 50 nM fluorophore-labeled securin were standardly used (fluorophore IRDye 800CW (LI-COR) was used for labelling). In case of K_{M} and k_{cat} determination, 50, 100, 250, 500, 750, 1000, 1250, 2500 and 5000 nM fluorophore-labelled securin was used. The reaction was done in the volume of 15 µl, it was quenched after the indicated time with LDS sample buffer (Thermo Fisher Scientific) supplemented with 100 mM DTT and subjected to SDS-PAGE. Detection of fluorescently labelled substrates was done by quantitative near-infrared scanning system Odyssey.

### Pull-down binding assay

HeLa K cell pellet was re-suspended in the extraction buffer (30 mM HEPES pH 7.5, 175 mM NaCl, 2.5 mM MgCl₂, 0.25% NP40, 10% glycerol, 1 mM DTT) supplemented with 10 µM MG132 (VWR), 1 mM PMSF (Sigma-Aldrich), complete protease inhibitor cocktail (Roche) and PhosSTOP phosphatase inhibitors (Roche) and incubated for 20 min on ice, followed by centrifugation of cell debris for 15 min at 4 °C 16 100 g. In mean time, inhibitor molecules containing Propargyl (Pra) chemical group were covalently attached to the azide agarose or azide magnetic resin (Jena Bioscience) by Cu(I)-catalysed azide-alkyne cycloaddition reaction. Specifically, the inhibitor molecules were mixed with azide agarose resin in ratio 0.125 µmol inhibitor / 25 µl agarose resin and azide magnetic resin in ratio 0.018 µmol inhibitor / 20 µl magnetic resin. The reaction was catalysed by 1 mM CuSO₄, 0.1 mM TBTA (Sigma-Aldrich) and 1 mM Sodium ascorbate and was performed on the rotating wheel for 30 min at room temperature in the volume of 1 ml. Subsequently, the resin was washed 5 times with the extraction buffer. The inhibitor-agarose resin was added to 1 mg of HeLa K cell extract and the inhibitor-magnetic resin was added to 0.5 mg of HeLa K cell extract followed by incubation at 4 °C for 2 h. Followed by washing the resin 5 times with the extraction buffer. Pull-down proteins were eluted by boiling for 15 min with 1x LDS sample buffer supplemented with 100 mM DTT (agarose resin) or by incubation with 1x LDS sample buffer for 10 min at room temperature followed by taking supernatant that was supplemented with 100 mM DTT and boiling it for 10 min. Samples were subjected to SDS PAGE and Western blot analysis.

### Statistical analysis

Prism 6.0 (Graphpad) was used, unless specified otherwise.

### EXAMPLE 2

Question: Do molecules from items (b)-(e) of the first aspect (generation 1) inhibit APC/C activity in vitro?
Approach: in vitro APC/C ubiquitination assay
Conclusion: Molecules of items (b)-(e) of the first aspect effectively inhibit APC/C in vitro. The molecules G1-3 (SEQ. ID. NO. 3) and G1-7 (SEQ. ID. NO. 6) are the most potent and will be used for further development. [Fig. 2]

Question: Do molecules of item (f) of the first aspect (generation 2) developed based on G1-1 (SEQ. ID. NO. 1), G1-2 (SEQ. ID. NO. 2), G1-3 (SEQ. ID. NO. 3) and G1-4 (SEQ. ID. NO. 48) inhibit APC/C activity in vitro?
Approach: in vitro APC/C ubiquitination assay
Conclusion: Molecules of item (f) of the first aspect inhibit APC/C in vitro and the molecule G2-4 (SEQ. ID. NO. 17) is the most potent. [Fig. 3]

Question: Do optimized molecules of item (f) of the first aspect (generation 2) developed based on G1-1 (SEQ. ID. NO. 1), G1-2 (SEQ. ID. NO. 2), G1-3 (SEQ. ID. NO. 3) and G1-4 (SEQ. ID. NO. 48) inhibit APC/C activity in vitro?
Approach: in vitro APC/C ubiquitination assay
Conclusion: The optimized molecules G2-6 (SEQ. ID. NO. 19) and G2-8 (SEQ. ID. NO. 22) inhibit APC/C in vitro the most effectively compared to other molecules of item (f) of the first aspect. [Fig. 4]

Question: Do molecules of items (a) and (g) of the first aspect (generation 3) developed based on molecules of items (b)-(f) of the first aspect inhibit APC/C activity in vitro?
Approach: in vitro APC/C ubiquitination assay
Conclusion: Molecules of items (a) and (g) of the first aspect (generation 3) inhibit APC/C in vitro the most potently compared to previous generations. [Fig. 5]

Question: Do molecules of items (a) and (g) of the first aspect (generation 3) inhibit APC/C activity more efficiently than TAME?
Approach: in vitro APC/C ubiquitination assay
Conclusion: Molecules of items (g) G3-1 (SEQ. ID. NO. 27) and G3-3 (SEQ. ID. NO. 30) and of item (a) G3-4 (SEQ. ID. NO. 31) and G3-6 (SEQ. ID. NO. 33) of the first aspect inhibit APC/C in vitro more efficiently than TAME. [Fig. 6]

Question: Which molecules from items (a) and (g) of the first aspect (generation 3) inhibit APC/C in vitro the most potently?
Approach: in vitro APC/C ubiquitination assay, titration of molecules of items (a) and (g) of the first aspect
Conclusion: G3-6 (SEQ. ID. NO. 33) inhibits APC/C in vitro the most potently and is the lead compound. [Fig. 7]

Question: What is the IC₅₀ of the lead molecule G3-6 (SEQ. ID. NO. 33)?
Approach: in vitro APC/C ubiquitination assay, titration of the G3-6 molecule (SEQ. ID. NO. 33)
Conclusion: IC₅₀ of the lead molecule G3-6 (SEQ. ID. NO. 33) is in low micromolar range. [Fig. 8]

Question: What is the mechanism of action of the G3-6 molecule (SEQ. ID. NO. 33) (type of the inhibition)?
Approach: in vitro APC/C ubiquitination assay, titration of substrate (securin)
Conclusion: G3-6 molecule (SEQ. ID. NO. 33) acts as a non-competitive inhibitor of APC/C in vitro. [Fig. 9]

Question: Is APC/C inhibition by the G3-6 molecule (SEQ. ID. NO. 33) dependent on a specific E2 enzyme?
Approach: in vitro APC/C ubiquitination assay, different E2 enzymes - UBE2C, UBE2D, UBE2S
Conclusion: The lead molecule G3-6 (SEQ. ID. NO. 33) inhibits APC/C in vitro independently on employed E2 enzymes. [Fig. 10]

Question: Is the G3-1 molecule (SEQ. ID. NO. 27) binding specifically to the APC/C?
Approach: Pulldown binding assay from HeLa K cell extract, azide agarose beads
Conclusion: The G3-1 molecule (SEQ. ID. NO. 27) binds specifically to the APC/C in HeLa K cell extract. [Fig. 11]

Question: Is the G3-6 molecule (SEQ. ID. NO. 33) binding to the APC/C?
Approach: Pulldown binding assay in HeLa K cell extract, azide magnetic beads
Conclusion: The G3-6 molecule (SEQ. ID. NO. 33) binds to the APC/C in HeLa K cell extract. [Fig. 12]

Question: Does the G3-6 molecule (SEQ. ID. NO. 33) bind specifically to the APC/C?
Approach: Pulldown binding assay in HeLa K cell extract, azide magnetic beads
Conclusion: The G3-6 molecule (SEQ. ID. NO. 33) binds specifically to the APC/C in cell extract. [Fig. 13]

### EXAMPLE 3

Question: What effect has the G3-6_mod1_miniPEG molecule (SEQ. ID. NO. 36) on mitosis of HeLa K cells?
Approach: Live cell imaging of HeLa K cells in the presence of the G3-6_mod1_miniPEG molecule (SEQ. ID. NO. 36)
Conclusion: The G3-6_mod1_miniPEG molecule (SEQ. ID. NO. 36) induces a metaphase delay and causes cell death after metaphase in HeLa K cells. [Fig. 14, 15]

Question: What effect has the G2-6 molecule (SEQ. ID. NO. 19) on mitosis of hTERT RPE-1 cells?
Approach: Live cell imaging of hTERT RPE-1 cells in the presence of G2-6 (SEQ. ID. NO. 19) and the G2-N2 control molecule
Conclusion: The G2-6 molecule (SEQ. ID. NO. 19) causes prolonged mitosis and mitotic or subsequent cell death in hTERT RPE-1 cells. [Fig. 16]

### Methodology for Example 3

### Live cell imaging

Automated time-lapse microscopy was performed using ImageXpress Micro XLS wide-field screening microscope (Molecular Devices) equipped with a 10x, 0.5 NA., 20x, 0.7 NA, and 40x, 0.95 NA Plan Apo air objectives (Nikon), a laser-based autofocus and a full environmental control (5% CO2, 37°C). Cells were grown in 96-well plastic bottom plates (µclear, Greiner Bio-One) and for live cell imaging media was changed to DMEM without phenol red and riboflavin (Thermo Fisher Scientific), supplemented with 10% (v/v) FBS, 1% (v/v) Glutamax, 1% (v/v) penicillin-streptomycin, and 0.5 µg/ml amphotericin B.

### Live cell imaging of cells treated with APC/C inhibitors

HeLa K cells were seeded into 96-well plates (4000 - 4500 cells) one day prior the treatment with APC/C inhibitors. Cells were treated with 50 µM APC/C inhibitor G3_mod1_mini PEG and cell division was monitored by live cell imaging using ImageXpress Micro XLS wide-field screening microscope, images were acquired every 3 minutes for time courses of 48 hours. The length of mitosis was determined manually.

hTERT RPE-1 cells were seeded into 96-well plates (5000 cells) one day prior the treatment with APC/C inhibitors. Cells were treated with 100 µM APC/C inhibitor G2-6 and the negative control molecule G2-N2 and cell division was monitored by live cell imaging using ImageXpress Micro XLS wide-field screening microscope, images were acquired every 3 minutes for time courses of 12-24 hours. The length of mitosis was determined manually.

## Claims

1. A peptide or peptidomimetic comprising or consisting of the following sequence
(a) X1-X2-X3-X4-X5-X6-X7-X8-X9-X10-X11-X12-X13-X14
wherein
a. X1 is an alpha-amino acid, preferably a proteinogenic amino acid, more preferably Glu or Nmglu;
b. X2 is an alpha-amino acid, preferably a proteinogenic amino acid, or preferably selected from Trp, 2-NaI and 1-NaI;
c. X3 is an alpha-amino acid, preferably a proteinogenic amino acid, or preferably selected from Trp, 2-NaI, 1-NaI and Pra;
d. X4 is an alpha-amino acid, preferably a proteinogenic amino acid, or preferably selected from Lys, Cys, D-Cys, Asp, Glu, Dap, Aib and (R)-2-(7'-octenyl)Ala;
e. X5 is an alpha-amino acid, preferably a proteinogenic amino acid, more preferably Ile or Lys;
f. X6 is an alpha-amino acid, preferably a proteinogenic amino acid, more preferably Leu or Lys;
g. X7 is an alpha-amino acid, preferably a proteinogenic amino acid, more preferably Asp or Glu;
h. X8 is an alpha-amino acid, preferably a proteinogenic amino acid, more preferably Ile or Leu;
i. X9 is an alpha-amino acid, preferably a proteinogenic amino acid, more preferably Gln;
j. X10 is an alpha-amino acid, preferably a proteinogenic amino acid, more preferably Arg;
k. X11 is an alpha-amino acid, preferably a proteinogenic amino acid, or preferably Asp, Glu, Cys, D-Cys, Aib or (S)-2-(4'-pentenyl)Ala;
l. X12 is an alpha-amino acid, preferably a proteinogenic amino acid, more preferably Arg;
m. X13 is an alpha-amino acid, preferably a proteinogenic amino acid, more preferably Arg;
n. X14 is an alpha-amino acid, preferably a proteinogenic amino acid, more preferably Ala;
wherein X2 to X12 of said peptide or peptidomimetic of assume an alpha-helix structure under physiological conditions;
(b) X1-X2-X3-X4-X5-X6-X7-X8-X9-X10-X11-X12
wherein
a. X1 is an amino acid, preferably an alpha-amino acid, more preferably a proteinogenic amino acid, or preferably selected from Arg, Phe(2-guanidino) and Phe(3-guanidino);
b. X2 is an alpha-amino acid, preferably a proteinogenic amino acid, or preferably selected from Trp, Phe, Tyr, Ile, Leu, Cys, L-propargylglycine (Pra), L-homopropargylglycine (Hpg), allylglycine (Agl), prenylglycine (Pre), crotylglycine (Crt) and (S)-2-amino-4-bromobutyric acid;
c. X3 is an alpha-amino acid, preferably a proteinogenic amino acid, or preferably selected from Ser, His and Dab;
d. X4 is an alpha-amino acid, preferably a proteinogenic amino acid, or preferably selected from Trp, Cys, Pra, Hpg, Agl, Pre, Crt and (S)-2-amino-4-bromobutyric acid;
e. X5 is an alpha-amino acid, preferably a proteinogenic amino acid, or preferably Gly or D-Ala;
f. X6 is an alpha-amino acid, preferably a proteinogenic amino acid, or preferably Pro or D-Pro;
g. X7 is an alpha-amino acid, preferably a proteinogenic amino acid, or preferably selected from Glu, Arg, Trp, Cit, Orn and pSer;
h. X8 is an alpha-amino acid, preferably a proteinogenic amino acid, more preferably Thr;
i. X9 is an alpha-amino acid, preferably a proteinogenic amino acid, or preferably selected from Phe, Cys, Agl, Pre, Crt, 3-azido-L-alanine (Dap(N3)), (S)-2-amino-4-azido-butyric acid (2Abu(y-N3), 5-azido-noravline (Nva(δ-N3), and (S)-2-amino-4-bromobutyric acid;
j. X10 is an alpha-amino acid, more preferably a proteinogenic amino acid, or preferably selected from Trp, 2-NaI and 1-NaI;
k. X11 is an alpha-amino acid, preferably a proteinogenic amino acid, or preferably selected from Trp, Phe, Tyr, Ile, Leu, Cys, (S)-2-amino-4-bromobutyric acid, Dap(N3), 2Abu(γ-N3), Nva(δ-N3), Agl and Crt;
l. X12 is an alpha-amino acid, preferably a proteinogenic amino acid, or preferably selected from Arg, Phe(3-guanidino), Phe(4-guanidino), Cit and Orn;
wherein said peptide or peptidomimetic assumes a beta-hairpin structure under physiological conditions, wherein preferably X1 to X4 and X9 to X12 are in beta-sheet conformation and/or a turn is formed by residues X5 to X8;
(c) X1-X2-X3-X4-X5-X6-X7-X8-X9-X10-X11-X12-X13-X14-X15-X16
wherein
a. X1 is an alpha-amino acid, preferably a proteinogenic amino acid, more preferably Thr;
b. X2 is an alpha-amino acid, preferably a proteinogenic amino acid, or preferably selected from Ser, His and hSer;
c. X3 is an alpha-amino acid, preferably a proteinogenic amino acid, or preferably selected from Cys, Pra, Hpg, Agl, Crt and (S)-2-amino-4-bromobutyric acid;
d. X4 is an alpha-amino acid, preferably a proteinogenic amino acid, more preferably Asp;
e. is an alpha-amino acid, preferably a proteinogenic amino acid, more preferably Asp;
f. X6 is an alpha-amino acid, preferably a proteinogenic amino acid, more preferably Pro;
g. is an alpha-amino acid, preferably a proteinogenic amino acid, or preferably Glu, Trp or Aad;
h. X8 is an alpha-amino acid, preferably a proteinogenic amino acid, more preferably Thr;
i. X9 is an alpha-amino acid, preferably a proteinogenic amino acid, or preferably selected from Trp, 2-NaI and 1-NaI;
j. X10 is a group consisting of any natural amino acid, preferably a proteinogenic amino acid, more preferably Arg;
k. X11 is alpha-amino acid, preferably a proteinogenic amino acid, or preferably selected from Trp, 2-NaI and 1-NaI;
l. X12 is an alpha-amino acid, preferably a proteinogenic amino acid, more preferably Asn;
m. X13 is an alpha-amino acid, preferably a proteinogenic amino acid, or preferably selected from Cys, (S)-2-amino-4-bromobutyric acid, Dap(N3), 2Abu(γ-N3), Nva(δ-N3), Agl and Crt;
n. X14 is a group consisting of any natural amino acid, preferably a proteinogenic amino acid, more preferably Gln;
o. X15 is alpha-amino acid, preferably a proteinogenic amino acid, or preferably selected from Arg, Phe(3-guanidino), Phe(4-guanidino) and Cit;
p. X16 is an alpha-amino acid, preferably a proteinogenic amino acid, more preferably Val;
wherein a side chain of X3 is covalently connected to a side chain of X13; and wherein said peptide or peptidomimetic assumes a helix-turn-helix structure under physiological conditions, wherein preferably X1 to X4 and X10 to X15 are in alpha-helical conformation and/or a turn is formed by X5 to X9;
(d) X1-X2-X3-X4-X5-X6-X7-X8-X9-X10-X11-X12-X13-X14-X25-X16-X17
wherein
a. X1 is an alpha-amino acid, preferably a proteinogenic amino acid, more preferably Gly;
b. X2 is an alpha-amino acid, preferably a proteinogenic amino acid, more preferably Asp;
c. X3 is an alpha-amino acid, preferably a proteinogenic amino acid, or preferably Thr or hSer;
d. X4 is an alpha-amino acid, preferably a proteinogenic amino acid, or preferably selected from Cys, Pra, Hpg, Agl, Crt and (S)-2-amino-4-bromobutyric acid;
e. X5 is an alpha-amino acid, preferably a proteinogenic amino acid, more preferably Asp;
f. X6 is an alpha-amino amino acid, preferably a proteinogenic amino acid, more preferably Asp;
g. X7 is an alpha-amino amino acid, preferably a proteinogenic amino acid, more preferably Pro;
h. X8 is an alpha-amino acid, preferably a proteinogenic amino acid, or preferably Glu, Trp or Aad;
i. X9 is an alpha-amino acid, preferably a proteinogenic amino acid, more preferably Thr;
j. X10 is an alpha-amino acid, preferably a proteinogenic amino acid, or preferably selected from Trp, 2-NaI and 1-NaI;
k. X11 is an alpha-amino acid, preferably a proteinogenic amino acid, more preferably Arg;
l. X12 is an alpha-amino acid, preferably a proteinogenic amino acid, or preferably selected from Trp, 2-NaI and 1-NaI;
m. X13 is an alpha-amino acid, preferably a proteinogenic amino acid, more preferably Asn;
n. X14 is an alpha-amino acid, preferably a proteinogenic amino acid, or preferably selected from Cys, (S)-2-amino-4-bromobutyric acid, Dap(N3), 2Abu(γ-N3), Nva(δ-N3), Pra, Agl and Crt;
o. X15 is an alpha-amino acid, preferably a proteinogenic amino acid, more preferably Gln;
p. X16 is an alpha-amino acid, preferably a proteinogenic amino acid, or preferably Arg or Cit;
q. X17 is an alpha-amino acid, preferably a proteinogenic amino acid, more preferably His;
wherein a side chain of X4 is covalently connected to a side chain of X14;
and wherein said peptide or peptidomimetic assumes a helix-turn-helix structure under physiological conditions, wherein preferably X2 to X5 and X11 to X16 are in alpha-helical conformation and/or a turn is formed by X6 to X10;
(e) X1-X2-X3-X4-X5-X6-X7-X8-X9-X10-X11-X12-X13-X14
wherein
a. X1 is an alpha-amino acid, preferably a proteinogenic amino acid, or preferably selected from Cys, Pra, Hpg, Agl, Crt and (S)-2-amino-4-bromobutyric acid;
b. X2 is an alpha-amino acid, preferably a proteinogenic amino acid, more preferably Glu;
c. X3 is an alpha-amino acid, preferably a proteinogenic amino acid, or preferably Ala or Aib;
d. X4 is an alpha-amino acid, preferably a proteinogenic amino acid, more preferably Gly;
e. X5 is an alpha-amino acid, preferably a proteinogenic amino acid, more preferably Pro;
f. X6 is an alpha-amino acid, preferably a proteinogenic amino acid, more preferably Ser;
g. X7 is an alpha-amino acid, preferably a proteinogenic amino acid, more preferably Gln;
h. X8 is an alpha-amino acid, preferably a proteinogenic amino acid, or preferably selected from Trp, 2-NaI and 1-NaI;
i. X9 is an alpha-amino acid, preferably a proteinogenic amino acid, more preferably selected from His, Asn and Gln;
j. X10 is an alpha-amino acid, preferably a proteinogenic amino acid, or preferably selected from Cys, (S)-2-amino-4-bromobutyric acid, Dap(N3), 2Abu(γ-N3), Nva(δ-N3), Agl and Crt;
k. X11 is an alpha-amino acid, preferably a proteinogenic amino acid, more preferably Thr or Glu;
l. X12 is an alpha-amino acid, preferably a proteinogenic amino acid, or preferably selected from Trp, 2-NaI and 1-NaI;
m. X13 is an alpha-amino acid, preferably a proteinogenic amino acid, or preferably Arg or Cit;
n. X14 is an alpha-amino acid, preferably a proteinogenic amino acid, more preferably Tyr;
wherein a side chain of X1 is covalently connected to a side chain of X10; wherein said peptide or peptidomimetic assumes a helix-turn structure under physiological conditions, wherein preferably X7 to X14 are in alpha-helical conformation and/or a turn is formed by X1 to X6;
(f) X1-X2-X3-X4-X5-X6-X7-X8-X9-X10-X11-X12-X13-X14
wherein
a. X1 is an alpha-amino acid, preferably a proteinogenic amino acid, more preferably Leu or Thr;
b. X2 is an alpha-amino acid, preferably a proteinogenic amino acid, or preferably selected from Trp, Phe and 2-NaI;
c. X3 is an alpha-amino acid, preferably a proteinogenic amino acid, more preferably Leu;
d. X4 is an alpha-amino acid, preferably a proteinogenic amino acid, more preferably Trp;
e. X5 is an alpha-amino acid, preferably a proteinogenic amino acid, more preferably Lys;
f. X6 is an alpha-amino acid, preferably a proteinogenic amino acid, or preferably selected from Ala, Trp, D-Leu, D-Thr and D-Glu;
g. X7 is an alpha-amino acid, preferably a proteinogenic amino acid, more preferably Glu;
h. X8 is an alpha-amino acid, preferably a proteinogenic amino acid, more preferably Trp;
i. X9 is an alpha-amino acid, preferably a proteinogenic amino acid, more preferably Glu or Thr;
j. X10 is an alpha-amino acid, preferably a proteinogenic amino acid, more preferably Lys;
k. X11 is an alpha-amino acid, preferably a proteinogenic amino acid, or preferably selected from Arg, Ser, Cit and hSer;
l. X12 is an alpha-amino acid, preferably a proteinogenic amino acid, more preferably Lys or Pra;
m. X13 is an alpha-amino acid, preferably a non-proteinogenic amino acid, or preferably selected from D-Pro, allylamine, cysteamine and Orn;
n. X14 is an alpha-amino acid, preferably a proteinogenic amino acid, more preferably selected from Pro, Aib or an acid comprising 4-pentenoyl or 3-bromopropionyl moiety;
wherein
1) the alpha-amino group of X1 is bound to the a carboxy group of X14; or
2) if X13 is Orn, the alpha-amino group of X1 is bound to the alpha-carboxy group of X13; and the side chain of X13 is bound to the alpha-carboxy group of X12;
and wherein said peptide or peptidomimetic assumes a beta-hairpin structure under physiological conditions, wherein X2 to X4 and X9 to X11 are in beta-sheet conformation and/or turns are formed by X5 to X8, and X12 to X14 and X1;
(g) X1-X2-X3-X4-X5-X6-X7-X8-X9-X10-X11-X12-X13-X14,
wherein
a. X1 is an alpha-amino acid, preferably a proteinogenic amino acid, more preferably Arg or Nmarg;
b. X2 is an alpha-amino acid, preferably a proteinogenic amino acid, or preferably selected from Trp, Glu, 2-NaI and 1-NaI;
c. X3 is an alpha-amino acid, preferably a proteinogenic amino acid, more preferably selected from Pro, Trp and Glu;
d. X4 is an alpha-amino acid, preferably a proteinogenic amino acid, or preferably selected from Gln, Leu, Arg, and Pra;
e. X5 is an alpha-amino acid, preferably a proteinogenic amino acid, or preferably selected from Lys, Glu, Ser, Lys(N3), (S)-2-(4'-pentenyl)Ala and Aib;
f. X6 is an alpha-amino acid, preferably a proteinogenic amino acid, more preferably Ile or Lys;
g. X7 is an alpha-amino acid, preferably a proteinogenic amino acid, or preferably selected from Lys, Trp, 2-NaI and 1-NaI;
h. X8 is an alpha-amino acid, preferably a proteinogenic amino acid, more preferably Asp or Glu;
i. X9 is an alpha-amino acid, preferably a proteinogenic amino acid, or preferably selected from Asp, Lys, Ser, (S)-2-(4'-pentenyl)Ala, Pra, D-Pra and Aib;
j. X10 is an alpha-amino acid, preferably a proteinogenic amino acid, more preferably Gln;
k. X11 is an alpha-amino acid, preferably a proteinogenic amino acid, more preferably Arg or Val;
l. X12 is an alpha-amino acid, preferably a proteinogenic amino acid, more preferably Arg or Thr;
m. X13 is an alpha-amino acid, preferably a proteinogenic amino acid, more preferably Arg or Trp;
n. X14 is an alpha-amino acid, preferably a proteinogenic amino acid, more preferably Trp or Arg;
wherein X2 to X13 of said peptide or peptidomimetic assume an alpha-helix structure under physiological conditions;
(h) X1-X2-X3-X4-X5-X6-X7-X8-X9-X10-X11-X12-X13-X14
wherein
a. X1 is an alpha-amino acid, preferably a proteinogenic amino acid, more preferably Glu or Nmglu;
b. X2 is an alpha-amino acid, preferably a proteinogenic amino acid, or preferably selected from Trp, 2-NaI, and 1-NaI;
c. X3 is an alpha-amino acid, preferably a proteinogenic amino acid, more preferably Lys, Glu, Ser, Lys(N3) or (S)-2-(4'-pentenyl)Ala;
d. X4 is an alpha-amino acid, preferably a proteinogenic amino acid, more preferably selected from Trp, 2-NaI, and 1-NaI;
e. X5 is an alpha-amino acid, preferably a proteinogenic amino acid, more preferably Ile, Trp or Arg;
f. X6 is an alpha-amino acid, preferably a proteinogenic amino acid, more preferably selected from Ile, Leu and Lys;
g. X7 is an alpha-amino acid, preferably a proteinogenic amino acid, more preferably Asp, Lys, Ser, Pra, D-Pra or (S)-2-(4'-pentenyl)Ala;
h. X8 is an alpha-amino acid, preferably a proteinogenic amino acid, more preferably Lys, Glu, Ser, Lys(N3) or (S)-2-(4'-pentenyl)Ala;
i. X9 is an alpha-amino acid, preferably a proteinogenic amino acid, more preferably Ile;
j. X10 is an alpha-amino acid, preferably a proteinogenic amino acid, more preferably Gln;
k. X11 is an alpha-amino acid, preferably a proteinogenic amino acid, more preferably Ala or Val;
l. X12 is an alpha-amino acid, preferably a proteinogenic amino acid, more preferably Asp, Lys, Ser, Pra, D-Pra or (S)-2-(4'-pentenyl)Ala;
m. X13 is an alpha-amino acid, preferably a proteinogenic amino acid, more preferably selected from Arg, Lys, and Trp;
n. X14 is an alpha-amino acid, preferably a proteinogenic amino acid, more preferably Ala or Trp;
wherein a side chain of X3 is covalently connected to a side chain of X7; and wherein a side chain of X8 is covalently connected to a side chain of X12; and wherein X1 to X12 of said peptide or peptidomimetic assume an alpha-helix structure under physiological conditions;
or
(i) X1-X2-X3-X4-X5-X6-X7-X8-X9-X10-X11-X12-X13-X14-X15
wherein
a. X1 is an alpha-amino acid, preferably a proteinogenic amino acid, more preferably Glu, Arg, Nmglu or Nmarg;
b. X2 is an alpha-amino acid, preferably a proteinogenic amino acid, or preferably selected from Trp, 2-NaI and 1-NaI;
c. X3 is an alpha-amino acid, preferably a proteinogenic amino acid, or preferably selected from Trp, 2-NaI, 1-NaI and Glu;
d. X4 is an alpha-amino acid, preferably a proteinogenic amino acid, more preferably Lys, Glu, Ser, Lys(N3) or (S)-2-(4'-pentenyl)Ala;
e. X5 is an alpha-amino acid, preferably a proteinogenic amino acid, more preferably Ile or Arg;
f. X6 is an alpha-amino acid, preferably a proteinogenic amino acid, more preferably selected from Ile, Leu and Lys;
g. X7 is an alpha-amino acid, preferably a proteinogenic amino acid, more preferably Lys;
h. X8 is an alpha-amino acid, preferably a proteinogenic amino acid, more preferably Asp, Lys, Ser, Pra, D-Pra or (S)-2-(4'-pentenyl)Ala;
i. X9 is an alpha-amino acid, preferably a proteinogenic amino acid, more preferably Ile;
j. X10 is an alpha-amino acid, preferably a proteinogenic amino acid, more preferably Gln;
k. X11 is an alpha-amino acid, preferably a proteinogenic amino acid, more preferably Lys, Glu, Ser, Lys(N3) or (S)-2-(4'-pentenyl)Ala;
l. X12 is an alpha-amino acid, preferably a proteinogenic amino acid, more preferably Trp or Ile;
m. X13 is an alpha-amino acid, preferably a proteinogenic amino acid, more preferably selected from Arg, Lys and Trp;
n. X14 is an alpha-amino acid, preferably a proteinogenic amino acid, more preferably Arg or Trp;
o. X15 is an alpha-amino acid, preferably a proteinogenic amino acid, more preferably Asp, Lys, Ser, Pra, D-Pra or (S)-2-(4'-pentenyl)Ala;
wherein a side chain of X4 is covalently connected to a side chain of X8; and wherein a side chain of X11 is covalently connected to a side chain of X15; and wherein X1 to X15 of said peptide or peptidomimetic assume an alpha-helix structure under physiological conditions.

2. The peptide or peptidomimetic of claim 1(a), wherein
X2 is selected from Trp, 2-NaI and 1-NaI;
X4 is selected from Lys, Cys, D-Cys, Asp, Glu, Dap, Aib and (R)-2-(7'-octenyl)Ala;
X5 is Ile or Lys;
X8 is Ile or Leu;
X9 is Gln;
X11 is Asp, Glu, Cys, D-Cys, Aib or (S)-2-(4'-pentenyl)Ala;
and
X12 is Arg.

3. The peptide or peptidomimetic of claim 1 (b), wherein
X1 is selected from Arg, Phe(2-guanidino) and Phe(3-guanidino);
X2 is selected from Trp, Phe, Tyr, Ile, Leu, Cys, Pra, Hpg, Agl, Pre, Crt and (S)-2-amino-4-bromobutyric acid;
X3 is selected from Ser, His and Dab;
X6 is Pro or D-Pro;
X7 is selected from Glu, Arg, Trp, Cit, Orn and pSer;
X8 is Thr;
X10 is selected from Trp, 2-NaI and 1-NaI;
X11 is selected from Trp, Phe, Tyr, Ile, Leu, Cys, (S)-2-amino-4-bromobutyric acid, Dap(N3), 2-amino-4-azido-butyric acid (2Abu(γ-N3)), 5-azido-norvaline (Nva(δ-N3)), Agl and Crt;
and
X12 is selected from Arg, Phe(3-guanidino), Phe(4-guanidino), Cit and Orn.

4. The peptide or peptidomimetic of claim 1(c), wherein
X2 is selected from Ser, His and hSer;
X3 is selected from Cys, Pra, Hpg, Agl, Crt and (S)-2-amino-4-bromobutyric acid; X6 is Pro;
X7 is Glu, Trp or Aad;
X8 is Thr;
X9 is selected from Trp, 2-NaI and 1-NaI;
X11 is selected from Trp, 2-NaI and 1-NaI;
X12 is Asn;
X13 is selected from Cys, (S)-2-amino-4-bromobutyric acid, Dap(N3), 2Abu(γ-N3), Nva(δ-N3), Agl and Crt;
and
X15 is selected from Arg, Phe(3-guanidino), Phe(4-guanidino) and Cit.

5. The peptide or peptidomimetic of claim 1(d), wherein
X3 is Thr or hSer;
X4 is selected from Cys, Pra, Hpg, Agl, Crt and (S)-2-amino-4-bromobutyric acid; X8 is Glu, Trp, or Aad;
X9 is Thr;
X10 is selected from Trp, 2-NaI and 1-NaI;
X12 is selected from Trp, 2-NaI and 1-NaI;
X13 is Asn;
X14 is selected from Cys, (S)-2-amino-4-bromobutyric acid, Dap(N3), 2Abu(γ-N3), Nva(δ-N3), Agl and Crt;
and
X16 is Arg or Cit.

6. The peptide or peptidomimetic of claim 1(e), wherein
X1 is selected from Cys, Pra, Hpg, Agl, Crt and (S)-2-amino-4-bromobutyric acid;
X2 is Glu;
X5 is Pro;
X8 is selected from Trp, 2-NaI and 1-NaI;
X9 is selected from His, Asn and Gln;
X10 is selected from Cys, (S)-2-amino-4-bromobutyric acid, Dap(N3), 2Abu(γ-N3), Nva(δ-N3), Agl and Crt;
X12 is selected from Trp, 2-NaI and 1-NaI;
and
X13 is Arg or Cit.

7. The peptide or peptidomimetic of claim 1(f), wherein
X2 is selected from Trp, Phe and 2-NaI;
X4 is Trp;
X6 is selected from Ala, Trp, D-Leu, D-Thr and D-Glu;
X7 is Glu;
X9 is Glu or Thr;
X11 is selected from Arg, Ser, Cit and hSer;
X13 is selected from D-Pro, allylamine, cysteamine and Orn; and
X14 is selected from Pro, Aib, 4-pentenoic acid and 3-bromopropionic acid.

8. The peptide or peptidomimetic of claim 1(g), wherein
X2 is selected from Trp, Glu, 2-NaI and 1-NaI;
X5 is selected from Lys, Glu, Lys(N3), (S)-2-(4'-pentenyl)Ala and Aib;
X6 is Ile or Lys;
X9 is selected from Asp, Lys, (S)-2-(4'-pentenyl)Ala, Pra, D-Pra and Aib; X10 is Gln;
X13 is Arg or Trp;
and
X14 is Trp or Arg.

9. The peptide or peptidomimetic of claim 1(h), wherein
X2 is selected from Trp, 2-NaI and 1-NaI;
X3 is Lys, Glu, Ser, Lys(N3) or (S)-2-(4'-pentenyl)Ala;
X5 is Ile, Trp or Arg;
X6 is selected from Ile, Leu and Lys;
X7 is Asp, Lys, Ser, Pra, D-Pra or (S)-2-(4'-pentenyl)Ala;
X8 is Lys, Glu, Ser, Lys(N3) or (S)-2-(4'-pentenyl)Ala;
X9 is Ile;
X10 is Gln;
X12 is Asp, Lys, Ser, Pra, D-Pra or (S)-2-(4'-pentenyl)Ala;
and
X13 is selected from Arg, Lys, and Trp.

10. The peptide or peptidomimetic of claim 1(i), wherein
X2 is selected from Trp, 2-NaI and 1-NaI;
X4 is Lys, Glu, Ser, Lys(N3) or (S)-2-(4'-pentenyl)Ala;
X6 is selected from Ile, Leu and Lys;
X8 is Asp, Lys, Ser, Pra, D-Pra or (S)-2-(4'-pentenyl)Ala;
X9 is Ile;
X10 is Gln;
X11 is Lys, Glu, Ser, Lys(N3) or (S)-2-(4'-pentenyl)Ala;
X13 is selected from Arg, Lys, and Trp;
and
X15 is Asp, Lys, Ser, Pra, D-Pra or (S)-2-(4'-pentenyl)Ala.

11. The peptide or peptidomimetic of any one of the preceding claims, wherein said peptide or peptidomimetic is capable of interfering with ubiquitination.

12. The peptide or peptidomimetic of any one of the preceding claims, wherein said peptide or peptidomimetic stops cell division or induces cell death.

13. The peptide or peptidomimetic of claim 1(a) and any one of claims 2 to 12, to the extent they refer back to claim 1(a), wherein
(a) the alpha-amino group of X1 is bound to a moiety selected from acetyl; benzoyl; Biotin-Ahx; methyl, Ac-Gly-Phe-Trp-Phe-Gly; Ac-Gly-Phe-Trp-Phe-Gly-NH-(CH₂)₂-O-CH₂-CO-; Ac-Gly-Phe-Trp-Phe-Gly-NH-(CH₂)₂-PEG-O-CH₂-CO-, PEG being polyethylene glycol with a polymerization degree from 1 to 6; and R-COO-(CH₂)ₙ-OCO-, R-COO-(CH₂)ₙ-OCO-N(CH₃)-CH₂-CO-, R-COO-(CH₂)ₙ-OCOO-(CH₂)₂-CO-, or R-COO-(CH₂)ₙ-OCO-NH-(CH₂)₂-CO-, R being alkyl, cycloalkyl, cycloheteroalkyl, aryl or heteroaryl, alkyl having 1 to 6 carbon atoms, cycles having 4 to 10, preferably 5 or 6 ring atoms and 1 or 2 cycles, R preferably being methyl, ethyl, phenyl or benzyl, and n being 1 or 2;
(b) X4 is (R)-2-(7'-octenyl)Ala and X11 is (S)-2-(4'-pentenyl)Ala, and a side chain of X4 is bound to a side chain of X11 by a covalent bond;
(c) X4 and X11 are Asp or preferably Glu, and their side chains are bound through a diamine alkyl moiety consisting of -NH-CH₂-(CH₂)ₙ-CH₂-NH-, n being 1, 2 or 3;
(d) X4 is D-Cys and X11 is Cys or D-Cys, and a side chain of X4 is bound to a side chain of X11 via an aryl moiety, preferably 1,1'-biphenyl-4,4'-bis(methyl) or 3,3'-bipyridine,6,6'-bis(methyl);
(e) X4 and X11 are Cys, and a side chain of X4 is bound to a side chain of X11 via an azoaryl moiety, preferably *N,N*'-[(1Z)-azodi-4,1-phenylene]diacetamide or *cis-3,3'-*bis(sulfonato)-4,4'-bis(acetamide)azobenzene;
(f) X4 is Dap and X11 is Asp, and a side chain of X4 is bound to a side chain of X11 via the aryl moiety -CO-CH₂-*p*-C₆H₄-CH₂-NH-, or -CO-CH₂-*p*-C₆F₄-CH₂-NH; or
(g) X14 is amidated, esterified or bound to a moiety selected from Gly-Phe-Trp-Phe-Gly-NH₂, -NH-(CH₂)₂-O-CH₂-CO-Gly-Phe-Trp-Phe-Gly-NH₂ and -NH-(CH₂)₂-PEG-O-CH₂-CO-Gly-Phe-Trp-Phe-Gly-NH₂, PEG being polyethylene glycol with a polymerization degree from 1 to 6.

14. The peptide or peptidomimetic of claim 1(b) and any one of claims 2 to 12, to the extent they refer back to claim 1(b), wherein
(a) the alpha-amino group of X1 is bound to a moiety selected from acetyl; benzoyl; Biotin-Ahx; methyl, Ac-Gly-Phe-Trp-Phe-Gly; Ac-Gly-Phe-Trp-Phe-Gly-NH-(CH₂)₂-O-CH₂-CO-; Ac-Gly-Phe-Trp-Phe-Gly-NH-(CH₂)₂-PEG-O-CH₂-CO-, PEG being polyethylene glycol with a polymerization degree from 1 to 6; and R-COO-(CH₂)ₙ-OCO-, R-COO-(CH₂)ₙ-OCO-N(CH₃)-CH₂-CO-, R-COO-(CH₂)ₙ-OCOO-(CH₂)₂-CO-, or R-COO-(CH₂)ₙ-OCO-NH-(CH₂)₂-CO-, R being alkyl, cycloalkyl, cycloheteroalkyl, aryl or heteroaryl, alkyl having 1 to 6 carbon atoms, cycles having 4 to 10, preferably 5 or 6 ring atoms and 1 or 2 cycles, R preferably being methyl, ethyl, phenyl or benzyl, and n being 1 or 2;
(b) wherein X2 and X11 are independently selected from Trp, Phe, Tyr, Ile and Leu;
(c) wherein a side chain of X2 is covalently connected to a side chain of X11;
a. wherein if X2 and X11 are Agl or Crt, respectively, the covalent connection may comprise a carbon-carbon double bond;
b. wherein if X2 is Cys and X11 is (S)-2-amino-4-bromobutyric acid, a C-S bond connects X2 with X11;
c. wherein if X2 is Pra and X11 is 2Abu(γ-N3) or Nva(δ-N3), a triazole group connects X2 with X11;
d. wherein if X2 and X11 are Cys, a disulfide bridge connects X2 with X11;
e. wherein if X2 is (S)-2-amino-4-bromobutyric acid and X11 is Cys, a C-S bond connects X2 with X11;
f. wherein if X2 is Hpg and X11 Dap(N3), a triazole group connects X2 with X11
(d) wherein X4 and X9 are independently selected from Trp and Phe;
(e) wherein a side chain of X4 is covalently connected to a side chain of X9;
a. wherein if X4 and X9 are Cys, a disulfide bridge connects X4 with X9;
b. wherein if X4 and X9 are Agl, the covalent connection may comprise a carbon-carbon double bond;
c. wherein if X4 and X9 are Crt, the covalent connection may comprise a carbon-carbon double bond;
d. wherein if X4 is Cys and X9 is (S)-2-amino-4-bromobutyric acid, a C-S bond connects X4 with X9;
e. wherein if X4 is (S)-2-amino-4-bromobutyric acid and X9 is Cys, a C-S bond connects X4 with X9;
f. wherein if X4 is Pra and X11 is 2Abu(γ-N3) or Nva(δ-N3), a triazole group connects X4 with X9;
g. wherein if X4 is Hpg and X11 Dap(N3), a triazole group connects X4 with X9; or
(f) wherein the alpha-carboxy group of X12 is amidated or bound to a moiety selected from -OEt, -OMe, -NHEt, -NHMe, -Gly-Phe-Trp-Phe-Gly-NH₂, -NH-(CH₂)₂-O-CH₂-CO-Gly-Phe-Trp-Phe-Gly-NH₂, and -NH-(CH₂)₂-PEG-O-CH₂-CO-Gly-Phe-Trp-Phe-Gly-NH₂, PEG being polyethylene glycol with a polymerization degree from 1 to 6;
wherein preferably the connections defined in parts (b) to (e) are implemented as follows:

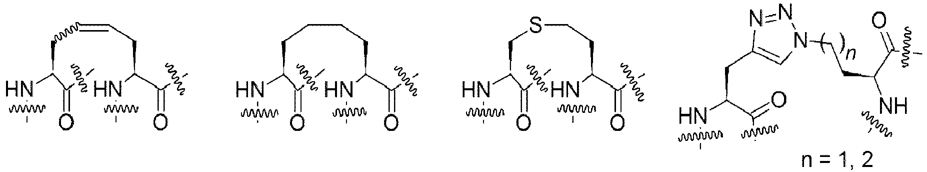

15. The peptide or peptidomimetic of claim 1(c) and any one of claims 2 to 12, to the extent they refer back to claim 1(c), wherein
(a) the alpha-amino group of X1 is bound to a moiety selected from acetyl; benzoyl; Biotin-Ahx; methyl, Ac-Gly-Phe-Trp-Phe-Gly; Ac-Gly-Phe-Trp-Phe-Gly-NH-(CH₂)₂-O-CH₂-CO-; Ac-Gly-Phe-Trp-Phe-Gly-NH-(CH₂)₂-PEG-O-CH₂-CO-, PEG being polyethylene glycol with a polymerization degree from 1 to 6; and R-COO-(CH₂)ₙ-OCO-, R-COO-(CH₂)ₙ-OCO-N(CH₃)-CH₂-CO-, R-COO-(CH₂)ₙ-OCOO-(CH₂)₂-CO-, or R-COO-(CH₂)ₙ-OCO-NH-(CH₂)₂-CO-, R being alkyl, cycloalkyl, cycloheteroalkyl, aryl or heteroaryl, alkyl having 1 to 6 carbon atoms, cycles having 4 to 10, preferably 5 or 6 ring atoms and 1 or 2 cycles, R preferably being methyl, ethyl, phenyl or benzyl, and n being 1 or 2;
(b) X3 and X13 are Cys, and the covalent connection between X3 and X13 comprises a sulfur-sulfur bond;
(c) X3 and X13 are Agl, and the covalent connection between X3 and X13 may comprise a carbon-carbon double bond;
(d) X3 and X13 are Crt, and the covalent connection between X3 and X13 may comprise a carbon-carbon double bond;
(e) X3 is Cys and X13 is (S)-2-amino-4-bromobutyric acid, and a C-S bond connects X3 with X13;
(f) X3 is (S)-2-amino-4-bromobutyric acid and X13 is Cys, and a C-S bond connects X3 with X13;
(g) X3 is Pra and X13 is 2Abu(γ-N3) or Nva(δ-N3), and a triazole group connects X3 with X13;
(h) X3 is Hpg and X13 is Dap(N3), and a triazole group connects X3 with X13; or
(i) the alpha-carboxy group of X16 is amidated or bound to a moiety selected from - OEt, -OMe, -NHEt, -NHMe, -Gly-Phe-Trp-Phe-Gly-NH₂, -NH-(CH₂)₂-O-CH₂-CO-Gly-Phe-Trp-Phe-Gly-NH₂ and -NH-(CH₂)₂-PEG-O-CH₂-CO-Gly-Phe-Trp-Phe-Gly-NH₂, PEG being polyethylene glycol with a polymerization degree from 1 to 6.

16. The peptide or peptidomimetic of claim 1(d) and any one of claims 2 to 12, to the extent they refer back to claim 1(d), wherein
(a) X4 and X14 are Cys, and the covalent connection between X4 and X14 comprises a sulfur-sulfur bond;
(b) X4 and X14 are Agl, and the covalent connection between X4 and X14 may comprise a carbon-carbon double bond;
(c) X4 and X14 are Crt, and the covalent connection between X4 and X14 may comprise a carbon-carbon double bond;
(d) X4 is Cys and X14 is (S)-2-amino-4-bromobutyric acid, and a C-S bond connects X4 with X14;
(e) X4 is (S)-2-amino-4-bromobutyric acid and X14 is Cys, and a C-S bond connects X4 with X14;
(f) X4 is Pra and X14 is 2Abu(γ-N3) or Nva(δ-N3), and a triazole group connects X4 with X14;
(g) X4 is Hpg and X14 is Dap(N3), and a triazole group connects X4 with X14; or
(h) the alpha-carboxy group of X17 is amidated, esterified or bound to a moiety selected from Gly-Phe-Trp-Phe-Gly-NH₂, -NH-(CH₂)₂-O-CH₂-CO-Gly-Phe-Trp-Phe-Gly-NH₂ and -NH-(CH₂)₂-PEG-O-CH₂-CO-Gly-Phe-Trp-Phe-Gly-NH₂, PEG being polyethylene glycol with a polymerization degree from 1 to 6.

17. The peptide or peptidomimetic of claim 1(e) and any one of claims 2 to 12, to the extent they refer back to claim 1(e), wherein
(a) the alpha-amino group of X1 is bound to a moiety selected from acetyl; benzoyl; Biotin-Ahx; methyl, Ac-Gly-Phe-Trp-Phe-Gly; Ac-Gly-Phe-Trp-Phe-Gly-NH-(CH₂)₂-O-CH₂-CO-; Ac-Gly-Phe-Trp-Phe-Gly-NH-(CH₂)₂-PEG-O-CH₂-CO-, R-COO-(CH₂)ₙ-OCO-N(CH₃)-CH₂-CO-, R-COO-(CH₂)ₙ-OCOO-(CH₂)₂-CO- or R-COO-(CH₂)ₙ-OCO-NH-(CH₂)₂-CO-, PEG being polyethylene glycol with a polymerization degree from 1 to 6; and R-COO-(CH₂)ₙ-OCO-, R being alkyl, cycloalkyl, cycloheteroalkyl, aryl or heteroaryl, alkyl having 1 to 6 carbon atoms, cycles having 4 to 10, preferably 5 or 6 ring atoms and 1 or 2 cycles, R preferably being methyl, ethyl, phenyl or benzyl and n being 1 or 2;
(b) X1 and X10 are Cys, and the covalent connection between X1 and X10 comprises a sulfur-sulfur bond;
(c) X1 and X10 are Agl, and the covalent connection between X1 and X10 may comprise a carbon-carbon double bond;
(d) X1 and X10 are Crt, and the covalent connection between X1 and X10 may comprise a carbon-carbon double bond;
(e) X1 is Cys and X10 is (S)-2-amino-4-bromobutyric acid, and a C-S bond connects X1 with X10;
(f) X1 is (S)-2-amino-4-bromobutyric acid and X10 is Cys, and a C-S bond connects X1 with X10;
(g) X1 is Pra and X10 is 2Abu(γ-N3) or Nva(δ-N3), and a triazole group connects X1 with X10;
(h) X1 is Hpg and X10 is Dap(N3), and a triazole group connects X1 with X10;
or
(i) X14 is amidated, esterified or functionalized with Gly-Phe-Trp-Phe-Gly-NH₂, -NH-(CH₂)₂-O-CH₂-CO-Gly-Phe-Trp-Phe-Gly-NH₂ and -NH-(CH₂)₂-PEG-O-CH₂-CO-Gly-Phe-Trp-Phe-Gly-NH₂, PEG being polyethylene glycol with a polymerization degree from 1 to 6.

18. The peptide or peptidomimetic of claim 1(f) and any one of claims 2 to 12, to the extent they refer back to claim 1(f), wherein
(a) X13 is allylamine or cysteamine, wherein the alpha-amino group of X13 is bound to a moiety selected from -CH₂CONH₂, -CH₂CO-Gly-Phe-Trp-Phe-Gly-NH₂, -CH₂COOEt and CH₂COOMe;
(b) X14 comprises a 4-pentenoyl, bromoacetyl or 3-bromopropionyl moiety, wherein the carboxy group of said moiety is covalently bound to the alpha-amino group of X1; or
(c) wherein a side chain of X13 is covalently connected to a side chain of X14;
iii. if X13 is allylamine and X14 is 4-pentenoic acid, the covalent connection may comprise a carbon-carbon double bond;
iv. if X13 is cysteamine and X14 is 3-bromopropionyl, a carbon-sulfur bond or a sulfur-sulfur bond is comprised in the covalent connection;
wherein preferably said covalent connection in accordance with (c) is selected from the following:

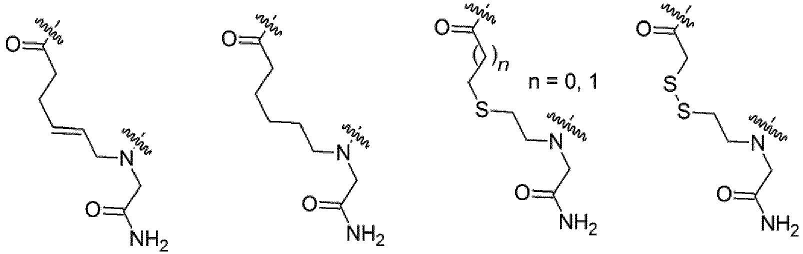

19. The peptide or peptidomimetic of claim 1(g) and any one of claims 2 to 12, to the extent they refer back to claim 1(g), wherein
(a) the alpha-amino group of X1 is bound to a moiety selected from acetyl; benzoyl; Biotin-Ahx; methyl, Ac-Gly-Phe-Trp-Phe-Gly; Ac-Gly-Phe-Trp-Phe-Gly-NH-(CH₂)₂-O-CH₂-CO-; Ac-Gly-Phe-Trp-Phe-Gly-NH-(CH₂)₂-PEG-O-CH₂-CO-, PEG being polyethylene glycol with a polymerization degree from 1 to 6; and R-COO-(CH₂)ₙ-OCO-, R-COO-(CH₂)ₙ-OCO-N(CH₃)-CH₂-CO-, R-COO-(CH₂)ₙ-OCOO-(CH₂)₂-CO- or R-COO-(CH₂)ₙ-OCO-NH-(CH₂)₂-CO-, R being alkyl, cycloalkyl, cycloheteroalkyl, aryl or heteroaryl, alkyl having 1 to 6 carbon atoms, cycles having 4 to 10, preferably 5 or 6 ring atoms and 1 or 2 cycles, R preferably being methyl, ethyl, phenyl or benzyl and n being 1 or 2;
(b) if neither X5 not X9 are Aib, a side chain of X5 is covalently connected to a side chain of X9, wherein the covalent connection may comprise a carbon-carbon double bond;
(c) X5 and X9 are Ser, and their side chains are bound through the adipoyl moiety - CO-(CH₂)₄-CO-;
or
(d) X14 is amidated, esterified or bound to a moiety selected from Gly-Phe-Trp-Phe-Gly-NH₂, -NH-(CH₂)₂-O-CH₂-CO-Gly-Phe-Trp-Phe-Gly-NH₂, and -NH-(CH₂)₂-PEG-O-CH₂-CO-Gly-Phe-Trp-Phe-Gly-NH₂, PEG being polyethylene glycol with a polymerization degree from 1 to 6.

20. The peptide or peptidomimetic of claim 1(h) and any one of claims 2 to 12, to the extent they refer back to claim 1(h), wherein
(a) the alpha-amino group of X1 is bound to a moiety selected from acetyl; benzoyl; Biotin-Ahx; methyl, Ac-Gly-Phe-Trp-Phe-Gly; Ac-Gly-Phe-Trp-Phe-Gly-NH-(CH₂)₂-O-CH₂-CO-; Ac-Gly-Phe-Trp-Phe-Gly-NH-(CH₂)₂-PEG-O-CH₂-CO-, PEG being polyethylene glycol with a polymerization degree from 1 to 6; and R-COO-(CH₂)ₙ-OCO-, R-COO-(CH₂)ₙ-OCO-N(CH₃)-CH₂-CO-, R-COO-(CH₂)ₙ-OCOO-(CH₂)₂-CO- or R-COO-(CH₂)ₙ-OCO-NH-(CH₂)₂-CO-, R being alkyl, cycloalkyl, cycloheteroalkyl, aryl or heteroaryl, alkyl having 1 to 6 carbon atoms, cycles having 4 to 10, preferably 5 or 6 ring atoms and 1 or 2 cycles, R preferably being methyl, ethyl, phenyl or benzyl and n being 1 or 2;
(b) wherein X3 is Lys and X7 is Asp, or X3 is Glu and X7 is Lys;
(c) wherein X8 is Lys and X12 is Asp, or X8 is Glu and X12 is Lys;
(d) wherein X3 and X7 are Ser, and their side chains are bound through the adipoyl moiety -CO-(CH₂)₄-CO-;
(e) wherein X8 and X12 are Ser, and their side chains are bound through the adipoyl moiety -CO-(CH₂)₄-CO-; or
(f) the alpha-carboxy group of X14 is amidated, esterified or bound to a moiety selected from Gly-Phe-Trp-Phe-Gly-NH₂, -NH-(CH₂)₂-O-CH₂-CO-Gly-Phe-Trp-Phe-Gly-NH₂ and -NH-(CH₂)₂-PEG-O-CH₂-CO-Gly-Phe-Trp-Phe-Gly-NH₂, PEG being polyethylene glycol of different polymerization degrees, preferably from 1 to 6.

21. The peptide or peptidomimetic of claim 1(i) and any one of claims 2 to 12, to the extent they refer back to claim 1(i), wherein
(a) the alpha-amino group of X1 is bound to a moiety selected from acetyl; benzoyl; Biotin-Ahx; methyl, Ac-Gly-Phe-Trp-Phe-Gly; Ac-Gly-Phe-Trp-Phe-Gly-NH-(CH₂)₂-O-CH₂-CO-; Ac-Gly-Phe-Trp-Phe-Gly-NH-(CH₂)₂-PEG-O-CH₂-CO-, PEG being polyethylene glycol with a polymerization degree from 1 to 6; and R-COO-(CH₂)ₙ-OCO-, R-COO-(CH₂)ₙ-OCO-N(CH₃)-CH₂-CO-, R-COO-(CH₂)ₙ-OCOO-(CH₂)₂-CO- or R-COO-(CH₂)ₙ-OCO-NH-(CH₂)₂-CO-, R being alkyl, cycloalkyl, cycloheteroalkyl, aryl or heteroaryl, alkyl having 1 to 6 carbon atoms, cycles having 4 to 10, preferably 5 or 6 ring atoms and 1 or 2 cycles, R preferably being methyl, ethyl, phenyl or benzyl and n being 1 or 2;
(b) wherein X4 is Lys and X8 is Asp, or X4 is Glu and X8 is Lys;
(c) wherein X11 is Lys and X15 is Asp, or X11 is Glu and X15 is Lys;
(d) wherein X4 and X8 are Ser, and their side chains are bound through the adipoyl moiety -CO-(CH₂)₄-CO-;
(e) wherein X11 and X15 are Ser, and their side chains are bound through the adipoyl moiety -CO-(CH₂)₄-CO-; or
(f) the corresponding carboxy group X15 is amidated, esterified or bound to a moiety selected from Gly-Phe-Trp-Phe-Gly-NH₂, -NH-(CH₂)₂-O-CH₂-CO-Gly-Phe-Trp-Phe-Gly-NH₂, and -NH-(CH₂)₂-PEG-O-CH₂-CO-Gly-Phe-Trp-Phe-Gly-NH₂, PEG being polyethylene glycol of different polymerization degrees, preferably from 1 to 6.

22. The peptide or peptidomimetic of any one of the preceding claims, wherein said peptide or peptidomimetic is conjugated, preferably via a linker, to a ligand of an E3 ligase.

23. A peptide or peptidomimetic of any one of the preceding claims for use in medicine.

24. A medicament comprising or consisting of the peptide or peptidomimetic of any one of the preceding claims.

25. The medicament of claim 24, wherein
(a) one or more peptides or peptidomimetics of any one of the preceding claims is/are the only pharmaceutically active agents comprised in said medicament;
(b) said medicament comprises one or more further pharmaceutically active agents, preferably selected from
a. agents which interfere with or stop cell division such as TAME, proTAME, apcin, GLMN or Emi1;
b. agents which induce DNA damage, such as Topoisomerase II inhibitors, preferably etoposide or doxorubicin; and
c. agents which interfere with microtubule assembly such as paclitaxel, vincristine, and PLK1 inhibitors including BI6727; or
(c) said medicament is to be administered to a patient which is undergoing, has undergone, or will undergo radiation therapy.

26. A peptide or peptidomimetic of any one of claims 1 to 23 for use in a method of treating, ameliorating or curing a hyperproliferative disease.

27. A method of purifying APC/C, said method comprising
(a) bringing into contact a sample comprising APC/C with a peptide or peptidomimetic of any one of claims 1 to 22; and
(b) separating a complex comprising APC/C and said peptide or peptidomimetic from the remainder of the constituents of said sample.

28. A method of detecting APC/C, said method comprising
(a) bringing a sample comprising or suspected to comprise APC/C in contact with a peptide or peptidomimetic of any one of claims 1 to 22, wherein said peptide or peptidomimetic carries a detectable label; and
(b) detecting a complex comprising APC/C and said peptide or peptidomimetic.

29. A kit comprising or consisting of one or more peptides or peptidomimetics of any one of claims 1 to 22.
